# EUROPEAN PATENT APPLICATION

(11) **EP 2 322 553 A2**
(43) Date of publication of application: **18.05.2011**
(21) Application number: 10176925.5
(22) Date of filing: 14.02.2006
(51) Int. Cl.: C07K 16/24, C07K 16/28, C07K 14/54, C07K 14/715, C12N 15/11, G01N 33/68, A61K 39/39, A61K 39/395, A61P 37/00, A61P 29/00, A61K 38/20

(54) **Interleukin-17F antibodies and other IL-17F signaling antagonists and uses therefor**

(30) Priority: 14.02.2005 US 653260 P; 01.04.2005 US 667492 P
(62) Divisional of application: 06734991.0
(71) Applicant: Wyeth LLC, Madison, NJ 07940 (US)
(72) Inventor: Carreno, Beatriz M., Clayton, MO 63105 (US); Collins, Mary, Natick, MA 01760 (US); Wright, Jill F., Ashland, MA 01721 (US); Wolfman, Neil M., Dover, MA 02030 (US); Arai, Maya, Brookline, MA 02446 (US); Jacobs, Kenneth, Newton, MA 02460 (US); Lu, Zhijian, Bedford, MA 01730 (US); Guo, Yongjing, Chesnut Hill, MA 02467 (US); Qiu, Yongchang, Acton, MA 01720 (US)
(74) Representative: Courgeon, Antoine

(57) **Abstract**

The present invention provides isolated and purified polynucleotides and polypeptides related to the IL-17F signaling pathway. The invention also provides antibodies to IL-17F homodimers and IL-17A/IL-17F heterodimers, and methods of isolating and purifying members of the IL-17 family, including IL-17A/IL-17F heterodimers, from a natural source. The present invention also is directed to novel methods for diagnosing, prognosing, monitoring the progress of, and treating and/or preventing disorders related to IL-17F signaling, i.e., IL-17F-associated disorders, including, but not limited to, inflammatory disorders, such as autoimmune diseases (e.g., arthritis (including rheumatoid arthritis), psoriasis, systemic lupus erythematosus, and multiple sclerosis), respiratory diseases (e.g., COPD, cystic fibrosis, asthma, allergy), transplant rejection (including solid organ transplant rejection) and inflammatory bowel diseases or disorders (IBDs, e.g., ulcerative colitis, Crohn's disease). The present invention is further directed to novel therapeutics and therapeutic targets, and to methods of screening and assessing test compounds for the intervention (treatment) and prevention of disorders related to IL-17F signaling.

## Description

### Related Applications

This application claims priority to U.S. Provisional Patent Application No. 60/653,260, filed February 14, 2005; and U.S. Provisional Patent Application No. 60/667,492, filed April 1, 2005, both of which are hereby incorporated by reference herein in their entireties.

### BACKGROUND OF THE INVENTION

### Field of the Invention

This invention relates to antibodies, e.g., intact antibodies and antigen-binding fragments thereof, and other IL-17F signaling antagonists, e.g., soluble IL-17F receptor(s), that interfere with interleukin-17F (IL-17F) signaling, in particular, human IL-17F, and their uses in regulating IL-17F-associated activities. The antibodies and related IL-17F molecules disclosed herein are useful in diagnosing, prognosing, monitoring, preventing, and/or treating IL-17F-associated disorders, e.g., inflammatory disorders (e.g., autoimmune diseases (e.g., arthritis (including rheumatoid arthritis), psoriasis, systemic lupus erythematosus (SLE), multiple sclerosis), respiratory diseases (e.g., COPD, cystic fibrosis, asthma, allergy), transplant rejection (including solid organ transplant rejection), and inflammatory bowel diseases or disorders (IBDs, e.g., ulcerative colitis, Crohn's disease)).

### Related Background Art

Cytokines are secreted soluble proteins with pleiotropic activities involved in immune and inflammatory responses, e.g., cytokines may cause differentiation, recruitment, or other physiological responses, e.g., secretion of proteins characteristic of inflammation, by target cells. Cytokines bind to specific cell surface receptors, triggering signal transduction pathways that lead to cell activation, proliferation, and differentiation. One such cytokine, interleukin-17 (IL-17), originally named CTLA-8, was isolated and cloned from murine hybridomas and shown to have homology to open reading frame 13 of the T lymphotropic Herpesvirus saimiri (Rouvier et al. (1993) J. Immunol. 150:5445-56; Yao et al. (1996) Gene 168:223-25; Golstein et al., published International Patent Application No. WO95/01826). Since then, five related cytokines that share 20-50% homology to IL-17 have been identified (see Moseley et al. (2003) *Cytokine & Growth Factor Reviews*14*:* 155-74). To indicate IL-17 as the founding member of the IL-17 cytokine family, it has been designated IL-17A (Moseley, *supra*); the other members have been designated IL-17B, IL-17C, IL-17D, IL-17E, and IL-17F. IL-17 cytokine family members share conserved cysteine residues. Of interest are IL-17A and particularly IL-17F, which share 50% identity; both cytokines are induced by IL-23, coexpressed by T cells, and considered potential targets for T cell-mediated autoimmune diseases. Similar to IL-17A, the conserved cysteine residues in IL-17F exhibit features of a classic cysteine knot motif found in bone morphogenetic proteins (BMPs), transforming growth factor-beta (TGF-β), nerve growth factor (NGF) and platelet-derived factor BB (PDGF-BB) (Hymowitz et al. (2001) EMBO J. 20:5332-41; McDonald et al. (1993) Cell 73:421-24).

IL-17F is a 17kD secreted protein that was cloned from an activated human PBMC library (SST) (U.S. Patent Nos. 6,043,344 and 6,074,849). It forms a 30-35kD disulfide-linked homodimer (Hymowitz, *supra)* and, similar to IL-17A, is expressed primarily by activated T cells (Moseley, *supra).* However, expression of IL-17F by activated monocytes, activated basophils and mast cells has also been shown (Kawaguchi et al. (2002) J. Immunol. 167:4430-35). IL-17F induces the expression of many cytokines and chemokines by macrophages, endothelial cells, epithelial cells, and fibroblasts (Moseley, *supra).*

IL-17F plays a role in inflammatory responses, in part, by inducing the production of inflammatory cytokines and neutrophilia. It is associated with the development of several autoimmune diseases, e.g., arthritis (including rheumatoid and Lyme arthritis), systemic lupus erythematosus (SLE), and asthma (Bettelli and Kuchroo (2005) J. Exp. Med. 201:169-71). For example, it has recently been shown that IL-23 is essential for the expansion of a T cell population which is characterized by, inter alia, production of IL-17F, that passive transfer of this T cell population is essential for the establishment of organ-specific inflammation associated with central nervous system autoimmunity (Langrish et al. (2005) J. Exp. Med. 201:233-40), and that IL-17-deficient mice are resistant to experimental autoimmune encephalomyelitis (EAE; an animal model for multiple sclerosis) (Nakae et al. (2003) J. Immunol. 171:6173-77). IL-17F is unique among known inflammatory cytokines in that it increases proteoglycan breakdown and decreases proteoglycan synthesis by articular cartilage (Hymowitz, *supra).* Additionally, increased expression of IL-17F has been demonstrated in bronchoalveolar lavages (BALs) taken from patients suffering with asthma after allergen challenge compared to BALs taken from these patients as controls (Kawaguchi, *supra).* Also, IL-17F mRNA expression is increased in patients with ulcerative colitis and Crohn's disease (Gurney et al. (2003) GTCBIO Conf. Cytokines and Beyond). These observations suggest that blockade of IL-17F signaling will reduce proinflammatory cytokine production and decrease bone erosion. Consequently, the IL-17F signaling pathway is an attractive target for treating and/or preventing inflammatory diseases, e.g., in which recruited neutrophils are critical mediators of tissue injury, e.g., during the development of autoimmune diseases (e.g., arthritis (including rheumatoid arthritis), psoriasis, systemic lupus erythematosus, multiple sclerosis), respiratory diseases (e.g., COPD, cystic fibrosis, asthma, allergy), transplant rejection (including solid organ transplant rejection), and inflammatory bowel disorders or diseases (IBDs, e.g., ulcerative colitis, Crohn's disease).

Currently, not much is known about the receptors for members of the IL-17 family. It has been shown that IL-17R, the receptor for IL-17A, is expressed in all tissues examined to date, and that binding of IL-17R by IL-17A generally results in the induction of proinflammatory cytokines through activation of NF-κB (Moseley, *supra).* Four additional receptors that share partial sequence homology to IL-17R have been identified: 1) IL-17RH1 (also called IL-17RB), 2) IL-17-receptor like protein (also called IL-17RL or IL-17RC), 3) IL-17RD (also called SEF or IL-17RLM, and 4) IL-17RE (Moseley, *supra*). Of these four additional receptors, only IL-17RH1 has been shown to bind to IL-17 cytokines, namely IL-17B and IL-17E; however, the function of IL-17B and IL-17E binding to IL-17RH1 has not been shown (Shi et al. (2000) J. Biol. Chem. 275:19167-76; Lee et al. (2001) J. Biol. Chem. 276:1660-64). To date, the receptor(s) for IL-17F has not been reported. Thus, IL-17F signaling has not been able to be targeted for the prevention and/or treatment of diseases, although it may play an important role in the homeostasis of tissues (e.g., joint tissues) and the progression of various diseases (e.g., arthritis, asthma, allergy, COPD, cystic fibrosis, ulcerative colitis, Crohn's disease, etc.). The present invention solves this problem by identifying and targeting key players involved in the signal transduction pathway of IL-17F protein.

### SUMMARY OF THE INVENTION

An object of the invention is to identify components of the IL-17F signaling pathway, e.g., IL-17F and its receptor, and to target these components in methods of treating disorders related to IL-17F signaling. Such IL-17F-associated disorders and disorders related to increased IL-17F signaling include, but are not limited to, inflammatory disorders, e.g., autoimmune diseases (e.g., arthritis (including rheumatoid arthritis), psoriasis, systemic lupus erythematosus, multiple sclerosis), respiratory diseases (e.g., COPD, cystic fibrosis, asthma, allergy), transplant rejection (including solid organ transplant rejection), and inflammatory bowel diseases (e.g., ulcerative colitis, Crohn's disease).

As such, the research underlying the present invention provides evidence that IL-17F mediates proteoglycan destruction and inflammatory responses through its binding to IL-17R and/or IL-17RC. The determination ofIL-17R and IL-17RC as receptors for IL-17F exposes these molecules as targets for the treatment of disorders related to IL-17F signaling.

Provided herein are IL-17F signaling antagonists, including, but not limited to, IL-17F inhibitory polynucleotides, IL-17R inhibitory polynucleotides, IL-17RC inhibitory polynucleotides, soluble polypeptides comprising IL-17R or IL-17F-binding fragments thereof, soluble polypeptides comprising IL-17RC or IL-17F-binding fragments thereof, inhibitory anti-IL-17F antibodies, inhibitory anti-IL-17R antibodies, inhibitory anti-IL-17RC antibodies, and antagonistic small molecules. Preferred examples of IL-17F signaling antagonists include siRNAs directed to IL-17R and IL-17RC, soluble fusion proteins comprising IL-17R and IL-17RC (or IL-17F-binding fragments thereof), and inhibitory (i.e., antagonistic) IL-17F antibodies. In another preferred embodiment of the invention, an IL-17F signaling antagonist, e.g., siRNAs directed against IL-17R or IL-17RC, soluble fusion proteins comprising IL-17R or IL-17RC (or IL-17F binding fragments thereof), or inhibitory IL-17F antibodies, decreases IL-17F bioactivity and/or the ability of NF-κB to activate NF-κB responsive genes.

Additionally, based on structural and sequence similarity between IL-17A and IL-17F, the inventors hypothesized and demonstrated the formation of novel IL-17A/IL-17F heterodimers. In demonstrating the existence of IL-17A/IL-17F heterodimers, the inventors are the first to demonstrate that IL-21 results in the increased production of IL-17A homodimers, IL-17F homodimers, and IL-17A/IL-17F heterodimers, and suggest that effects associated with IL-21 binding to and activating IL-21R may be due, at least in part, to IL-17 signaling. The inventors are also the first to isolate IL-17A homodimers, IL-17F homodimers and IL-17A/IL-17F heterodimers from a natural source of these cytokines, e.g., activated T cells. Thus, the invention also provides methods of mitigating effects associated with IL-21 binding to and activating IL-21R, e.g., by inhibiting IL-17A and/or IL-17F signaling. Additionally, the invention provides natural (i.e., nonrecombinant) IL-17A homodimers, IL-17F homodimers, and IL-17A/IL-17F heterodimers, and methods of isolating and targeting the same, e.g., in methods of treating disorders associated with increased IL-17F signaling and/or disorders associated with IL-21 binding to and activating IL-21R. Disclosed herein additionally are recombinant IL-17A homodimers, IL-17F homodimers, and IL-17A/IL-17F heterodimers, and methods of isolating IL-17A/IL-17F heterodimers (either recombinant or natural) substantially free of IL-17A homodimers and IL-17F homodimers.

Methods that target IL-17F signaling may involve IL-17F, IL-17R and/or IL-17RC polynucleotides (including inhibitory polynucleotides such as antisense, siRNA, and aptamers), polypeptides, and fragments thereof as IL-17F signaling antagonists. Additionally, antibodies capable of inhibiting the interaction of IL-17F protein (either as an IL-17F homodimer or as an IL-17A/IL-17F heterodimer) with its receptor(s) may also be used.

The invention also relates to using the molecules disclosed herein in methods of screening test compounds capable of targeting the IL-17F signaling pathway, and diagnosing, prognosing, monitoring and/or treating disorders related to IL-17F signaling.

In one embodiment, the present invention provides a method of screening for test compounds capable of antagonizing IL-17F signaling comprising the steps of: contacting a sample containing IL-17F and IL-17R with a compound; and determining whether the interaction of IL-17F with IL-17R in the sample is decreased relative to the interaction of IL-17F with IL-17R in a sample not contacted with the compound, whereby such a decrease in the interaction of IL-17F with IL-17R in the sample contacted with the compound identifies the compound as one that inhibits the interaction of IL-17F with IL-17R and is capable of antagonizing IL-17F signaling. In another embodiment, the invention provides a similar method of screening related to IL-17RC.

In another embodiment, the invention provides a method for diagnosing a disorder related to increased IL-17F signaling in a subject comprising the steps of: detecting a test amount of an IL-17F signaling gene product in a sample from the subject; and comparing the test amount with a normal amount of the same IL-17F signaling gene product in a control sample, whereby a test amount significantly above the normal amount provides a positive indication in the diagnosis of a disorder related to increased IL-17F signaling. In another embodiment, the disorder is selected from the group consisting of autoimmune diseases, respiratory diseases, and inflammatory bowel diseases. In other embodiments, the IL-17F signaling gene product is an IL-17F gene product, an IL-17R gene product, or an IL-17RC gene product.

In another embodiment, the invention provides a method of treating a subject at risk for, or diagnosed with, a disorder related to increased IL-17F signaling comprising administering to the subject a therapeutically effective amount of an IL-17F signaling antagonist. In another embodiment, the IL-17F signaling antagonist is selected from the group consisting of IL-17F inhibitory polynucleotides, IL-17R inhibitory polynucleotides, IL-17RC inhibitory polynucleotides, soluble polypeptides comprising IL-17R or IL-17F binding fragments thereof, soluble polypeptides comprising IL-17RC or IL-17F binding fragments thereof, inhibitory anti-IL-17F antibodies, inhibitory anti-IL-17R antibodies, inhibitory IL-17RC antibodies, and antagonistic small molecules. In some embodiments, the IL-17F signaling antagonist is an IL-17R inhibitory polynucleotide or an IL-17RC inhibitory polynucleotide. In some further embodiments, the inhibitory polynucleotide is an siRNA selected from the group consisting of the nucleotide sequences set forth in SEQ ID NOs:17-32. In some embodiments, the IL-17F signaling antagonist is a soluble polypeptide comprising IL-17R or IL-17F binding fragments thereof, or comprising IL-17RC or IL-17F binding fragments thereof. In some further embodiments, the soluble polypeptide has the amino acid sequence set forth in SEQ ID NO:34 or SEQ ID NO:35. In some other embodiments, (1) the IL-17F inhibitory polynucleotide comprises the nucleotide sequence set forth in, or a nucleotide sequence complementary to the nucleotide sequence set forth in, SEQ ID NO:1 or a fragment of SEQ ID NO:1, or an RNA equivalent thereof, and wherein expression of the inhibitory polynucleotide in a cell results in the decreased expression of IL-17F; (2) the IL-17R inhibitory polynucleotide comprises the nucleotide sequence set forth in, or a nucleotide sequence complementary to the nucleotide sequence set forth in, SEQ ID NO:5 or a fragment of SEQ ID NO:5, or an RNA equivalent thereof, and wherein expression of the inhibitory polynucleotide in a cell results in the decreased expression of IL-17R; and (3) the IL-17RC inhibitory polynucleotide comprises a nucleotide sequence selected from the group consisting of the nucleotide sequences set forth in, or a nucleotide sequence complementary to a nucleotide sequence selected from the group consisting of the nucleotide sequences set forth in, SEQ ID NO:7, SEQ ID NO:9, SEQ ID NO: 11, SEQ ID NO: 13, and SEQ ID NO:15 or a fragment of a nucleotide sequence selected from the group consisting of the nucleotide sequences set forth in SEQ ID NO:7, SEQ ID NO:9, SEQ ID NO:11, SEQ ID NO:13, and SEQ ID NO:15, or an RNA equivalent thereof, and wherein expression of the inhibitory polynucleotide in a cell results in the decreased expression of IL-17RC. In some embodiments, the disorder related to increased IL-17F signaling is an inflammatory disorder. In some further embodiments, the inflammatory disorder is selected from the group consisting of an autoimmune disease, a respiratory disease, and an inflammatory bowel disease. In some further embodiments, the inflammatory disorder is an autoimmune disease, and the autoimmune disease is selected from the group consisting of arthritis (including rheumatoid arthritis), psoriasis, systemic lupus erythematosus, and multiple sclerosis. In some further embodiments, the inflammatory disorder is a respiratory disease, and the respiratory disease is cystic fibrosis; or the inflammatory disorder is an inflammatory bowel disease.

In another embodiment, the invention further comprises administering to the subject a therapeutically effective amount of at least one additional therapeutic agent. In another embodiment, the at least one additional therapeutic agent is selected from the group consisting of cytokine inhibitors, growth factor inhibitors, immunosuppressants, anti-inflammatory agents, metabolic inhibitors, enzyme inhibitors, cytotoxic agents, and cytostatic agents. In another embodiment, the at least one additional therapeutic agent is selected from the group consisting of TNF antagonists, anti-TNF agents, IL-12 antagonists, IL-15 antagonists, IL-17 antagonists, IL-18 antagonists, IL-22 antagonists, T cell-depleting agents, B cell-depleting agents, cylosporin, FK-506, CCI-779, etanercept, infliximab, rituximab, adalimumab, prednisolone, azathioprine, gold, sulphasalazine, chloroquine, hydroxychloroquine, minocycline, anakinra, abatacept, methotrexate, leflunomide, rapamycin, rapamycin analogs, Cox-2 inhibitors, cPLA2 inhibitors, NSAIDs, p38 inhibitors, antagonists of B7.1, B7.2, ICOSL, ICOS and/or CD28, and agonists of CTLA4.

In another embodiment, the invention provides a method of inhibiting the ability of NF-κB to activate NF-κB-responsive promoters in a cell population or a subject, comprising administering an IL-17F signaling antagonist to the cell population or the subj ect. In another embodiment, the invention provides a method for inhibiting an IL-17F bioactivity in a cell population or a subject, the method comprising administering an IL-17F signaling antagonist to the cell population or the subject. In another embodiment, the IL-17F bioactivity is selected from the group consisting of neutrophil differentiation, neutrophil recruitment and cytokine induction.

In anther embodiment, the invention provides a pharmaceutical composition comprising an IL-17F signaling antagonist and a pharmaceutically acceptable carrier. In another embodiment, the invention provides a vaccine adjuvant comprising an IL-17F signaling antagonist and an antigen selected from the group consisting of an autoantigen, an allergen, an alloantigen, and fragments thereof. In another embodiment, the invention provides isolated antibodies capable of specifically binding to the amino acid sequences related to the present invention, including those set forth in SEQ ID NOs:6, 7, 9, 11, 13, and 15; in some embodiments, the antibody antagonizes IL-17F signaling.

In another embodiment, the invention provides an isolated antibody capable of specifically binding to IL-17F protein, and further embodiments wherein the IL-17F protein is derived from a human or a primate; wherein the IL-17F protein is multimeric; wherein the IL-17F protein is IL-17F homodimer or an IL-17F heterodimer; wherein the IL-17F heterodimer is IL-17A/IL-17F; and wherein the antibody inhibits IL-17F bioactivity.

In another embodiment, the invention provides the above-identified methods, wherein IL-17F signaling and/or IL-17F bioactivity is mediated by IL-17F homodimer, an IL-17F heterodimer, or both IL-17F homodimer and an IL-17F heterodimer, including wherein the IL-17F heterodimer is IL-17A/IL-17F.

In another embodiment, the invention provides the above-identified pharmaceutical composition and/or the above-identified vaccine adjuvant, wherein the IL-17F signaling antagonist antagonizes IL-17F homodimer, an IL-17F heterodimer, or both IL-17F homodimer and an IL-17F heterodimer.

In another embodiment, the invention provides a method of inhibiting at least one activity associated with IL-21 signaling comprising antagonizing IL-17F signaling. In another embodiment, the invention provides a method of inhibiting at least one activity associated with IL-23 signaling comprising antagonizing IL-17F signaling. In some further embodiments, the IL-17F signaling is mediated by IL-17F homodimer, an IL-17F heterodimer, or both IL-17F homodimer and an IL-17F heterodimer, including wherein the IL-17F heterodimer is IL-17A/IL-17F.

In another embodiment, the invention provides a method of purifying natural IL-17A protein comprising: activating T cells in media; and immunoprecipitating IL-17A protein from the media. In another embodiment, the invention provides a method of purifying natural IL-17F protein comprising: activating T cells in media; and immunoprecipitating IL-17F protein from the media. In some further embodiments, such methods are provided wherein the IL-17A protein is IL-17A homodimer, an IL-17A heterodimer, or both IL-17A homodimer and an IL-17A heterodimer, and/or wherein the IL-17F protein is IL-17F homodimer, an IL-17F heterodimer, or both IL-17F homodimer and an IL-17F heterodimer; and wherein the IL-17A or IL-17F heterodimer is IL-17A/IL-17F. In another embodiment, the media comprises IL-21 and/or IL-23.

In another embodiment, the invention provides an isolated IL-17F protein, wherein the IL-17F protein is IL-17F homodimer or an IL-17F heterodimer; wherein the IL-17F protein is isolated from a natural source; wherein the natural source is at least one T cell. In another embodiment, the invention provides an isolated IL-17A protein, wherein the IL-17A protein is IL-17A homodimer or an IL-17A heterodimer; wherein the IL-17A protein is isolated from a natural source; wherein the natural source is at least one T cell.

In another embodiment, the invention provides a method of inhibiting at least one activity associated with IL-17A signaling, comprising administering an IL-17F antagonist.

In another embodiment, the invention provides a method of isolating IL-17A/IL-17F heterodimers substantially free from IL-17A homodimers and IL-17F homodimers, comprising: (a) expressing an IL-17A fusion protein and an IL-17F fusion protein in host cells cultured in media, wherein the IL-17A fusion protein comprises an IL-17A protein or fragment thereof fused to a first affinity tag, and wherein the IL-17F fusion protein comprises an IL-17F protein or fragment thereof fused to a second affinity tag; (b) allowing the host cells to secrete the IL-17A fusion protein and IL-17F fusion protein into the media; (c) placing the media over a first affinity column under nonreducing conditions such that the IL-17A fusion protein binds to the first affinity column; (d) eluting the bound protein from the first affinity column under nonreducing conditions; (e) placing the eluent obtained from step (d) over a second affinity column under nonreducing conditions such that the IL-17F fusion protein binds to the second affinity column; and (f) eluting the bound protein from the second affinity column under nonreducing conditions, wherein the eluent obtained from step (f) contains both IL-17A fusion protein and IL-17F fusion protein in the form of IL-17A/IL-17F heterodimers. In other embodiments, variations of this method are provided. In another embodiment, the invention provides an IL-17A/IL-17F heterodimer isolated according to these various methods.

### BRIEF DESCRIPTION OF THE DRAWINGS

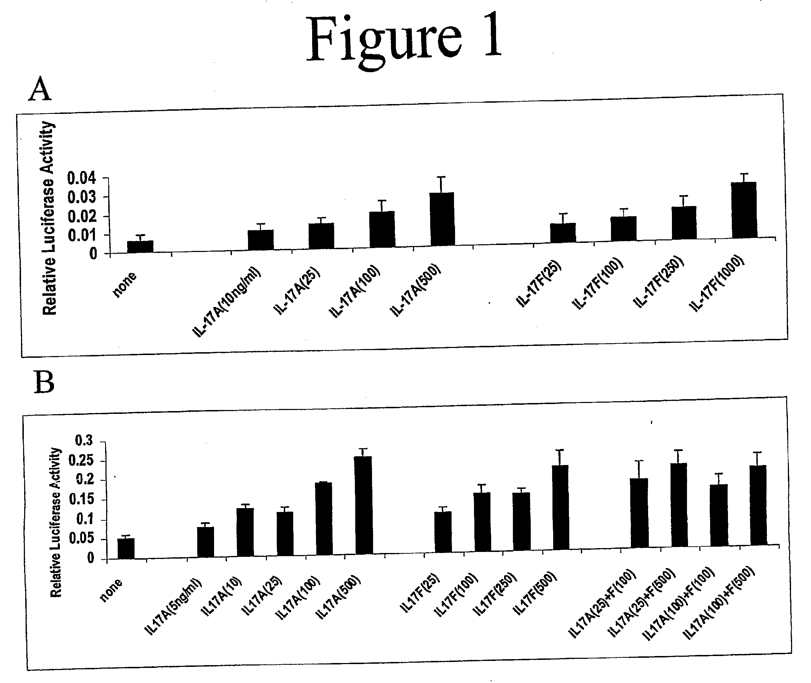

Shown in **Figure 1** is NF-κB-mediated reporter transactivation (Relative Luciferase Activity; y-axes) in **(A)** primary human chondrocytes or **(B)** primary porcine chondrocytes cultured in various concentrations (ng/ml) of IL-17A and/or IL-17F (x-axes).

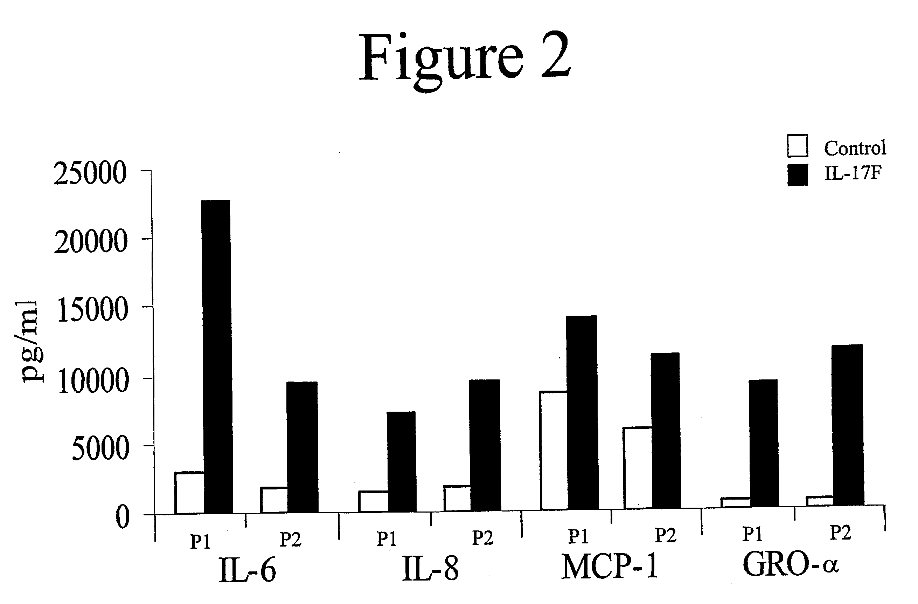

The concentration (pg/ml; y-axis) of cytokines (IL-6, IL-8, MCP-1 or GRO-α; x-axis) from each of two patients (P1, P2; x-axis) in supernatant collected from human fibroblast-like synoviocytes cultured in media (control; 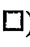 or in the presence of 20 ng /ml IL-17F (IL-17F; ■) is shown in Figure 2.

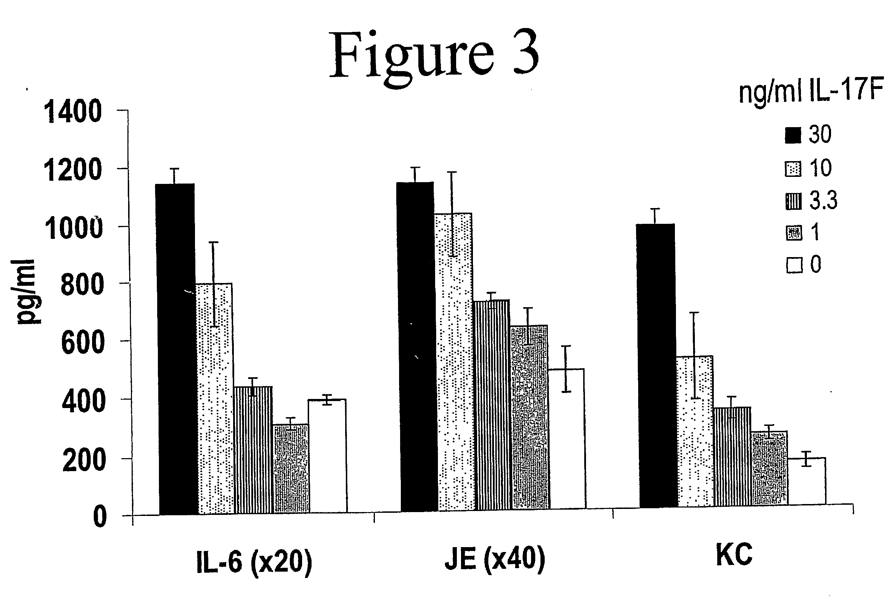

**Figure 3** demonstrates the concentration (pg/ml; y-axis) of inflammatory cytokines (IL-6, JE (CCL2), KC; x-axis) in supernatants collected from cultures of primary murine lung fibroblasts cultured in media (0 ng/ml IL-17F;□), or with 1 ng/ml ( 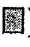 ), 3.3. ng/ml ( 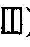 ), 10 ng/ml ( 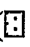 ), or 30 ng/ml (■) IL-17F.

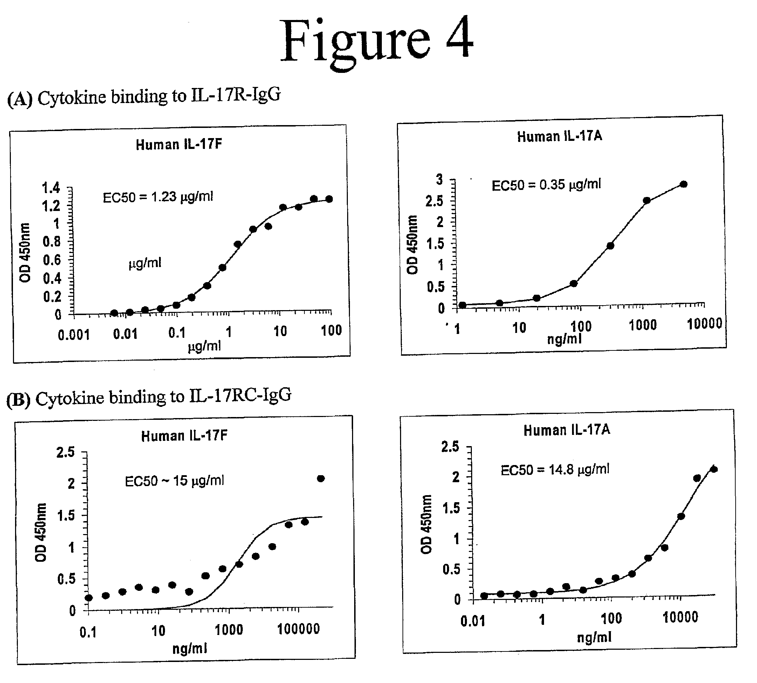

**Figure 4** demonstrates binding (OD 450nm; y-axes) of increasing concentrations of human IL-17F (left panels) or human IL-17A (right panels) (x-axes) to **(A)** IL-17R-IgG (upper panels) or **(B)** IL-17RC-IgG (lower panels) as measured by ELISA. Also noted are EC₅₀ values for each receptor/cytokine interaction.

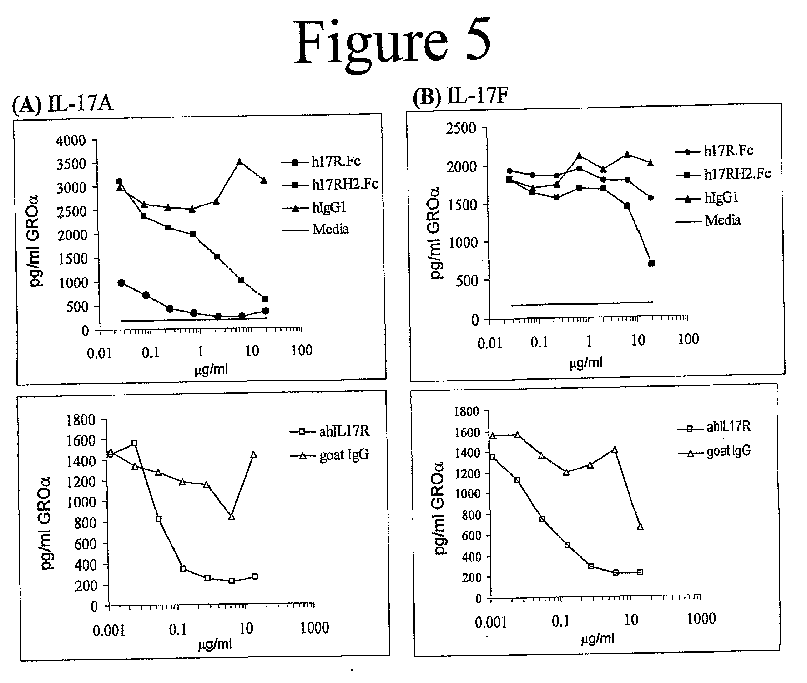

Shown in **Figure 5** is the concentration of GRO-α (pg/ml; y-axes) in supernatant collected from human fibroblasts cultured alone (Media; -) or with increasing concentrations (µg/ml; x-axes) of an IL-17R-IgG fusion protein (h17R.Fc; •), an IL-17RC-IgG fusion protein (h17RH2.Fc; ■), a control IgG protein (hIgG1;▲)**,** an anti-IL-17R antibody (ahIL17R; D) or control antibody (goat IgG;△) in the presence of either **(A)** 0.5 ng/ml IL-17A (left panels) or **(B)** 20 ng/ml IL-17F (right panels).

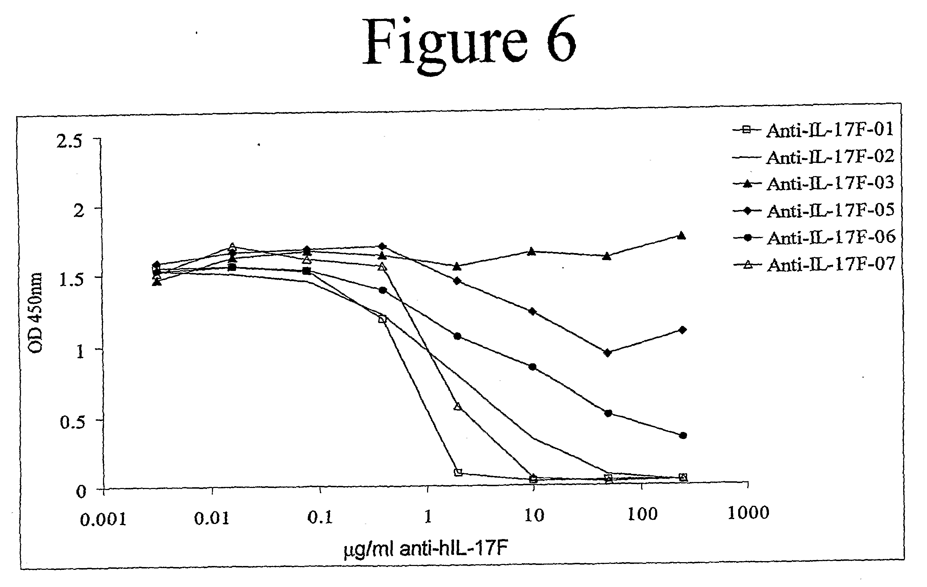

**Figure 6** demonstrates the ability of anti-human IL-17F antibodies to inhibit the binding of IL-17F to IL-17R (OD 450nm; y-axis) in the presence of increasing concentrations (µg/ml; x-axis) of one of the following six anti-IL-17F antibodies: anti-IL-17F-01 (□), anti-IL-17F-02 (-), anti-IL-17F-03 (▲), anti-IL-17F-05 (◆), anti-IL-17F-06 (•), and anti-IL-17F-07 (△).

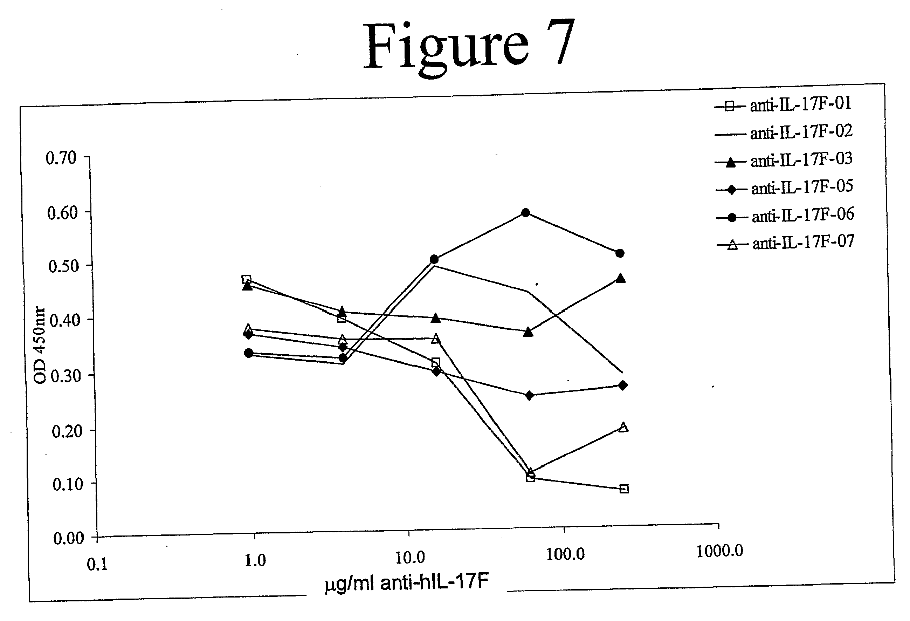

**Figure 7** demonstrates the ability of anti-human IL-17F antibodies to inhibit the binding of IL-17F to IL-17RC (OD 450nm; y-axis) in the presence of increasing concentrations (µg/ml; x-axis) of each of the following six anti-IL-17F antibodies: anti-IL-17F-O1 (□), anti-IL-17F-02 (-), anti-IL-17F-03 (▲), anti-IL-17F-05 (◆), anti-IL-17F-06 (•), and anti-IL-17F-07 (△).

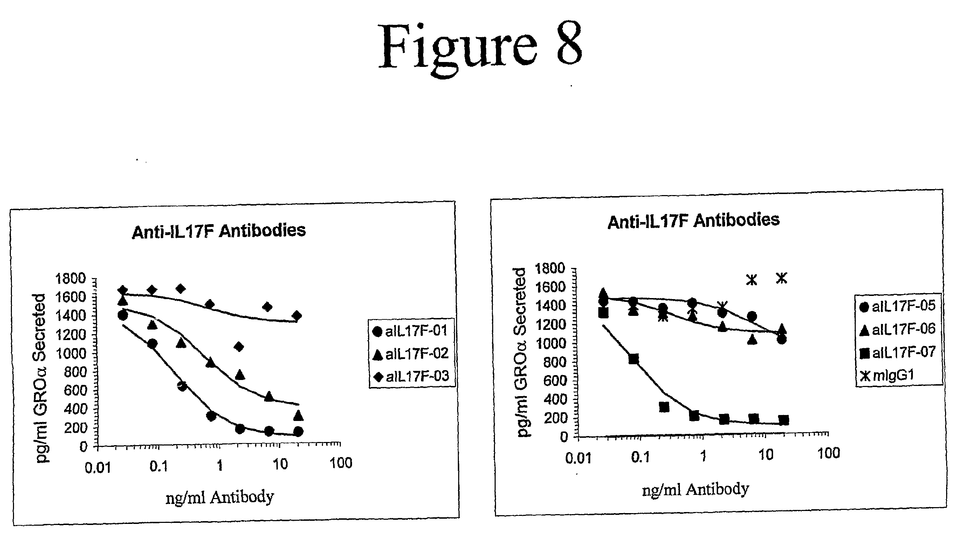

Shown in **Figure 8** is the concentration of GRO-α (pg/ml; y-axes) in supernatant collected from human fibroblasts cultured in 20 ng/ml IL-17F and increasing concentrations (µg/ml; x-axis) of (left panel) anti-IL-17F-01 (aIL-17F-01), anti-IL-17F-02 (aIL-17F-02), or anti-IL-17F-03 (aIL-17F-03) and (right panel) anti-IL-17F-05 (aIL-17F-05), anti-IL-17F-06 (aIL-17F-06), or anti-IL-17F-07 (aIL-17F-07), or control mIgG1 antibodies.

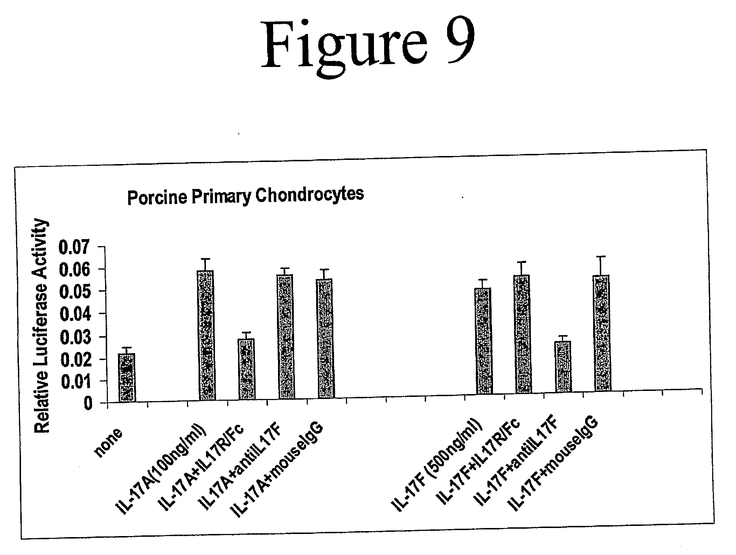

Shown in **Figure 9** is NF-κB-mediated reporter transactivation (Relative Luciferase Activity; y-axis) in porcine primary chondrocytes cultured in media only (none), in 100 ng/ml IL-17A (IL-17A(100 ng/ml)), in 100 ng/ml IL-17A in the presence of an IL-17R-IgG fusion protein (IL-17A+IL17R/Fc), in 100 ng/ml IL-17A in the presence of an anti-IL-17F antibody (IL17A+antiIL17F), in100 ng/ml IL-17A in the presence of a control mouse IgG (IL-17A+mouseIgG), in 500 ng/ml IL-17F (IL-17F(500 ng/ml)), in 500 ng/ml IL-17F in the presence of an IL-17R-IgG fusion protein (IL-17F+IL17R/Fc), in 500 ng/ml IL-17F in the presence of an anti-IL-17F antibody (IL-17F+antiIL17F), or in 500 ng/ml IL-17F in the presence of a control mouse IgG (IL-17F+mouse IgG).

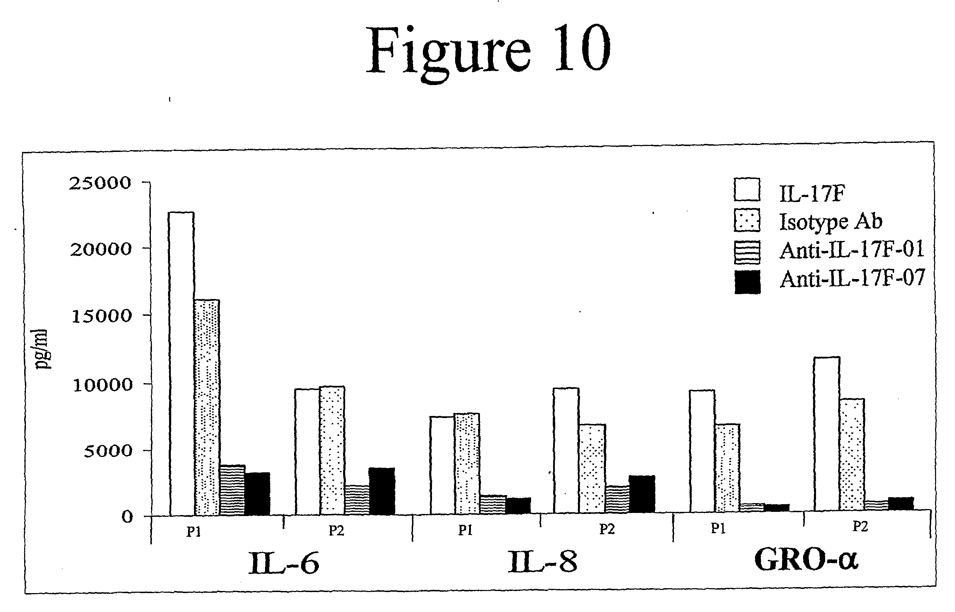

The concentration (pg/ml; y-axis) of cytokines (IL-6, IL-8, or GRO-α; x-axis) from each of two patients (P1, P2; x-axis) in supernatant collected from human fibroblast-like synoviocytes cultured in the presence of 20 ng/ml IL-17F (IL-17F; D), an isotype control antibody (Isotype Ab; 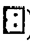 ), Anti-IL-17F-01 antibody ( 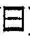 ), or Anti-IL-17F-07 antibody (■) is shown in Figure 10.

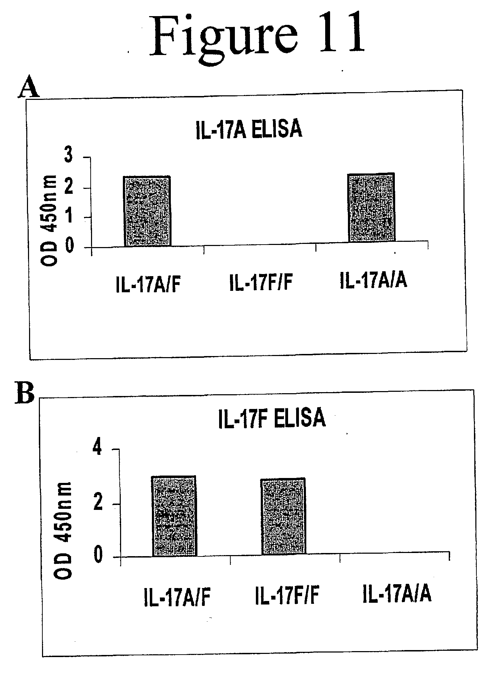

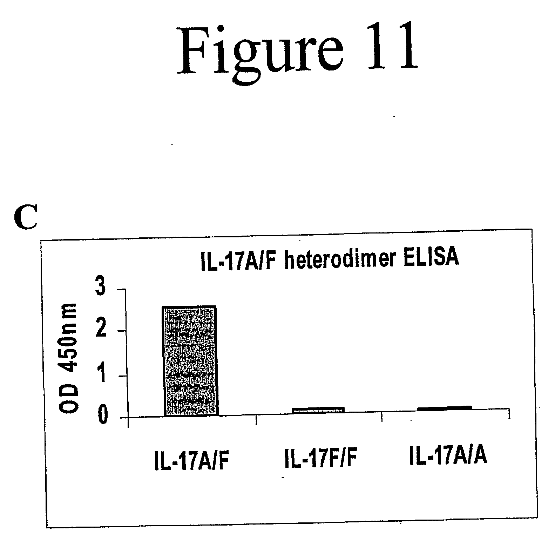

**Figure 11** demonstrates the detection (OD 450nm; y-axes) of IL-17A homodimers (IL-17A/A; x-axes), IL-17F homodimers (IL-17F/F; x-axes), or IL-17A/IL-17F heterodimers (IL-17A/F; x-axes) using ELISA formats specific for the detection of **(A)** IL-17A protein (including IL-17A homodimers and IL-17A heterodimers) **(B)** IL-17F protein (including IL-17F homodimers and IL-17F heterodimers), or **(C)** IL-17A/IL-17F heterodimers.

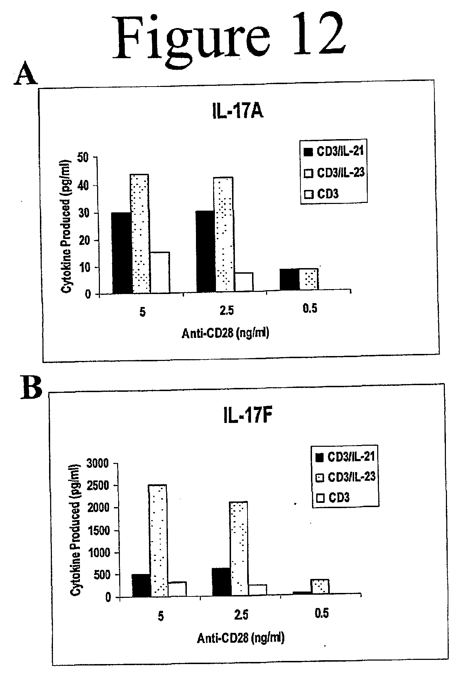

**Figure 12** demonstrates the concentration (Cytokine Produced (pgfml); y-axes) of **(A)** IL-17A or **(B)** IL-17F in media isolated from T cells undergoing primary activation in the presence of bead-bound anti-CD3 antibody, increasing concentrations of anti-CD28 antibody (Anti-CD28 (ng/ml); x-axes), and in the absence (□) or presence of IL-21 (■) or IL-23 ( 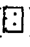 ).

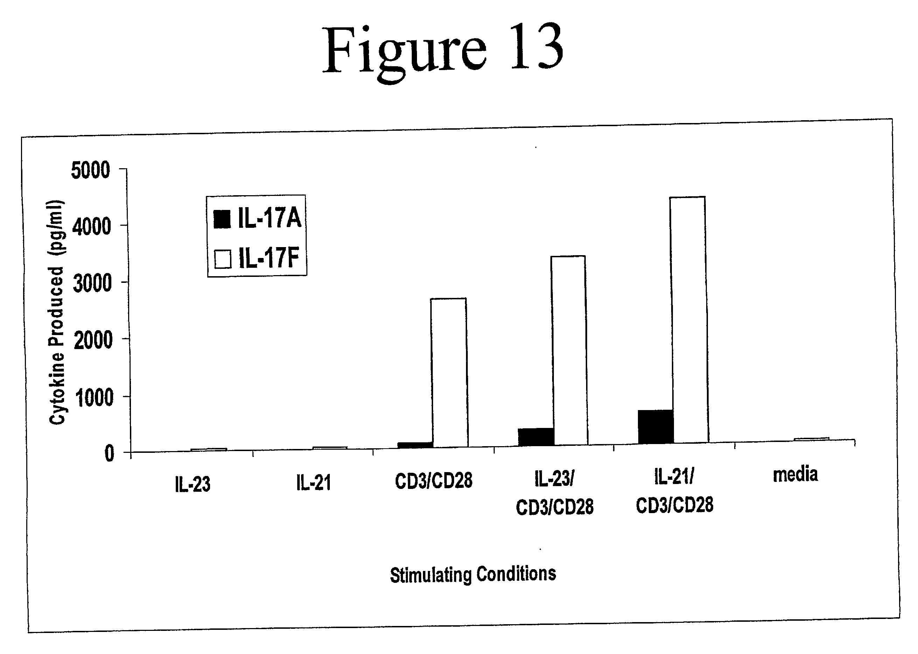

**Figure 13** demonstrates the concentration (Cytokine Produced (pg/ml); y-axis) of IL-17A (■) or IL-17F (□) in media isolated from T cells undergoing secondary activation under the following stimulating conditions (x-axis): IL-23 only (IL-23); IL-21 only (IL-21); bead-bound anti-CD3 antibody and anti-CD28 antibody (CD3/CD28); IL-23, bead-bound anti-CD3 antibody and anti-CD28 antibody (IL-23/CD3/CD28); IL-21, bead-bound anti-CD3 antibody and anti-CD28 antibody (IL-21/CD3/CD28); or media.

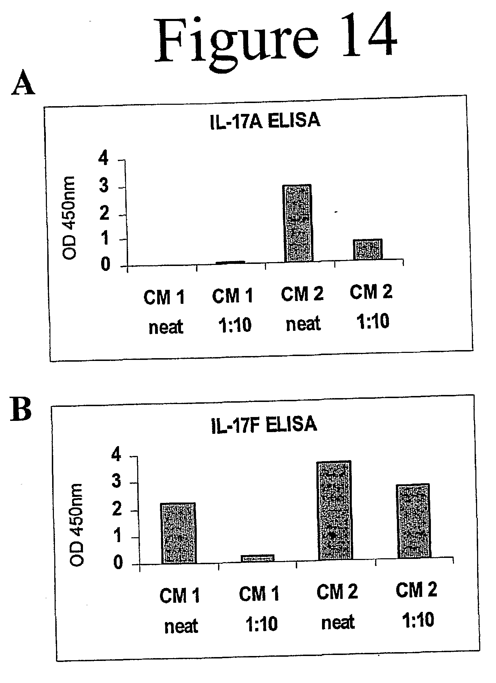

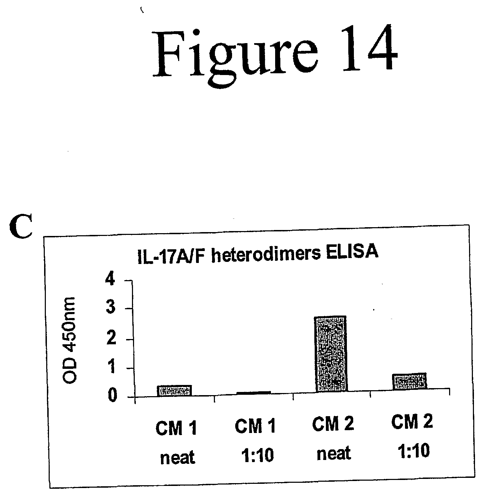

**Figure 14** demonstrates the detection (OD 450nm; y-axes) of IL-17A homodimers, IL-17F homodimers, or IL-17A/IL-17F heterodimers in undiluted (neat) or diluted (1:10) media obtained from T cells subject to primary activation (CM1) or restimulation (CM2) (x-axes) using ELISA formats specific for the detection of **(A)** IL-17A protein (including IL-17A homodimers and IL-17A heterodimers), **(B)** IL-17F protein (including IL-17F homodimers and IL-17F heterodimers), or **(C)** IL-17A/IL-17F heterodimers.

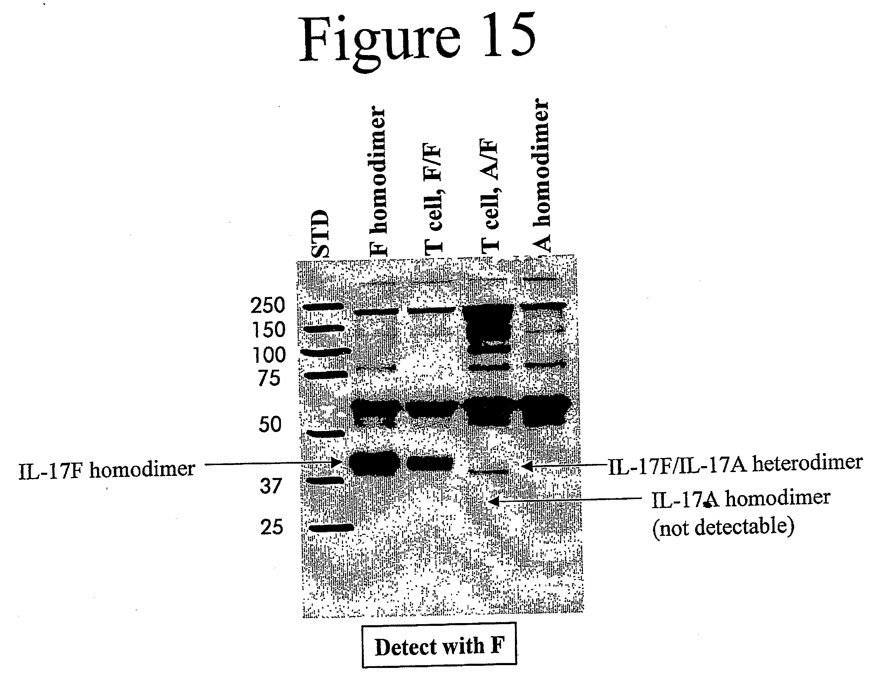

Shown in **Figure 15** is a Western blot analysis performed with polyclonal rabbit anti-human IL-17F antibody to detect anti-human IL-17F-01 immunoprecipitates from 500 µl of conditioned media obtained from T cells undergoing secondary activation. Controls consist of IL-17F homodimer (second lane) prepared as described in Example 5.3, or IL-17A homodimers (fifth lane) purchased from R&D Systems (Minneapolis, MN). The molecular weight standard is shown in first lane. The positions of the IL-17A and IL-17F homodimers and IL-17F/IL-17A heterodimers are indicated by arrows.

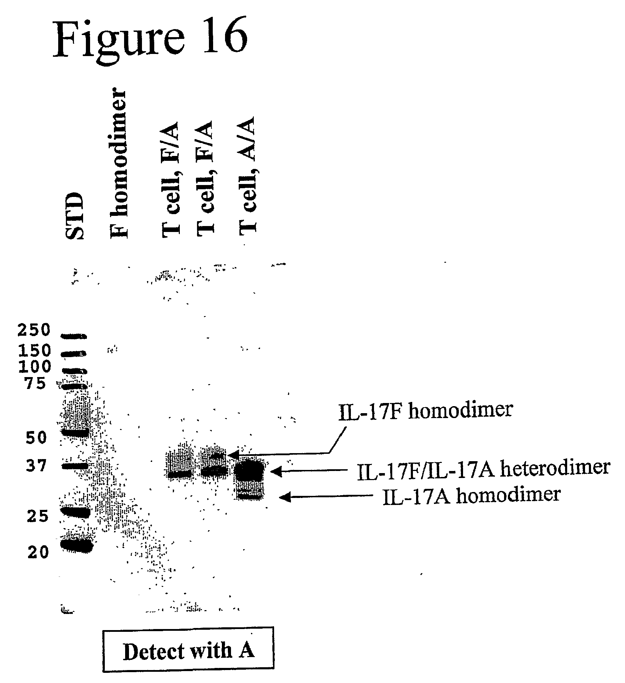

**Figure 16** is the result of a Western blot analysis performed with biotin-conjugated goat anti-human IL-17A antibody to detect the anti-human IL-17A-02 immunoprecipitates from 500 µl of conditioned media obtained from T cells undergoing secondary activation. Control (lane 2) consists of IL-17F homodimer prepared as described in Example 5.3. The molecular weight standard is shown in lane 1. The positions of the IL-17A and IL-17F homodimers and IL-17F/IL-17A heterodimers are indicated by arrows.

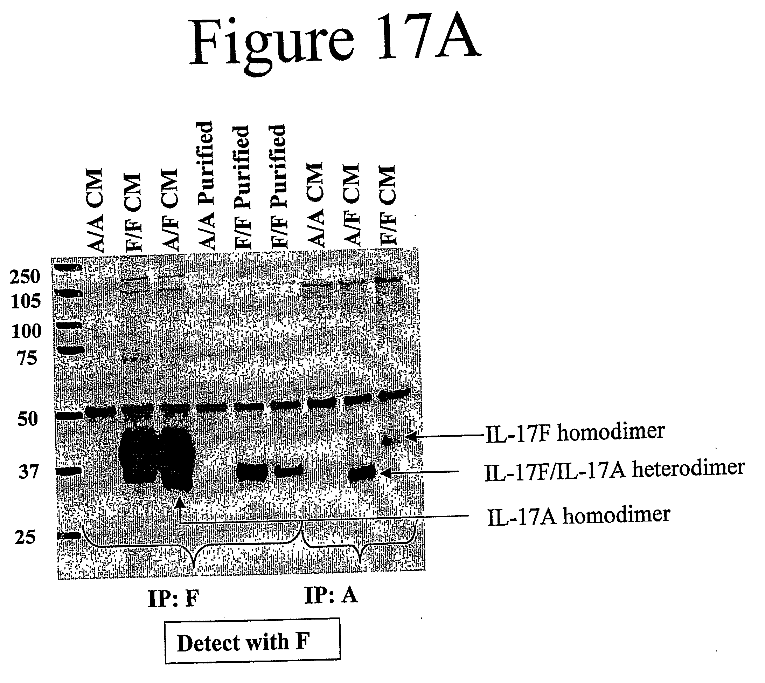

**Figure 17A** shows anti-IL-17F immunoprecipitates (lanes 2-7) or anti-IL-17A immunoprecipitates (lanes 8-10) immunoprobed with anti-IL-17F antibody. Immunoprecipitates were obtained from the conditioned media (CM) of COS cells overexpressing IL-17A (lanes 2 and 8), IL-17F (lanes 3 and 10), IL-17A and IL-17F (lanes 4 and 9), purified IL-17A homodimer (lane 5), or purified IL-17F homodimer (lanes 6 and 7). Controls ("A/A Purified," lane 5, and "F/F purified," lanes 6-7) consist of purified recombinant IL-17A and IL-17F homodimers as described in Example 5.4. The molecular weight standard is shown in lane 1. The positions of the IL-17A and IL-17F homodimers and IL-17F/IL-17A heterodimer are indicated by arrows.

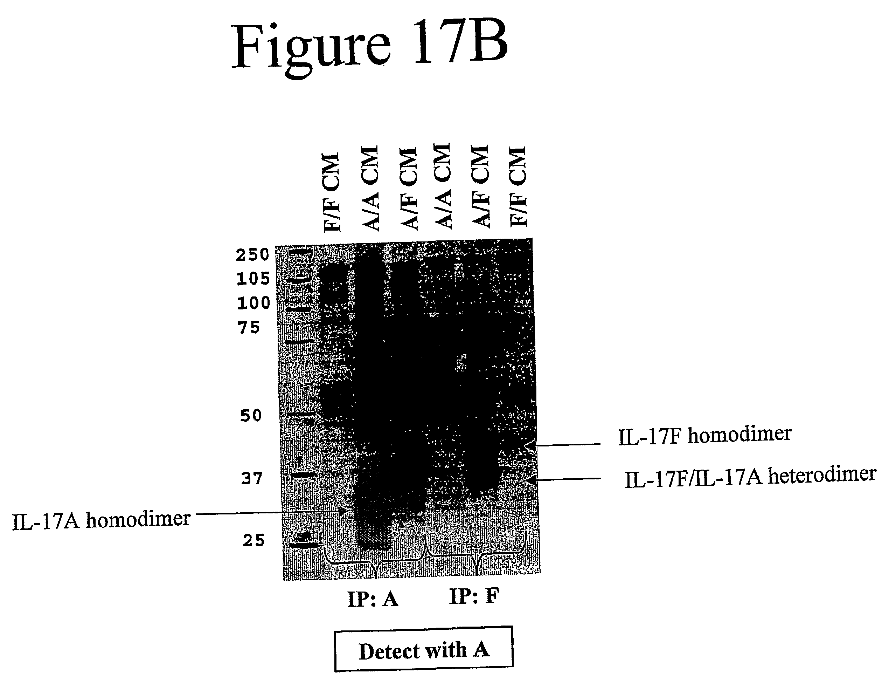

**Figure 17B** shows anti-IL-17A immunoprecipitates (lanes 2-4) or anti-IL-17F immunoprecipitates (lanes 5-7) immunoprobed with anti-IL-17A antibody. Immunoprecipitates were obtained from the conditioned media (CM) of COS cells overexpressing IL-17A (lanes 3 and 5), IL-17F (lanes 2 and 7), or IL-17A and IL-17F (lanes 4 and 6). The molecular weight standard is shown in lane 1. The positions of the IL-17A and IL-17F homodimers and IL-17F/IL-17A heterodimer are indicated by arrows.

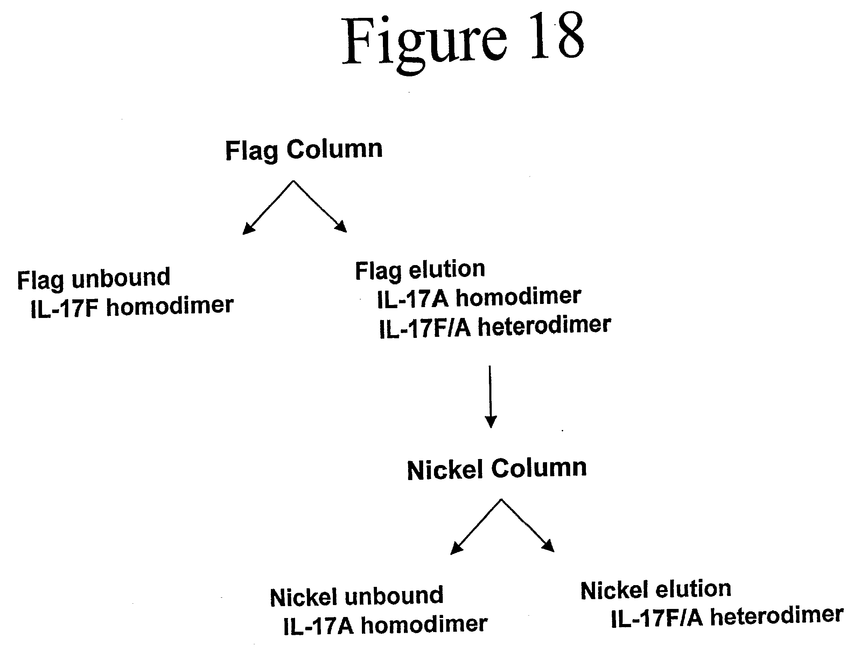

**Figure 18** is a diagram showing a method of purifying recombinant IL-17F/IL-17A heterodimers substantially free from IL-17A and IL-17F homodimers. The method employs IL-17A and IL-17F with two different affinity tags, and uses two separate and sequential affinity columns to isolate IL-17F/IL-17A heterodimers.

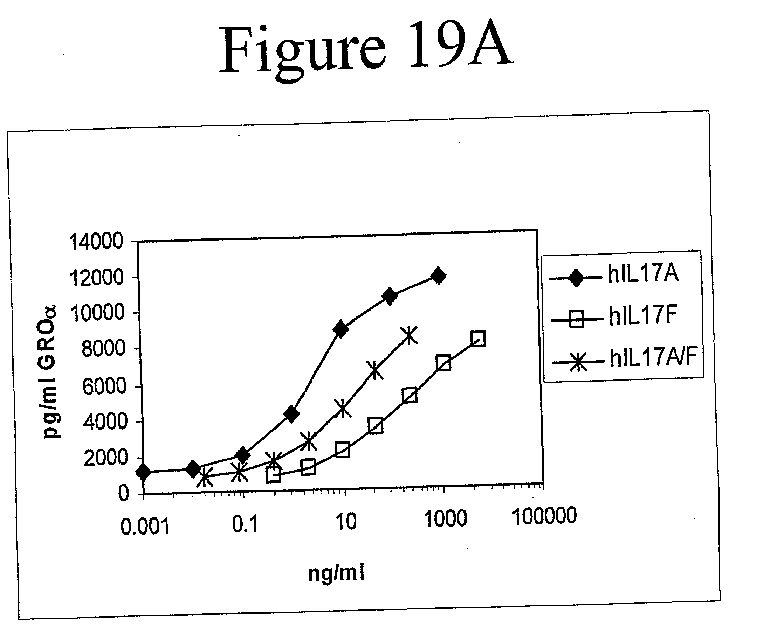

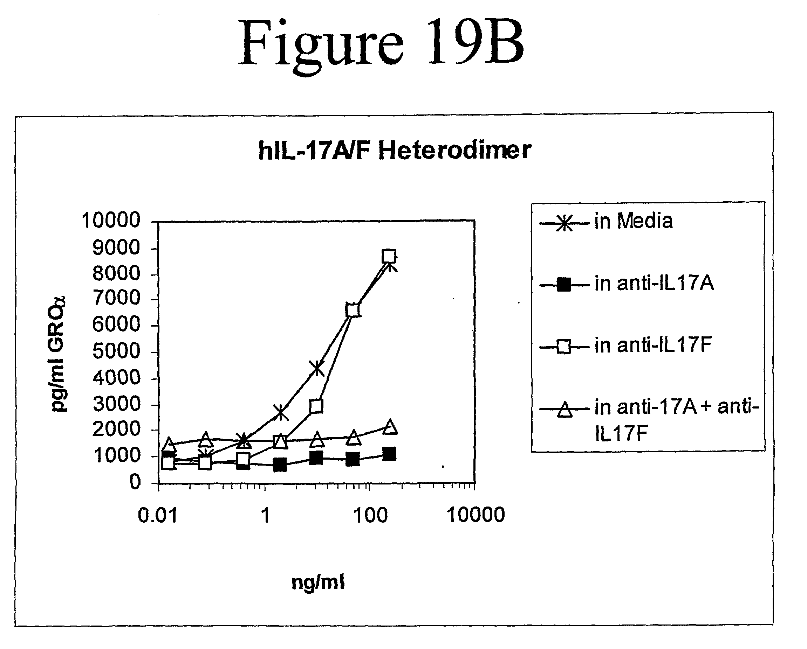

**Figure 19A** shows that recombinant purified IL-17F/IL-17A heterodimers (X), similar to IL-17A(◆) and IL-17F (□) homodimers, stimulate GRO-α levels (pg/ml) in the media ofBJ cell cultures. **Figure 19B** shows that cotreatment of BJ cultures with anti-IL-17A antibody (■), or anti-IL-17A in combination with, anti-IL-17F antibodies (△), but not IL-17F antibodies alone (□), abrogates IL-17F/IL-17A heterodimer stimulation of GRO-α levels. Controls consisted of cultures provided with media lacking both IL-17F and IL-17A antibodies (X).

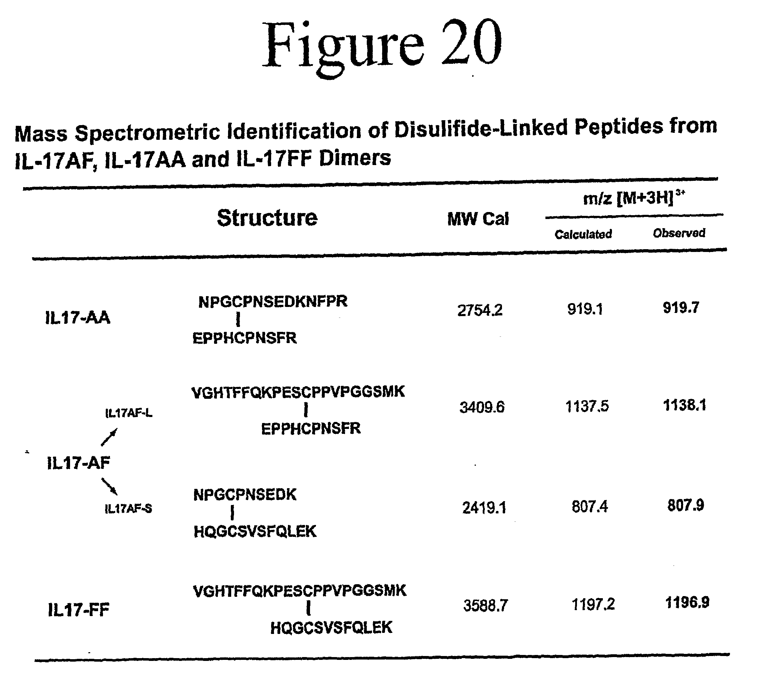

**Figure 20** is a table summarizing MALDI-TOF mass spectrometry data for tryptic peptide masses prepared by digestion of IL-17F homodimers, IL-17A homodimers, and IL-17F/IL-17A heterodimers. The first column of the table shows the origin of the peptide fragment analyzed, the second column (Structure) shows the peptide fragment sequence, the third column (MW Cal) shows the calculated molecular weight of the fragment, the fourth column shows the calculated mass-to-charge ratio (m/z value) of the fragment (Calculated), and the fifth column shows the actual mass-to-charge ratio (m/z value) (Observed) as determined by mass spectrometry.

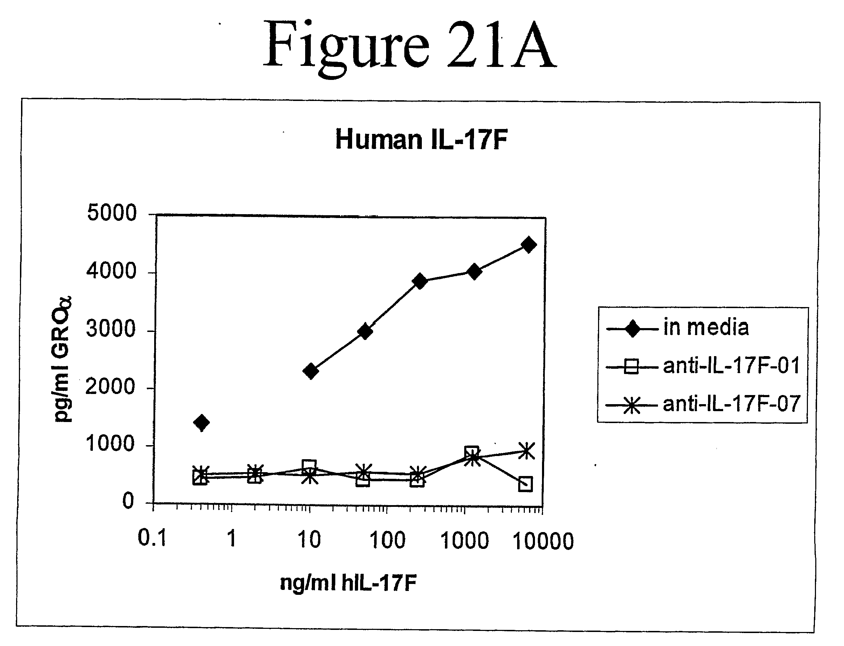

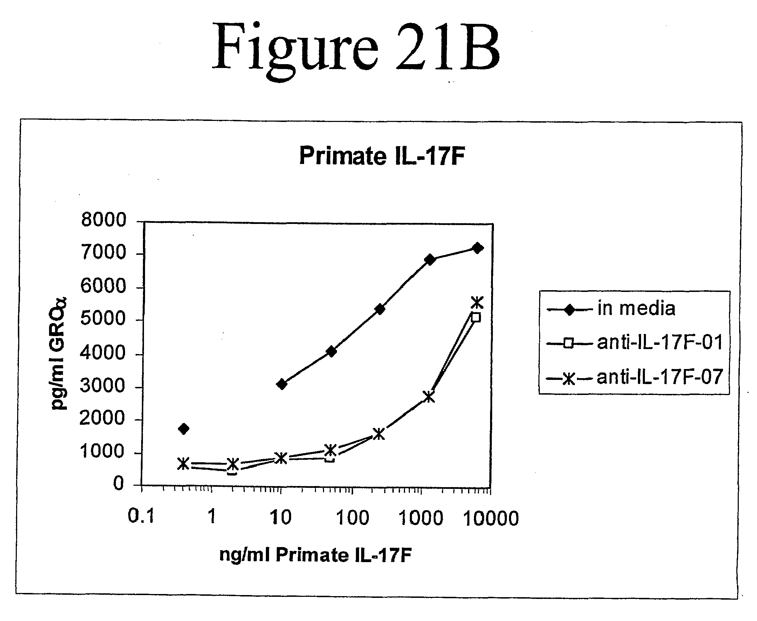

**Figure 21** shows that anti-human IL-17F antibodies can partially inhibit the biological activity of primate IL-17F. **Figure 21A** and **21B** show that BJ cells stimulated with human or primate (macaque) IL-17F display increased levels of GRO-α in response to increasing levels of IL-17F. **Figure 21A** shows that anti-IL-17F-01 (□) and anti-IL-17F-07 (X) antibodies decrease the ability of human IL-17F (◆) to stimulate GRO-α levels. Similarly, **Figure 21B** shows that anti-IL-17F-01 (□) and anti-IL-17F-07 (X) antibodies decrease the ability of primate IL-17F (◆) to stimulate GRO-α levels, albeit to a lesser extent than the antibodies reduce human IL-17F biological activity.

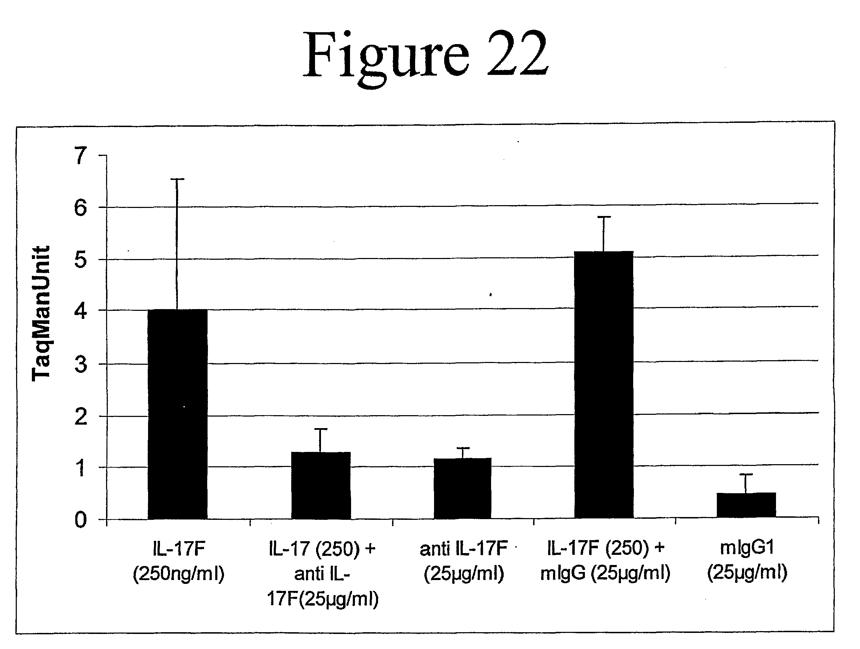

**Figure 22** shows that IL-17F treatment increases the expression of ADAMTS-4 (Aggrecanase 1) in chondrocytes obtained from human donors, and that treatment with anti-IL-17F antibodies abrogates this stimulation. Cultured chondrocytes were treated with 250 ng/ml IL-17F, 250 ng/ml IL-17F and 25 µg/ml anti-IL-17F, 25 µg/ml anti-IL-17F, 250 ng/ml IL-17F and 25 µg/ml control IgG1, or 25 µg/ml control IgG1 (x-axis), and transcript levels of Aggrecanase 1 measured by real-time PCR (expressed as TAQMAN® units; y-axis). GAPDH expression levels were used as normalizer.

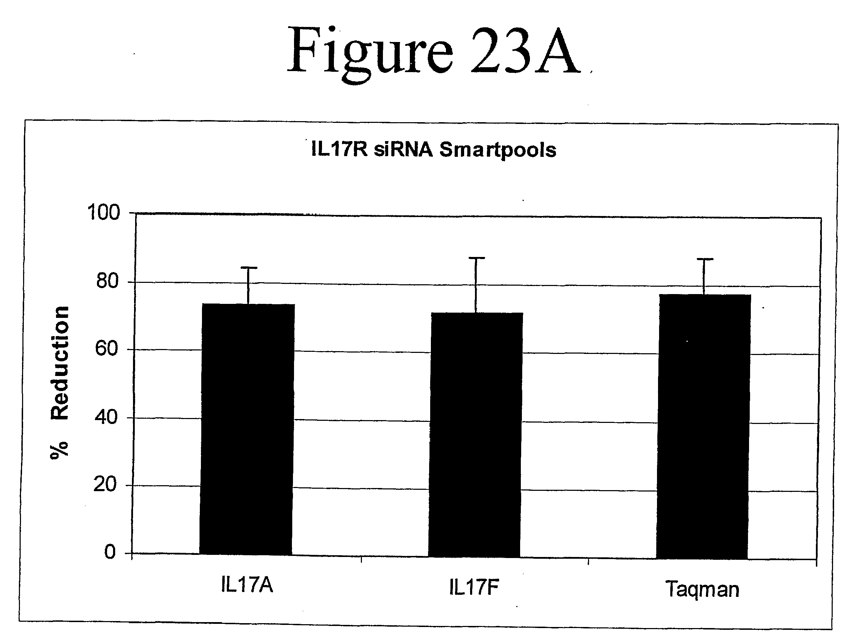

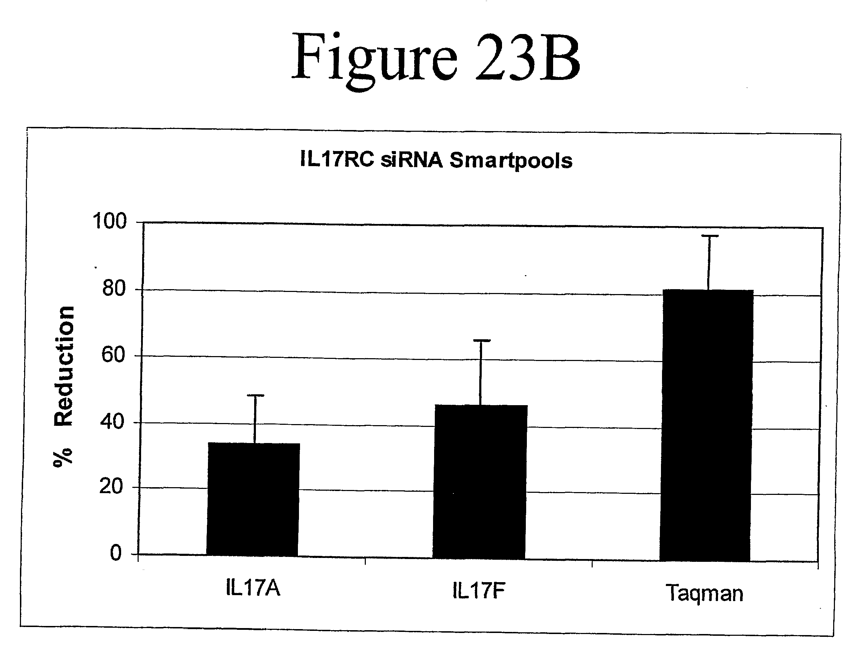

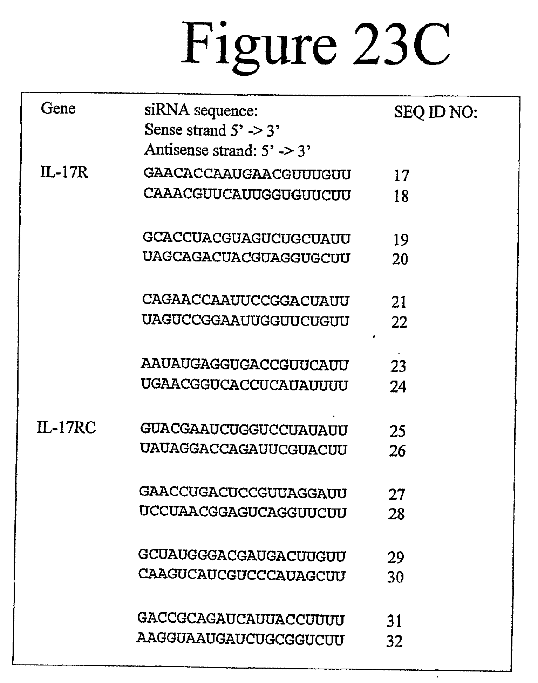

**Figure 23** shows that treatment ofBJ cells with siRNA directed to transcripts of IL-17R and IL-17RC reduces the ability of IL-17F and IL-17A to increase GRO-α levels **Figure 23A****:** Taqman = % reduction in IL-17R transcript levels in cells treated with siRNA to IL-17R; IL-17F = % reduction in the ability of IL-17F to stimulate GRO-α levels in cells treated with siRNA to IL-17R; IL-17A = % reduction in the ability of IL-17A to stimulate GRO-α levels in cells treated with siRNA to IL-17R. **Figure 23B** shows that treatment of BJ cells with siRNA directed to transcripts of IL-17RC reduces the ability of IL-17F and IL-17A to increase GRO-α levels. Taqman = % reduction in IL-17RC transcript levels in cells treated with siRNA to IL-17RC; IL-17F = % reduction in the ability of IL-17F to stimulate GRO-α levels in cells treated with siRNA to IL-17RC; IL-17A = % reduction in the ability of IL-17A to stimulate GRO-α levels in cells treated with siRNA to IL-17RC. **Figure 23C** discloses several siRNA molecules of the present invention (SEQ ID NOs:17-32) that target mRNA polynucleotides related to the present invention (i.e., IL-17R and IL-17RC).

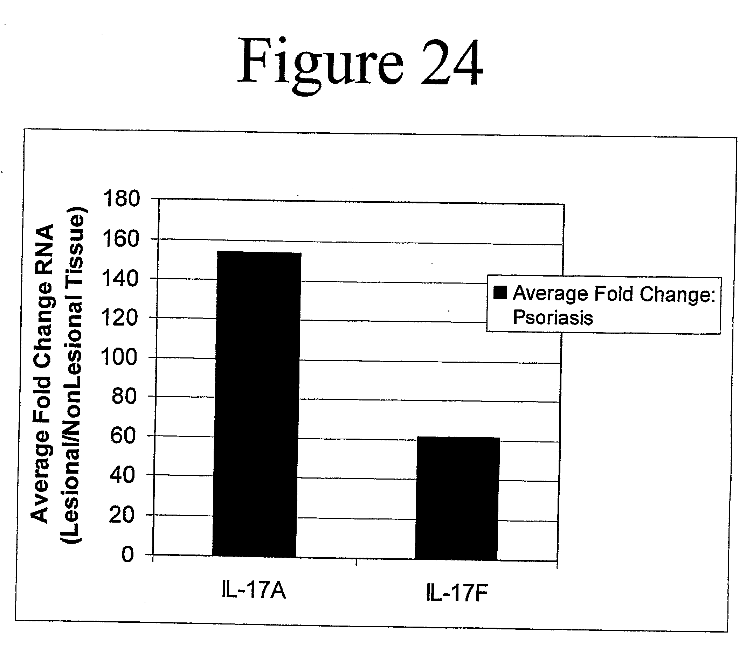

**Figure 24** shows the average fold-change (lesional / nonlesional (nonaffected) tissues) of IL-17F and IL-17A transcript expression in 48 pairs of tissue biopsy samples from patients suffering from psoriasis. Both IL-17A and IL-17F transcript levels are increased in psoriatic lesional tissues with respect to nonaffected tissue. P-values from paired t-tests are as follows: IL-17A p= 2.8 x 10⁻¹³, IL_17F p=1.1 x 10⁻⁹.

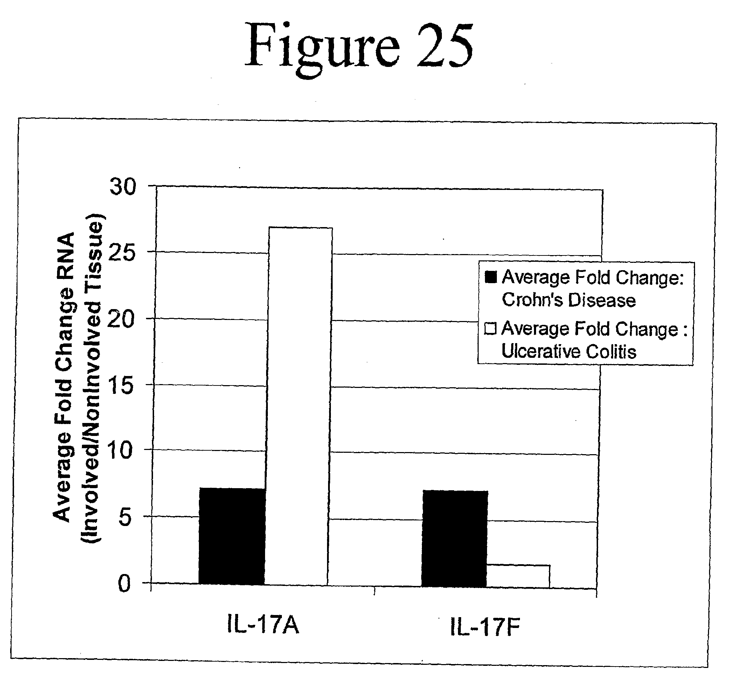

**Figure 25** shows the average fold-change (involved / noninvolved tissues) of IL-17F and IL-17A transcript expression in paired tissue biopsy samples from patients suffering from ulcerative colitis (UC) (□) (12 pairs) or Crohn's disease (CD) (■) (16 pairs). Both IL-17A and IL-17F transcript levels are increased in affected tissues relative to noninvolved tissues in both sets of IBD samples. P-values from paired t-tests are as follows: IL-17A (UC), p=0.309; IL-17A (CD), p=0.069; IL-17F (UC), p=0.406; IL-17F (CD), p=0.206.

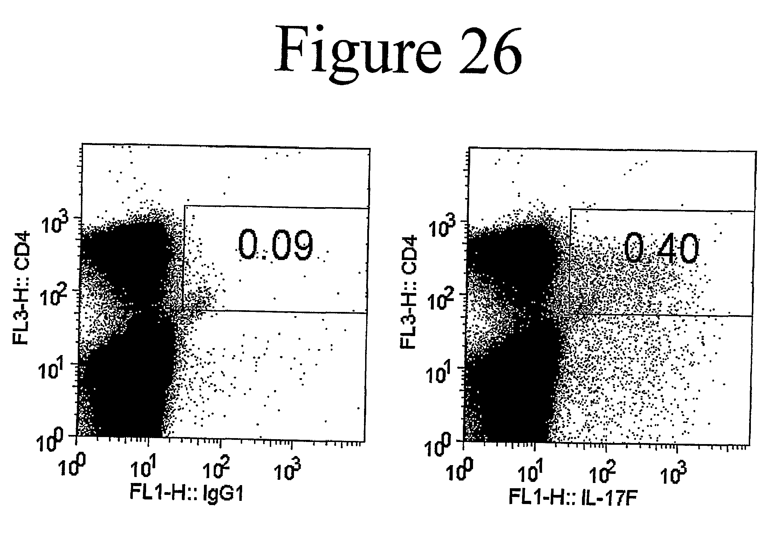

**Figure 26** shows intracellular cytokine staining for IL-17F. Staining for IL-17F was performed on (lymph node) LN cells from C57BL/6 mice immunized with 100 µg ovalbumin emulsified in complete Freund's adjuvant. Cells were surface-stained for CD4, fixed, permeabilized and stained with an anti-IgG1 isotype control or with rat anti-murine IL-17F (clone 15-1). Numbers denote percent of positive cells.

### DETAILED DESCRIPTION OF THE INVENTION

Interleukin-17F (IL-17F) is a cytokine that belongs to the IL-17 family of proteins and induces expression of inflammatory cytokines and chemokines, e.g., IL-6, IL-8, GM-CSF, G-CSF, GRO-α, MCP-1, IL-1β, TNF-α, TGF-β, etc. Expression of IL-17F is correlated with neutrophilia and various autoimmune diseases (Bettelli and Kuchroo, *supra).* For example, IL-17F is associated with increased proteoglycan breakdown and decreased proteoglycan synthesis by articular cartilage (Hymowitz, *supra),* central nervous system autoimmunity (Langrish, *supra),* allergic and asthmatic responses (Kawaguchi, *supra)* and inflammatory bowel diseases (Gurney, *supra).* Thus, IL-17F signaling is believed to be involved with disorders including, but not limited to, inflammatory disorders, such as autoimmune diseases (e.g:, arthritis (including rheumatoid arthritis), psoriasis, systemic lupus erythematosus (SLE), multiple sclerosis), respiratory diseases (e.g., COPD, cystic fibrosis, asthma, allergy), transplant rejection (including solid organ transplant rejection), and inflammatory bowel diseases (e.g., ulcerative colitis, Crohn's disease).

As part of the invention, the inventors have confirmed involvement of IL-17F in inflammatory disorders by demonstrating the following responses to administration of IL-17F: e.g., neutrophil influx into the peritoneum (Example 1.1), activation of a primary transcription factor of inflammatory cytokines correlated with an increased secretion of inflammatory cytokines by primary chondrocytes (Example 1.2), increased secretion of inflammatory cytokines by lung fibroblasts (Example 1.3), and increased levels of Aggrecanase in primary human chondrocytes (Example 7). The inventors have also determined that both IL-17F and IL-17A may be involved in autoimmune arthritis (Example 7), psoriasis (Example 9) and inflammatory bowel disease (IBD) (Example 9). The inventors have also identified IL-17R and IL-17RC as receptors for IL-17F (Example 2), thus providing novel targets for inhibition of the IL-17F signaling pathway. The inventors have also generated and characterized anti-IL-17F antibodies in terms of each antibody's binding specificity, affinity, and ability to inhibit IL-17F signaling, i.e., IL-17F bioactivity (Examples 3 and 5). In one embodiment, antibodies help to characterize IL-17F epitopes that may be required for IL-17R and/or IL-17RC recognition; i.e., five of six murine anti-human IL-17F antibodies are able to interfere with binding of IL-17F to IL-17R, and two of the five are also able to interfere with binding of IL-17F to IL-17RC. The inventors have also demonstrated the ability of some of these antibodies to inhibit (i.e., decrease, limit, block, or otherwise reduce) IL-17F bioactivities, e.g., IL-17F-mediated activation of a primary transcription factor for inflammatory cytokines, and subsequently, IL-17F-mediated cytokine secretion by primary fibroblast-like synoviocytes (Example 4). Also disclosed herein are inhibitory polynucleotides that decrease IL-17A and IL-17F signaling through the IL-17R and IL-17RC (Example 8). The inventors have also demonstrated a direct relationship between IL-21 and IL-17F, i.e., the ability of IL-21 to enhance the production of both IL-17A and IL-17F by activated T cells. Thus, it is reasoned that inhibition of IL-17F signaling may also inhibit at least one effect associated with IL-21 binding to and activation of IL-21R, e.g., methods of inhibiting IL-17F signaling may be used in methods of treating IL-17F-associated disorders and/or disorders associated with IL-21 binding to and activating IL-21R. The inventors also isolated for the first time IL-17A and IL-17F from the cytokines' natural source. The inventors have also demonstrated and purified a novel IL-17A/IL-17F heterodimer (e.g., in T cells, and HEK-293 and COS cells, respectively), and have shown that the heterodimer transduces IL-17F signaling, e.g., by inducing expression of GRO-α levels (Example 5). Thus the inventors have provided the heterodimer as a novel target for inhibition of the IL-17F-signaling pathway and/or in the treatment of inflammatory disorders and/or disorders associated with IL-21 binding to and activating IL-21R.

As such, the present invention provides IL-17F signaling antagonists, (e.g., IL-17F, IL-17R, and/or IL-17RC inhibitory polynucleotides; soluble IL-17R and/or IL-17RC polypeptides (including fragments (e.g., IL-17F binding fragments) and/or fusion proteins thereof); inhibitory anti-IL-17F, anti-IL-17R, or IL-17RC antibodies; and/or antagonistic small molecules), which may be used to suppress IL-17F-mediated (including IL-17F homodimer- and IL-17A/IL-17F heterodimer-mediated) inflammatory responses *in vivo,* and consequently, which may be used in the diagnosis, prognosis, monitoring and/or treatment of disorders related to increased IL-17F signaling, i.e., IL-17F-associated disorders and/or disorders associated with IL-21 binding to and activating IL-21R. The identification and isolation of the novel IL-17A/IL-17F heterodimer indicates that disorders related to IL-17F signaling may be mediated by IL-17F homodimers and/or IL-17F heterodimers. Thus the term "IL-17F" as used herein, where appropriate, refers to IL-17F homodimers or IL-17A/IL-17F heterodimers, e.g., the IL-17F signaling pathway encompasses a signaling pathway that may comprise either or both IL-17F homodimers and IL-17A/IL-17F heterodimers.

Accordingly, the present application provides IL-17F signaling-related polynucleotides and polypeptides, including IL-17R and IL-17RC polynucleotides and polypeptides. The present invention also provides antibodies, i.e., intact antibodies and antigen-binding fragments thereof, that bind to IL-17F, in particular, human IL-17F, including, but not limited to, IL-17F homodimers and IL-17A/IL-17F heterodimers. In one embodiment, an anti-IL-17F antibody inhibits or antagonizes at least one IL-17F-associated (e.g., IL-17F homodimer and/or IL-17A/IL-17F heterodimer) activity. For example, the anti-IL-17F antibody can bind to IL-17F and interfere with, e.g., block, an interaction between IL-17F and an IL-17F receptor complex, e.g., complexes comprising IL-17R and/or IL-17RC. Thus, the antibodies of the invention may be used detect, and optionally inhibit (e.g., decrease, limit, block or otherwise reduce), an IL-17F bioactivity, e.g., binding between IL-17F and an IL-17F receptor complex, or subunit thereof. Thus, the anti-IL-17F antibodies of the invention may be used to diagnose, prognose, monitor and/or treat or prevent disorders related to IL-17F signaling and/or disorders associated with IL-21 binding to and activating IL-21R.

### Polynucleotides and Polypeptides of IL-17F, IL-17R, and IL-17RC

The present invention provides further characterization of the IL-17F signaling pathway, i.e., determination of IL-17R and/or IL-17RC as an IL-17F receptor, elucidation of the effects of interfering with IL-17F binding to IL-17R and/or IL-17RC using inhibitory molecules, e.g., antibodies, receptor fusion proteins and siRNA, and the purification of IL-17A/IL-17F heterodimers. As such, the present invention relates to IL-17F, IL-17R, and IL-17RC polynucleotides and polypeptides, including inhibitory IL-17F, IL-17R and IL-17RC polynucleotides and polypeptides.

IL-17F nucleotide and amino acid sequences are known in the art and are provided. The nucleotide sequence of human IL-17F is set forth in SEQ ID NO:1. The amino acid sequence of full-length IL-17F protein coded by that nucleotide sequence is set forth in SEQ ID NO:2. The amino acid sequence of mature IL-17F corresponds to a protein beginning at about amino acid 31 of SEQ ID NO:2 (see, e.g., U.S. Patent Application No. 10/102,080, incorporated herein in its entirety by reference).

IL-17A nucleotide and amino acid sequences are known in the art and are provided. The nucleotide sequence of human IL-17A is set forth in SEQ ID NO:3, which includes a poly(A) tail. The amino acid sequence of full-length IL-17A protein corresponding to that nucleotide sequence is set forth in SEQ ID NO:4.

IL-17R nucleotide and amino acid sequences are known in the art and are provided. The nucleotide sequence of human IL-17R is set forth as SEQ ID NO:5, which includes a poly(A) tail. The amino acid sequence of full-length IL-17R protein corresponding to that nucleotide sequence is set forth in SEQ ID NO:6.

IL-17RC nucleotide and amino acid sequences are known in the art and are provided. The nucleotide sequences of several human IL-17RC polynucleotides, which include poly(A) tails, are set forth as SEQ ID NOs:7, 9, 11,13, and 15. The amino acid sequences of several full-length human IL-17RC proteins corresponding to those nucleotide sequences are set forth in SEQ ID NOs:8, 10, 12, 14, and 16.

The nucleic acids related to the present invention may comprise DNA or RNA and may be wholly or partially synthetic. Reference to a nucleotide sequence as set forth herein encompasses a DNA molecule with the specified sequence (or a complement thereof), and encompasses an RNA molecule with the specified sequence in which U is substituted for T, unless context requires otherwise.

The isolated polynucleotides related to the present invention may be used as hybridization probes and primers to identify and isolate nucleic acids having sequences identical to or similar to those encoding the disclosed polynucleotides. Hybridization methods for identifying and isolating nucleic acids include polymerase chain reaction (PCR), Southern hybridization, *in situ* hybridization and Northern hybridization, and are well known to those skilled in the art.

Hybridization reactions may be performed under conditions of different stringency. The stringency of a hybridization reaction includes the difficulty with which any two nucleic acid molecules will hybridize to one another. Preferably, each hybridizing polynucleotide hybridizes to its corresponding polynucleotide under reduced stringency conditions, more preferably stringent conditions, and most preferably highly stringent conditions. Examples of stringency conditions are shown in Table 1 below: highly stringent conditions are those that are at least as stringent as, for example, conditions A-F; stringent conditions are at least as stringent as, for example, conditions G-L; and reduced stringency conditions are at least as stringent as, for example, conditions M-R.

**Table 1. Stringency Conditions**

| Stringency Condition | Polynucleotide Hybrid | Hybrid Length (bp)¹ | Hybridization Temperature and Buffer² | Wash Temperature and Buffer² |
|---|---|---|---|---|
| A | DNA:DNA | > 50 | 65°C; 1xSSC -or- | 65°C; 0.3xSSC |
| | | | 42°C; 1xSSC, 50% formamide | |
| B | DNA:DNA | <50 | T_{B}*; 1xSSC | T_{B}*; 1xSSC |
| C | DNA:RNA | 50 | 67°C; 1xSSC -or- | 67°C; 0.3xSSC |
| | | | 45°C; 1xSSC, 50% formamide | |
| D | DNA:RNA | <50 | T_{D}*; 1xSSC | T_{D}*; 1xSSC |
| E | RNA:RNA | >50 | 70°C; 1xSSC -or- | 70°C; 0.3xSSC |
| | | | 50°C; 1xSSC, 50% formamide | |
| F | RNA:RNA | <50 | T_{F}*; 1xSSC | T_{F}*; 1xSSC |
| G | DNA:DNA | >50 | 65°C; 4xSSC -or- | 65°C; 1xSSC |
| | | | 42°C; 4xSSC, 50% formamide | |
| H | DNA:DNA | <50 | T_{H}*; 4xSSC | T_{H}*; 4xSSC |
| I | DNA:RNA | >50 | 67°C; 4xSSC -or- | 67°C; 1xSSC |
| | | | 45°C; 4xSSC, 50% formamide | |
| J | DNA:RNA | <50 | T_{J}*; 4xSSC | T_{J}*; 4xSSC |
| K | RNA:RNA | >50 | 70°C; 4xSSC -or- | 67°C; 1xSSC |
| | | | 50°C; 4xSSC, 50% formamide | |
| L | RNA:RNA | <50 | T_{L}*; 2xSSC | T_{L}*; 2xSSC |
| M | DNA:DNA | >50 | 50°C; 4xSSC -or- | 50°C; 2xSSC |
| | | | 40°C; 6xSSC, 50% formamide | |
| N | DNA:DNA | <50 | T_{N}*; 6xSSC | T_{N}*; 6xSSC |
| O | DNA:RNA | >50 | 55°C; 4xSSC -or- | 55°C; 2xSSC |
| | | | 42°C; 6xSSC, 50% formamide | |
| P | DNA:RNA | <50 | T_{P}*; 6xSSC | T_{P}*; 6xSSC |
| Q | RNA:RNA | >50 | 60°C;4xSSC-or- | 60°C; 2xSSC |
| | | | 45°C; 6xSSC, 50% formamide | |
| R | RNA:RNA | <50 | T_{R}*; 4xSSC | T_{R}*; 4xSSC |

| | | | | |
|---|---|---|---|---|
| 1: The hybrid length is that anticipated for the hybridized region(s) of the hybridizing polynucleotides. When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. 2: SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes after hybridization is complete. T_{B}* - T_{R}*: The hybridization temperature for hybrids anticipated to be less than 50 base pairs in length should be 5-10°C less than the melting temperature (Tₘ of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀Na⁺) + 0.41(%G+C) - (600/N), where N is the number of bases in the hybrid, and Na⁺ is the concentration of sodium ions in the hybridization buffer (Na⁺ for 1xSSC = 0.165M). Additional examples of stringency conditions for polynucleotide hybridization are provided in Sambrook, J., E.F. Fritsch, and T. Maniatis, 1989, Molecular Cloning: A Laboratory Manual, Cold Spring Harbor Laboratory Press, Cold Spring Harbor, NY, chapters 9 and 11, and Current Protocols in Molecular Biology, 1995, F.M. Ausubel et al., eds., John Wiley & Sons, Inc., sections 2.10 and 6.3-6.4, incorporated herein by reference. | | | | |

The isolated polynucleotides related to the present invention may be used as hybridization probes and primers to identify and isolate DNA having sequences encoding allelic variants of the disclosed polynucleotides. Allelic variants are naturally occurring alternative forms of the disclosed polynucleotides that encode polypeptides that are identical to or have significant similarity to the polypeptides encoded by the disclosed polynucleotides. Preferably, allelic variants have at least 90% sequence identity (more preferably, at least 95% identity; most preferably, at least 99% identity) with the disclosed polynucleotides. Alternatively, significant similarity exists when the nucleic acid segments will hybridize under selective hybridization conditions (e.g., highly stringent hybridization conditions) to the disclosed polynucleotides.

The isolated polynucleotides related to the present invention may also be used as hybridization probes and primers to identify and isolate DNAs having sequences encoding polypeptides homologous to the disclosed polynucleotides. These homologs are polynucleotides and polypeptides isolated from a different species than that of the disclosed polypeptides and polynucleotides, or within the same species, but with significant sequence similarity to the disclosed polynucleotides and polypeptides. Preferably, polynucleotide homologs have at least 50% sequence identity (more preferably, at least 75% identity; most preferably, at least 90% identity) with the disclosed polynucleotides, whereas polypeptide homologs have at least 30% sequence identity (more preferably, at least 45% identity; most preferably, at least 60% identity) with the disclosed polypeptides. Preferably, homologs of the disclosed polynucleotides and polypeptides are those isolated from mammalian species.

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and nonhomologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent sequence identity between two sequences may be accomplished using a mathematical algorithm. In a preferred embodiment, the percent identity between two amino acid sequences is determined using the Needleman and Wunsch ((1970) J. Mol. Biol. 48:444-53) algorithm, which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine whether a molecule is within a sequence identity or homology limitation of the invention) is a Blossum 62 scoring matrix with a gap penalty of 12, a gap extend penalty of 4, and a frameshift gap penalty of 5. The percent identity between two amino acid or nucleotide sequences can also be determined using the algorithm of Meyers and Miller ((1989) CABIOS 4:11-17), which has been incorporated into the ALIGN program (version 2.0), using a PAM120 weight residue table, a gap length penalty of 12 and a gap penalty of 4.

The isolated polynucleotides related to the present invention may also be used as hybridization probes and primers to identify cells and tissues that express the polypeptides related to the present invention and the conditions under which they are expressed.

Additionally, the function of the polypeptides related to the present invention may be directly examined by using the polynucleotides encoding the polypeptides to alter (i.e., enhance, reduce, or modify) the expression of the genes corresponding to the polynucleotides related to the present invention in a cell or organism. These "corresponding genes" are the genomic DNA sequences related to the present invention that are transcribed to produce the mRNAs from which the polynucleotides related to the present invention are derived.

Altered expression of the genes related to the present invention maybe achieved in a cell or organism through the use of various inhibitory polynucleotides, such as antisense polynucleotides, siRNAs, and ribozymes that bind and/or cleave the mRNA transcribed from the genes related to the invention (see, e.g., Galderisi et al. (1999) J. Cell Physiol. 181:251-57; Sioud (2001) Curr. Mol. Med. 1:575-88). Inhibitory polynucleotides to, e.g., IL-17F, IL-17R, and/or IL-17RC, may be useful as IL-17F signaling antagonists and, as such, may also be useful in preventing or treating disorders related to IL-17F signaling. Inhibitory polynucleotides may also consist of aptamers, i.e., polynucleotides that bind to and regulate protein activity, e.g., the activity of IL-17F, IL-17A, IL-17R, and/or TL-17RC. Aptamers are described throughout the literature, see, e.g., Nimjee et al. (2005) Anne. Rev Med. 56:555-83 and Patel (1997) Curr. Opin. Chem. Biol. 1:32-46.

The antisense polynucleotides or ribozymes related to the invention may be complementary to an entire coding strand of a gene related to the invention, or to only a portion thereof Alternatively, antisense polynucleotides or ribozymes can be complementary to a noncoding region of the coding strand of a gene related to the invention. The antisense polynucleotides or ribozymes can be constructed using chemical synthesis and enzymatic ligation reactions using procedures well known in the art. The nucleoside linkages of chemically synthesized polynucleotides can be modified to enhance their ability to resist nuclease-mediated degradation, as well as to increase their sequence specificity. Such linkage modifications include, but are not limited to, phosphorothioate, methylphosphonate, phosphoroamidate, boranophosphate, morpholino, and peptide nucleic acid (PNA) linkages (Galderisi et al., *supra*; Heasman (2002) Dev. Biol. 243:209-14; Micklefield (2001) Curr. Med. Chem. 8:1157-79). Alternatively, these molecules can be produced biologically using an expression vector into which a polynucleotide related to the present invention has been subcloned in an antisense (i.e., reverse) orientation.

The inhibitory polynucleotides of the present invention also include triplex-forming oligonucleotides (TFOs) that bind in the major groove of duplex DNA with high specificity and affinity (Knauert and Glazer (2001) Hum. Mol. Genet. 10:2243-51). Expression of the genes related to the present invention can be inhibited by targeting TFOs complementary to the regulatory regions of the genes (i.e., the promoter and/or enhancer sequences) to form triple helical structures that prevent transcription of the genes.

In one embodiment of the invention, the inhibitory polynucleotides of the present invention are short interfering RNA (siRNA) molecules. These siRNA molecules are short (preferably 19-25 nucleotides; most preferably 19 or 21 nucleotides), double-stranded RNA molecules that cause sequence-specific degradation of target mRNA. This degradation is known as RNA interference (RNAi) (e.g., Bass (2001) Nature 411:428-29). Originally identified in lower organisms, RNAi has been effectively applied to mammalian cells and has recently been shown to prevent fulminant hepatitis in mice treated with siRNA molecules targeted to Fas mRNA (Song et al. (2003) Nature Med. 9:347-51). In addition, intrathecally delivered siRNA has recently been reported to block pain responses in two models (agonist-induced pain model and neuropathic pain model) in the rat (Dorn et al. (2004) Nucleic Acids Res. 32(5):e49).

The siRNA molecules of the present invention may be generated by annealing two complementary single-stranded RNA molecules together (one of which matches a portion of the target mRNA) (Fire et al., U.S. Patent No. 6,506,559) or through the use of a single hairpin RNA molecule that folds back on itself to produce the requisite double-stranded portion (Yu et al. (2002) Proc. Natl. Acad. Sci. USA 99:6047-52). The siRNA molecules may be chemically synthesized (Elbashir et al. (2001) Nature 411:494-98) or produced by *in vitro* transcription using single-stranded DNA templates (Yu et al., *supra).* Alternatively, the siRNA molecules can be produced biologically, either transiently (Yu et al., *supra*; Sui et al. (2002) Proc. Natl. Acad. Sci. USA 99:5515-20) or stably (Paddison et al. (2002) Proc. Natl. Acad. Sci. USA 99:1443-48), using an expression vector(s) containing the sense and antisense siRNA sequences. Recently, reduction of levels of target mRNA in primary human cells, in an efficient and sequence-specific manner, was demonstrated using adenoviral vectors that express hairpin RNAs, which are further processed into siRNAs (Arts et al. (2003) Genome Res. 13:2325-32).

The siRNA molecules targeted to the polynucleotides related to the present invention can be designed based on criteria well known in the art (e.g., Elbashir et al. (2001) EMBO J. 20:6877-88). For example, the target segment of the target mRNA preferably should begin with AA (most preferred), TA, GA, or CA; the GC ratio of the siRNA molecule preferably should be 45-55%; the siRNA molecule preferably should not contain three of the same nucleotides in a row; the siRNA molecule preferably should not contain seven mixed G/Cs in a row; and the target segment preferably should be in the ORF region of the target mRNA and preferably should be at least 75 bp after the initiation ATG and at least 75 bp before the stop codon. Based on these criteria, or on other known criteria (e.g., Reynolds et al. (2004) Nature Biotechnol. 22:326-30), siRNA molecules of the present invention that target the mRNA polynucleotides related to the present invention may be designed by one of ordinary skill in the art. Preferred examples of siRNAs for use in the disclosed methods are set forth in SEQ ID NOs:17-32 and correspond to siRNAs useful to target IL-17R (SEQ ID NOs:17-24) and IL-17RC (SEQ ID NOs:25-32).

Altered expression of the genes related to the present invention in an organism may also be achieved through the creation of nonhuman transgenic animals into whose genomes polynucleotides related to the present invention have been introduced. Such transgenic animals include animals that have multiple copies of a gene (i.e., the transgene) of the present invention. A tissue-specific regulatory sequence(s) may be operably linked to the transgene to direct expression of a polypeptide related to the present invention to particular cells or a particular developmental stage. Methods for generating transgenic animals via embryo manipulation and microinjection, particularly animals such as mice, have become conventional and are well known in the art (e.g., Bockamp et al., Physiol. Genomics 11:115-32 (2002)).

Altered expression of the genes related to the present invention in an organism may also be achieved through the creation of animals whose endogenous genes corresponding to the polynucleotides related to the present invention have been disrupted through insertion of extraneous polynucleotide sequences (i.e., a knockout animal). The coding region of the endogenous gene may b e disrupted, thereby generating a nonfunctional protein. Alternatively, the upstream regulatory region of the endogenous gene may be disrupted or replaced with different regulatory elements, resulting in the altered expression of the still-functional protein. Methods for generating knockout animals include homologous recombination and are well known in the art (e.g., Wolfer et al., Trends Neurosci. 25:336-40 (2002)).

The isolated polynucleotides of the present invention also may be operably linked to an expression control sequence and/or ligated into an expression vector for recombinant production of the polypeptides (including active fragments and/or fusion polypeptides thereof) related to the present invention. General methods of expressing recombinant proteins are well known in the art.

An expression vector, as used herein, is intended to refer to a nucleic acid molecule capable of transporting another nucleic acid to which it has been linked. One type of vector is a plasmid, which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., nonepisomal mammalian vectors) can be integrated into the genome of a host cell upon introduction into the host cell, and thereby are replicated along with the host genome. Moreover, certain vectors are capable of directing the expression of genes to which they are operably linked. Such vectors are referred to herein as recombinant expression vectors (or simply, expression vectors). In general, expression vectors of utility in recombinant DNA techniques are often in the form of plasmids. In the present specification, plasmid and vector may be used interchangeably as the plasmid is the most commonly used form of vector. However, the invention is intended to include other forms of expression vectors, such as viral vectors (e.g., replication defective retroviruses, adenoviruses and adeno-associated viruses) that serve equivalent functions.

In one embodiment, the polynucleotides related to the present invention are used to create recombinant IL-17F agonists, e.g., those that can be identified based on the presences of at least one "IL-17F receptor-binding motif." As used herein, the term "IL-17F receptor-binding motif" includes amino acid sequences or residues that are important for binding of IL-17F to its requisite receptor. An example of an IL-17F agonist includes IL-17F homodimer, IL-17A/IL-17F heterodimer, fragments thereof, e.g., IL-17R or IL-17RC binding fragments, and/or small molecules (as described below). Such agonists may be useful in regulation of hematopoiesis, and consequently, in the treatment of myeloid or lymphoid cell deficiencies. In another embodiment, the polynucleotides related to the present invention are used to create IL-17F signaling antagonists (e.g., IL-17F, IL-17R, and/or IL-17RC inhibitory polynucleotides; soluble IL-17R and/or IL-17RC polypeptides (including fragments (e.g., IL-17F binding fragments) and/or fusion proteins thereof); inhibitory anti-IL-17F, anti-IL-17R, or IL-17RC antibodies, which may inhibit the bioactivity of IL-17F homodimers and/or IL-17A/IL-17F heterodimers; and/or antagonistic small molecules, etc.).

Methods of creating fusion polypeptides, i.e., a first polypeptide moiety linked with a second polypeptide moiety, are well known in the art. For example, an IL-17F polypeptide or an IL-17F receptor polypeptide (e.g., IL-17R and/or IL-17RC, including fragments thereof) may be fused to a second polypeptide moiety, e.g., an immunoglobulin or a fragment thereof (e.g., an Fc binding fragment thereof). In some embodiments, the first polypeptide moiety includes, e.g., full-length IL-17RC polypeptide. Alternatively, the first polypeptide may comprise less than the full-length IL-17RC polypeptide. Additionally, soluble forms of, e.g., IL-17RC may be fused through "linker" sequences to the Fc portion of an immunoglobulin. Other fusions proteins, such as those with glutathione-*S-*transferase (GST), Lex-A, thioredoxin (TRX) or maltose-binding protein (MBP), may also be used.

The second polypeptide moiety is preferably soluble. In some embodiments, the second polypeptide moiety enhances the half-life, (e.g., the serum half-life) of the linked polypeptide. In some embodiments, the second polypeptide moiety includes a sequence that facilitates association of the fusion polypeptide with a second IL-17F or IL-17R polypeptide. In preferred embodiments, the second polypeptide includes at least a region of an immunoglobulin polypeptide. Immunoglobulin fusion polypeptide are known in the art and are described in, e.g., U.S. Patent Nos. 5,516,964; 5,225,538; 5,428,130; 5,514,582; 5,714,147; and 5,455,165, all of which are hereby incorporated by reference. The fusion proteins may additionally include a linker sequence joining the first polypeptide moiety, e.g., IL-17F or IL-17R, including fragments thereof, to the second moiety. Use of such linker sequences are well known in the art. For example, the fusion protein can include a peptide linker, e.g., a peptide linker of about 2 to 20, more preferably less than 10, amino acids in length. In one embodiment, the peptide linker may be 2 amino acids in length.

In another embodiment, the recombinant protein includes a heterologous signal sequence (i.e., a polypeptide sequence that is not present in a polypeptide encoded by an IL-17F, IL-17R or IL-17RC nucleic acid) at its N-terminus. For example, a signal sequence from another protein may be fused with an IL-17R and/or IL-17RC polypeptide, including fragments and/or fusion proteins thereof In certain host cells (e.g., mammalian host cells), expression and/or secretion of recombinant proteins can be increased through use of a heterologous signal sequence. A signal peptide that may be included in the fusion protein is the melittin signal peptide MKFLVNVALVFWVVYISYIYA (SEQ ID NO:33).

A fusion protein of the invention may be produced by standard recombinant DNA techniques. For example, DNA fragments coding for the different polypeptide sequences are ligated together in-frame in accordance with conventional techniques by employing, e.g., blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments may be carried out using anchor primers that give rise to complementary overhangs between two consecutive gene fragments that can subsequently be annealed and reamplified to generate a chimeric gene sequence (see, for example, Ausubel et al. (Eds.) Current Protocols in Molecular Biology, John Wiley & Sons, 1992). Moreover, many expression vectors are commercially available that encode a fusion moiety (e.g., an Fc region of an immunoglobulin heavy chain). An IL-17F-, IL-17Rand/or IL-17RC-encoding nucleic acid may be cloned into such an expression vector such that the fusion moiety is linked in-frame to the immunoglobulin protein. In some embodiments, IL-17F, IL-17R and/or IL-17RC fusion polypeptides exist as oligomers, such as dimers or trimers.

The recombinant expression vectors of the invention may carry additional sequences, such as sequences that regulate replication of the vector in host cells (e.g., origins of replication) and selectable marker genes. The selectable marker gene facilitates selection of host cells into which the vector has been introduced. For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr- host cells with methotrexate selection/amplification) and the *neo* gene (for G418 selection).

Suitable vectors can be chosen or constructed, containing appropriate regulatory sequences, including promoter sequences, terminator sequences, polyadenylation sequences, enhancer sequences, marker genes and other sequences, e.g., sequences that regulate replication-of the vector in the host cells (e.g., origins of replication) as appropriate. Vectors may be plasmids or viral, e.g., phage, or phagemid, as appropriate. For further details see, for example, Molecular Cloning: a Laboratory Manual: 2nd ed., Sambrook et al., Cold Spring Harbor Laboratory Press, 1989. Many known techniques and protocols for manipulation of nucleic acid, for example, in preparation of nucleic acid constructs, mutagenesis, sequencing, introduction of DNA into cells and gene expression, and analysis of proteins, are described in detail in Current Protocols in Molecular Biology, 2nd ed., Ausubel et al. eds., John Wiley & Sons, 1992.

Thus, a further aspect of the present invention provides a host cell comprising a nucleic acid as disclosed herein. A still further aspect provides a method comprising introducing such nucleic acid into a host cell. The introduction may employ any available technique. For eukaryotic cells, suitable techniques may include calcium phosphate transfection, DEAE-Dextran, electroporation, liposome-mediated transfection, and transduction using retrovirus or other viruses, e.g., vaccinia or, for insect cells, baculovirus. For bacterial cells, suitable techniques may include calcium chloride transformation, electroporation and transfection using bacteriophage. The introduction may be followed by causing or allowing expression from the nucleic acid, e.g., by culturing host cells under conditions for expression of the gene.

A number of cell lines may act as suitable host cells for recombinant expression of the polypeptides related to the present invention. Mammalian host cell lines include, for example, COS cells, CHO cells, 293 cells, A431 cells, 3T3 cells, CV-1 cells, HeLa cells, L cells, BHK21 cells, HL-60 cells, U937 cells, HaK cells, Jurkat cells, as well as cell strains derived from *in vitro* culture of primary tissue and primary explants.

Alternatively, it may be possible to recombinantly produce the polypeptides related to the present invention in lower eukaryotes, such as yeast, or in prokaryotes. Potentially suitable yeast strains include *Saccharomyces cerevisiae, Schizosaccharomyces pombe, Kluyveromyces* strains, and *Candida* strains. Potentially suitable bacterial strains include *Escherichia coli, Bacillus subtilis,* and *Salmonella typhimurium.* If the polypeptides related to the present invention are made in yeast or bacteria, it may be necessary to modify them by, for example, phosphorylation or glycosylation of appropriate sites, in order to obtain functionality. Such covalent attachments may be accomplished using well-known chemical or enzymatic methods.

Expression in bacteria may result in formation of inclusion bodies incorporating the recombinant protein. Thus, refolding of the recombinant protein may be required in order to produce active or more active material. Several methods for obtaining correctly folded heterologous proteins from bacterial inclusion bodies are known in the art. These methods generally involve solubilizing the protein from the inclusion bodies, then denaturing the protein completely using a chaotropic agent. When cysteine residues are present in the primary amino acid sequence of the protein, it is often necessary to accomplish the refolding in an environment that allows correct formation of disulfide bonds (a redox system). General methods of refolding are disclosed in Kohno (1990) Meth. Enzymol. 185:187-95. EP 0433225, and U.S. Patent 5,399,677 describe other appropriate methods.

The polypeptides related to the present invention may also be recombinantly produced by operably linking the isolated polynucleotides of the present invention to suitable control sequences in one or more insect expression vectors, such as baculovirus vectors, and employing an insect cell expression system. Materials and methods for baculovirus/Sf9 expression systems are commercially available in kit form (e.g., MAXBAC^{®} kit, Invitrogen, Carlsbad, CA).

Following recombinant expression in the appropriate host cells, the recombinant polypeptides of the present invention may then be purified from culture medium or cell extracts using known purification processes, such as immunoprecipitation, gel filtration and ion exchange chromatography. For example, soluble forms of IL-17F signaling antagonists, e.g., IL-17R protein and/or IL-17RC proteins (including fragments, and/or fusion proteins thereof); or IL-17F agonists, e.g., soluble IL-17F (in homodimer or IL-17A/IL-17F heterodimer formation), may be purified from conditioned media. Membrane-bound forms of, e.g., an IL-17F signaling antagonist, may be purified by preparing a total membrane fraction from the expressing cell and extracting the membranes with a nonionic detergent such as Triton X-100. A polypeptide related to the present invention may be concentrated using a commercially available protein concentration filter, for example, an AMICON^{®} or Millipore PELLICON^{®} ultrafiltration unit (Millipore, Billerica, MA). Following the concentration step, the concentrate can be applied to a purification matrix such as a gel filtration medium. Alternatively, an anion exchange resin can be employed, for example, a matrix or substrate having pendant diethylaminoethyl (DEAE) or polyetheyleneimine (PEI) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred (e.g., S-SEPHAROSE^{®} columns, Sigma-Aldrich, St. Louis, MO). The purification of recombinant proteins from culture supernatant may also include one or more column steps over such affinity resins as concanavalin A-agarose, heparin-TOYOPEARL^{®} (Toyo Soda Manufacturing Co., Ltd., Japan) or Cibacrom blue 3GA SEPHAROSE^{®} (Tosoh Biosciences, San Francisco, CA); or by hydrophobic interaction chromatography using such resins as phenyl ether, butyl ether, or propyl ether; or by immunoaffinity chromatography. Finally, one or more reverse-phase high performance liquid chromatography (RP-HPLC) steps employing hydrophobic RP-HPLC media, e.g., silica gel having pendant methyl or other aliphatic groups, can be employed to further purify the recombinant protein. Affinity columns including antibodies (e.g., those described using the methods herein) to the recombinant protein may also be used in purification in accordance with known methods. Some or all of the foregoing purification steps, in various combinations or with other known methods, may also be employed to provide a substantially purified isolated recombinant protein. Preferably, the isolated recombinant protein is purified so that it is substantially free of other mammalian proteins. Additionally, these purification processes may also be used to purify the polypeptides of the present invention from other sources, including natural sources. For example, polypeptides related to the invention, e.g., IL-17F agonists (e.g., soluble IL-17F) or IL-17F signaling antagonists (e.g., soluble IL-17R and/or soluble IL-17RC proteins, including fragments and/or fusion proteins thereof), which are expressed as a product of transgenic animals, e.g., as a component of the milk of transgenic cows, goats, pigs, or sheep, maybe purified as described above.

Alternatively, the polypeptides may also be recombinantly expressed in a form that facilitates purification. For example, the polypeptides may be expressed as fusions with proteins such as maltose-binding protein (MBP), glutathione-*S*-transferase (GST), or thioredoxin (TRX). Kits for expression and purification of such fusion proteins are commercially available from New England BioLabs (Beverly, MA), Pharmacia (Piscataway, NJ), and Invitrogen, respectively. Recombinant proteins can also be tagged with a small epitope and subsequently identified or purified using a specific antibody to the epitope. A preferred epitope is the FLAG epitope, which is commercially available from Eastman Kodak (New Haven, CT).

Alternatively, recombinant IL-17F and IL-17A fusion proteins may be tagged with different epitopes to allow purification of IL-17A/IL-17F heterodimers. The existence of different tags on IL-17F and IL-17A allows isolation of IL-17A/IL-17F heterodimers that are substantially free from both IL-17A and IL-17F homodimers. For example, IL-17A may be tagged with an epitope such as FLAG or myc epitope, while IL-17F is concurrently tagged with an epitope such as His or GST epitope, and both proteins simultaneously expressed in a cell. Extracts from the recombinant host cell, or media in which the host cells are cultured, would be obtained and subjected to two-step affinity chromatography purification under nonreducing conditions. The first affinity column would bind one of the two different tags, e.g., a FLAG epitope fused to IL-17A (or a fragment of IL-17A), and therefore the wash from the first column would contain (predominantly) IL-17F homodimers and the eluent from the first column would contain both IL-17A/IL-17F heterodimers and IL-17A homodimers. The eluent from the first column would then be placed over a second affinity column that specifically binds the other of the two different tags, e.g., a His tag fused to IL-17F. Thus, the wash from the second column would contain IL-17A homodimers and the eluent from the second column would predominantly or exclusively contain IL-17A/IL-17F heterodimers (i.e., substantially free of both IL-17A and IL-17F homodimers). The extracts from the recombinant host cells or the host cell media could be obtained under nonreducing conditions such that protein-protein interactions are not interrupted, or could be obtained under reducing conditions and then treated to allow proper refolding and interactions of the IL-17F and IL-17A monomers contained therein. One skilled in the art would readily realize that a host cell need not express both IL-17F and IL-17A fusion proteins; rather cell or media extracts from single transfectants, e.g., a host cell expressing either a IL-17A or IL-17F fusion protein, could be obtained and combined under conditions that allow the IL-17A and IL-17F monomers to dimerize.

The polypeptides related to the present invention, including IL-17F signaling antagonists, may also be produced by known conventional chemical synthesis. Methods for chemically synthesizing such polypeptides are well known to those skilled in the art. Such chemically synthetic polypeptides may possess biological properties in common with the natural, purified polypeptides, and thus may be employed as biologically active or immunological substitutes for the natural polypeptides.

The inventors were also able to isolate the "natural", i.e., nonrecombinant form, of the polypeptides of the invention, including a natural form of IL-17A (see, e.g., Example 5). Thus, the polypeptides of the present invention include natural IL-17A homodimer, IL-17F homodimer, IL-17A/IL-17F heterodimer, etc.

The polypeptides related to the present invention, including IL-17F signaling antagonists, also encompass molecules that are structurally different from the disclosed polypeptides (e.g., which have a slightly altered sequence), but have substantially the same biochemical properties as the disclosed polypeptides (e.g., are changed only in functionally nonessential amino acid residues). Such molecules include naturally occurring allelic variants and deliberately engineered variants containing alterations, substitutions, replacements, insertions, or deletions. Techniques for such alterations, substitutions, replacements, insertions, or deletions are well known to those skilled in the art. In some embodiments, the polypeptide moiety is provided as a variant polypeptide having mutations in the naturally occurring sequence (wild type) that results in a sequence more resistant to proteolysis (relative to the nonmutated sequence).

IL-17F (including IL-17F homodimers and IL-I7A/IL-17F heterodimers), IL-17R, IL-17RC polypeptides, fragments and/or fusion polypeptides thereof, recombinant and natural forms thereof, and/or natural IL-17A may be used to screen agents (e.g., other IL-17F signaling antagonists, e.g., anti-IL-17F antibodies) that are capable of binding IL-17F and/or inhibiting IL-17F bioactivity. Binding assays utilizing a desired binding protein, immobilized or not, are well known in the art and may be used for this purpose with the polypeptides related to the present invention, including the IL-17F signaling antagonists of the invention, e.g., IL-17R and/or IL-I7RC. Purified cell-based or protein-based (cell-free) screening assays may be used to identify such agents. For example, IL-17F protein may be immobilized in purified form on a carrier and binding of potential ligands to purified IL-17F may be measured. Antibodies

In other embodiments, the invention provides IL-17F signaling antagonists as antibodies, i.e., intact antibodies and antigen binding fragments thereof, that specifically bind to IL-17F (including IL-17F homodimers and/or IL-17A/IL-17F heterodimers), preferably mammalian (e.g., human) IL-17F, or to the receptors for IL-17F, e.g., IL-17R and/or IL-17RC. In one embodiment, the antibodies are inhibitory antibodies, i.e., they inhibit at least one IL-17F bioactivity (e.g., binding of IL-17F and its receptor, IL-17F-mediated activation of signaling components (e.g., NF-κB), IL-17F-mediated induction of cytokine production, IL-17F-mediated increase in Aggrecanase etc.) and may be useful in diagnosing, prognosing, monitoring and/or treating disorders related to IL-17F signaling. The upregulation of IL-17A and IL-17F production by IL-21 (see Example 5) suggests that the proinflammatory effects associated with IL-21 binding to and activating IL-21R (e.g., IL-21 signaling) are mediated by IL-17A homodimer, IL-17F homodimer, and/or IL-17A/IL-17F heterodimer. Consequently, the antibodies of the invention that mitigate IL-17F signaling may also be inhibitory antibodies to at least one activity associated with IL-21 signaling (e.g., modulation of cytokine production, inflammation in inflammatory/autoimmune disorders (such as inflammatory bowel disorders or diseases (IBDs), rheumatoid arthritis, transplant/graft rejection, and psoriasis), etc.; see U.S. Patent Application Nos. 60/599,086 and 60/639,176) and may be useful in diagnosing, prognosing, monitoring and/or treating disorders associated with IL-21 signaling.

Additionally, the invention provides anti-IL-17F antibodies that specifically bind to but do not inhibit IL-17F signaling (i.e., detecting antibodies); such antibodies may be used to detect the presence of IL-17F protein (e.g., as a homodimer and/or heterodimer), e.g., as part of a kit for diagnosing, prognosing, and/or monitoring a disorder(s) related to IL-17F signaling. In one embodiment, the antibody is directed to IL-17F. In another embodiment, the antibody is a monoclonal or single specificity antibody. The antibodies may also be human, humanized, chimeric, or *in vitro-* generated antibodies against human IL-17F.

One of skill in the art will recognize that, as used herein, the term "antibody" refers to a protein comprising at least one, and preferably two, heavy (H) chain variable regions (abbreviated herein as VH), and at least one and preferably two light (L) chain variable regions (abbreviated herein as VL). The VH and VL regions can be further subdivided into regions ofhypervariability, termed "complementarity determining regions" ("CDRs"), interspersed with regions that are more conserved, termed "framework regions" ("FR"). The extent of the FRs and CDRs has been precisely defined (see, Kabat et al. (1991) Sequences of Proteins of Immunological Interest, Fifth Edition, U.S. Department of Health and Human Services, NIH Publication No. 91-3242; and Chothia et al. (1987) J. Mol. Biol. 196:901-917, which are hereby incorporated by reference). Each VH and VL is composed of three CDRs and four FRs, arranged from amino-terminus to carboxy-terminus in the following order: FR1, CDR1, FR2, CDR2, FR3, CDR3, FR4.

The antibody may further include a heavy and light chain constant region to thereby form a heavy and light immunoglobulin chain, respectively. In one embodiment, the antibody is a tetramer of two heavy immunoglobulin chains and two light immunoglobulin chains, wherein the heavy and light immunoglobulin chains are interconnected, e.g., by disulfide bonds. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (C1q) of the classical complement system.

Immunoglobulin refers to a protein consisting of one or more polypeptides substantially encoded by immunoglobulin genes. The recognized human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin "light chains" (about 25 Kd, or 214 amino acids) are encoded by a variable region gene at the NH₂-terminus (about 110 amino acids) and a kappa or lambda constant region gene at the COOH-terminus. Full-length immunoglobulin "heavy chains" (about 50 Kd, or 446 amino acids), are similarly encoded by a variable region gene (about 116 amino acids) and one of the other aforementioned constant region genes, e.g., gamma (encoding about 330 amino acids). The immunoglobulin heavy chain constant region genes encode for the antibody class, i.e., isotype (e.g., IgM or IgG1). The antigen binding fragment of an antibody (or simply "antibody portion," or "fragment"), as used herein, refers to one or more fragments of a full-length antibody that retain the ability to specifically bind to an antigen (e.g., CD3). Examples of binding fragments encompassed within the term "antigen binding fragment" of an antibody include, but are not limited to, (i) an Fab fragment, a monovalent fragment consisting of the VL, VH, CL and CH1 domains; (ii) an F(ab')₂ fragment, a bivalent fragment comprising two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment, which consists of a VH domain; and (vi) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain Fv (scFv)). Such single chain antibodies are also intended to be encompassed within the term "antigen binding fragment" of an antibody. These antibody fragments are obtained using conventional techniques known to those skilled in the art, and the fragments are screened for utility in the same manner as are intact antibodies.

Antibody molecules to the polypeptides of the present invention, e.g., antibodies to IL-17F protein, IL-17R, and/or IL-17RC, may be produced by methods well known to those skilled in the art. For example, monoclonal antibodies may be produced by generation of hybridomas in accordance with known methods. Hybridomas formed in this manner are then screened using standard methods, such as an enzyme-linked immunosorbent assay (ELISA), to identify one or more hybridomas that produce an antibody that specifically binds with the polypeptides of the present invention. For example, IL-17F proteins of the invention may also be used to immunize animals to obtain polyclonal and monoclonal antibodies that react with the IL-17F protein and which may inhibit binding of IL-17F (e.g., IL-17F homodimer and/or IL-17A/IL-17F heterodimer) to its receptor, e.g., IL-17R or IL-17RC. Similarly, IL-17R or IL-17RC proteins may be used to obtain polyclonal and monoclonal antibodies that specifically react with IL-17R or IL-17RC, respectively, and which may inhibit binding of these receptors to IL-17F protein specifically (including IL-17F homodimer and IL-17A/IL-17F heterodimer), i.e., these antibodies do not inhibit binding of either or both of these receptors to other IL-17F family members, e.g., IL-17A homodimer. The peptide immunogens additionally may contain a cysteine residue at the carboxyl terminus, and may be conjugated to a hapten such as keyhole limpet hemocyanin (KLH). Additional peptide immunogens may be generated by replacing tyrosine residues with sulfated tyrosine residues. Methods for synthesizing such peptides are well known in the art. A full-length polypeptide of the present invention may be used as the immunogen, or, alternatively, antigenic peptide fragments of the polypeptides may be used. An antigenic peptide of a polypeptide of the present invention comprises at least 7 continuous amino acid residues and encompasses an epitope such that an antibody raised against the peptide forms a specific immune complex with the polypeptide. Preferably, the antigenic peptide comprises at least 10 amino acid residues, more preferably at least 15 amino acid residues, even more preferably at least 20 amino acid residues, and most preferably at least 30 amino acid residues.

Monoclonal antibodies may be generated by other methods known to those skilled in the art of recombinant DNA technology. As an alternative to preparing monoclonal antibody-secreting hybridomas, a monoclonal antibody to a polypeptide of the present invention may be identified and isolated by screening a recombinant combinatorial immunoglobulin library (e.g., an antibody phage display library) with a polypeptide related to the present invention (e.g., IL-17F, IL-17R, IL-17RC) to thereby isolate immunoglobulin library members that bind to the polypeptides related to the present invention (e.g., IL-17F, IL-17R, IL-17RC, respectively). Techniques and commercially available kits for generating and screening phage display libraries are well known to those skilled in the art. Additionally, examples of methods and reagents particularly amenable for use in generating and screening antibody display libraries can be found in the literature. For example, the "combinatorial antibody display" method is well known and was developed to identify and isolate antibody fragments having a particular antigen specificity, and can be utilized to produce monoclonal antibodies. After immunizing an animal with an immunogen as described above, the antibody repertoire of the resulting B-cell pool is cloned. Methods are generally known for obtaining the DNA sequence of the variable regions of a diverse population of immunoglobulin molecules by using a mixture of oligomer primers and PCR. For instance, mixed oligonucleotide primers corresponding to the 5' leader (signal peptide) sequences and/or framework 1 (FR1) sequences, as well as primers to a conserved 3' constant region, can be used for PCR amplification of the heavy and light chain variable regions from a number of murine antibodies; a similar strategy has also been used to amplify human heavy and light chain variable regions from human antibodies.

Polyclonal sera and antibodies may be produced by immunizing a suitable subject with a polypeptide of the present invention. The antibody titer in the immunized subject may be monitored over time by standard techniques, such as with ELISA using immobilized protein. If desired, the antibody molecules directed against a polypeptide of the present invention may be isolated from the subject or culture media and further purified by well-known techniques, such as protein A chromatography, to obtain an IgG fraction.

Fragments of antibodies to the polypeptides of the present invention may be produced by cleavage of the antibodies in accordance with methods well known in the art. For example, immunologically active Fab and F(ab')₂ fragments may be generated by treating the antibodies with an enzyme such as pepsin.

Additionally, chimeric, humanized, and single-chain antibodies to the polypeptides of the present invention, comprising both human and nonhuman portions, may be produced using standard recombinant DNA techniques and/or a recombinant combinatorial immunoglobulin library. Humanized antibodies may also be produced using transgenic mice which are incapable of expressing endogenous immunoglobulin heavy and light chain genes, but which can express human heavy and light chain genes. For example, human monoclonal antibodies (mAbs) directed against, e.g., IL-17F protein, may be generated using transgenic mice carrying the human immunoglobulin genes rather than murine immunoglobulin genes. Splenocytes from these transgenic mice immunized with the antigen of interest may then be used to produce hybridomas that secrete human mAbs with specific affinities for epitopes from a human protein.

Chimeric antibodies, including chimeric immunoglobulin chains, may be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted.

An antibody or an immunoglobulin chain may be humanized by methods known in the art. Humanized antibodies, including humanized immunoglobulin chains, may be generated by replacing sequences of the Fv variable region that are not directly involved in antigen binding with equivalent sequences from human Fv variable regions. General methods for generating humanized antibodies are provided by Morrison (1985) Science 229:1202-07; Oi et al. (1986) BioTechniques 4:214; Queen et al., U.S. Patent Nos. 5,585,089; 5,693,761; 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid sequences are well known to those skilled in the art and, for example, may be obtained from a hybridoma producing an antibody against a predetermined target. The recombinant DNA encoding the humanized antibody, or fragment thereof, then can be cloned into an appropriate expression vector.

Humanized or CDR-grafted antibody molecules or immunoglobulins may be produced by CDR grafting or CDR substitution, wherein one, two, or all CDRs of an immunoglobulin chain can be replaced. See, e.g., U.S. Patent No. 5,225,539; Jones et al. (1986) Nature 321:552-25; Verhoeyan et al. (1988) Science 239:1534; Beidler et al. (1988) J. Immunol. 141:4053-60; Winter, U.S. Patent No. 5,225,539, the contents of all of which are hereby incorporated by reference. Winter describes a CDR-grafting method that may be used to prepare the humanized antibodies of the present invention (UK Patent Application GB 2188638A; Winter, U.S. Patent No. 5,225,539), the contents of which are hereby incorporated by reference. All of the CDRs of a particular human antibody may be replaced with at least a portion of a nonhuman CDR, or only some of the CDRs may be replaced with nonhuman CDRs. It is only necessary to replace the number of CDRs required for binding of the humanized antibody to a predetermined antigen.

Human antibodies may additionally be produced using transgenic nonhuman animals that are modified so as to produce fully human antibodies rather than the animal's endogenous antibodies in response to challenge by an antigen. See, e.g., PCT publication WO 94/02602. The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the XENOMOUSE^{™} as disclosed in PCT publications WO 96/33735 and WO 96/34096. This animal produces B cells that secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

Monoclonal, chimeric and humanized antibodies that have been modified by, e.g., deleting, adding, or substituting other portions of the antibody, e.g., the constant region, are also within the scope of the invention. As nonlimiting examples, an antibody can be modified by deleting the constant region, by replacing the constant region with another constant region, e.g., a constant region meant to increase half-life, stability, or affinity of the antibody, or a constant region from another species or antibody class, and by modifying one or more amino acids in the constant region to alter, for example, the number of glycosylation sites, effector cell function, Fc receptor (FcR) binding, complement fixation, etc.

Methods for altering an antibody constant region are known in the art. Antibodies with altered function, e.g., altered affinity for an effector ligand, such as FcR on a cell, or the C1 component of complement, can be produced by replacing at least one amino acid residue in the constant portion of the antibody with a different residue (see, e.g., EP 388,151 A1, U.S. 5,624,821 and U.S. 5,648,260, the contents of all of which are hereby incorporated by reference). Similar types of alterations to the murine (or other species) immunoglobulin may be applied to reduce or eliminate these functions, and are known in the art.

For example, it is possible to alter the affinity of an Fc region of an antibody (e.g., an IgG, such as a human IgG) for an FcR (e.g., Fc gamma R1), or for C1q binding by replacing the specified residue(s) with a residue(s) having an appropriate functionality on its side chain, or by introducing a charged functional group, such as glutamate or aspartate, or an aromatic nonpolar residue such as phenylalanine, tyrosine, tryptophan or alanine (see, e.g., U.S. 5,624,821).

Anti-IL-17F, anti-IL-17R, or anti-IL-17RC antibodies of the invention may be useful for isolating, purifying, and/or detecting IL-17F protein (e.g., in monomer, homodimer, or heterodimer formation), IL-17R, or IL-17RC polypeptides, respectively, in supernatant, cellular lysate, or on the cell surface. Antibodies disclosed in this invention may be also used diagnostically to monitor, e.g., IL-17F protein levels, as part of a clinical testing procedure, or clinically to target a therapeutic modulator to a cell or tissue comprising the antigen of the antibody. For example, a therapeutic such as a small molecule, or other therapeutic of the invention may be linked to an anti-IL-17F, anti-IL-17R, or anti-IL17RC antibody in order to target the therapeutic to the cell or tissue expressing IL-17F, IL-17R, or IL-17RC, respectively. Antagonistic antibodies (preferably monoclonal antibodies) that bind to IL-17F, IL-17R, or IL-17RC protein may also be useful in the treatment of a disease(s) related to IL-17F signaling, and/or a disease(s) associated with IL-21 signaling (e.g., IL-21 binding to and activation of IL-21R), due to the relationship between IL-21 and IL-17F production. Thus, the present invention further provides compositions comprising an inhibitory antibody that specifically binds to IL-17F (in monomeric and/or dimerized forms), IL-17R, or IL-17RC that decreases, limits, blocks, or otherwise reduces IL-17F signaling. Similarly, anti-IL-17F, anti-IL-17R, or anti-IL-17RC antibodies may be useful in isolating, purifying, detecting, and/or diagnostically monitoring IL-17F, IL-17R, or IL-17RC, respectively, and/or clinically targeting a therapeutic modulator to a cell or tissue comprising IL-17F, IL-17R, or IL-17RC, respectively.

In addition to antibodies for use in the instant invention, antibody-based molecules may also be employed to modulate the activity of IL-17F homodimers, IL-17A homodimers, and/or IL-17F/IL-17A homodimers. Such antibody-based molecules include small modular immunopharmaceutical (SMIP^{™}) drugs (Trubion Pharmaceuticals, Seattle, WA). SMIPs are single-chain polypeptides composed of a binding domain for a cognate structure such as an antigen, a counterreceptor or the like, a hinge-region polypeptide having either one or no cysteine residues, and immunoglobulin CH2 and CH3 domains (see also www.trubion.com). SMIPs exhibit the binding specificity and activity of monoclonal antibodies, but are approximately one-third to one-half the size of conventional therapeutic monoclonal antibodies, and have an extensive *in vivo* half-life. SMIPs and their uses and applications are disclosed in, e.g., U.S. Published Patent Appln. Nos. 2003/0118592, 2003/0133939, 2004/0058445, 2005/0136049, 2005/0175614, 2005/0180970, 2005/0186216, 2005/0202012, 2005/0202023, 2005/0202028, 2005/0202534, and 2005/023 8646, and related patent family members thereof, all of which are hereby incorporated by reference herein in their entireties.

### Screening Assays

The polynucleotides and polypeptides related to IL-17F signaling may be used in screening assays to identify pharmacological agents or lead compounds for agents that are capable of modulating the activity of IL-17F in a cell or organism and are thereby potential regulators of inflammatory responses. For example, samples containing IL-17F (either natural or recombinant) may be contacted with one of a plurality of test compounds (either biological agents or small organic molecules), and the biological activity of IL-17F in each of the treated samples can be compared with the biological activity of IL-17F in untreated samples or in samples contacted with different test compounds. Such comparisons will determine whether any of the test compounds results in: 1) a substantially decreased level of expression or biological activity of IL-17F, thereby indicating an antagonist of IL-17F, or 2) a substantially increased level of expression or biological activity of IL-17F, thereby indicating an agonist of IL-17F. In one embodiment, the identification of test compounds capable of modulating IL-17F activity is performed using high-throughput screening assays, such as BIACORE^{®} (Biacore International AB, Uppsala, Sweden), BRET (bioluminescence resonance energy transfer), and FRET (fluorescence resonance energy transfer) assays, as well as ELISA and cell-based assays.

### Small Molecules

Decreased IL-17F activity (and/or at least one activity associated with IL-21 binding to and activation of IL-21R) in an organism (or subject) afflicted with (or at risk for) disorders related to IL-17F signaling (and/or disorders associated with IL-21 binding to and activation of IL-21R), e.g., inflammatory bowel disease, rheumatoid arthritis, transplant rejection, psoriasis, etc., or in a cell from such an organism (or subject) involved in such disorders, may also be achieved through the use of small molecules (usually organic small molecules) that antagonize, i.e., inhibit the activity of, IL-17F. Novel antagonistic small molecules may be identified by the screening methods described above and may be used in the treatment methods of the present invention described herein.

Conversely, increased IL-17F activity (and/or IL-21 associated activity) in an organism (or subject) afflicted with (or at risk for) an immune deficiency, e.g., neutropenia, or in a cell from such an organism (or subject) involved in such a disorder, may also be achieved through the use of small molecules (usually organic small molecules) that agonize, i.e., enhance the activity of, IL-17F. Novel agonistic small molecules may be identified by the screening methods described above and may be used in the methods of treating immune deficiencies, e.g., as described in U.S. Patent Nos. 5,707,829; 6,043,344; 6,074,849 and U.S. Patent Application No. 10/102,080, all of which are incorporated by reference in their entireties.

The term small molecule refers to compounds that are not macromolecules (see, e.g., Karp (2000) Bioinformatics Ontology 16:269-85; Verkman (2004) AJP-Cell Physiol. 286:465-74). Thus, small molecules are often considered those compounds that are, e.g., less than one thousand daltons (e.g., Voet and Voet, Biochemistry, 2nd ed., ed. N. Rose, Wiley and Sons, New York, 14 (1995)). For example, Davis et al. (2005) Proc. Natl. Acad. Sci. USA 102:5981-86, use the phrase small molecule to indicate folates, methotrexate, and neuropeptides, while Halpin and Harbury (2004) PLos Biology 2:1022-30, use the phrase to indicate small molecule gene products, e.g., DNAs, RNAs and peptides. Examples of natural small molecules include, but are not limited to, cholesterols, neurotransmitters, and siRNAs; synthesized small molecules include, but are not limited to, various chemicals listed in numerous commercially available small molecule databases, e.g., FCD (Fine Chemicals Database), SMID (Small Molecule Interaction Database), ChEBI (Chemical Entities of Biological Interest), and CSD (Cambridge Structural Database) (see, e.g., Alfarano et al. (2005) Nuc. Acids Res. Database Issue 33:D416-24).

### Methods for Diagnosing, Prognosing, and Monitoring the Progress of Disorders Related to IL-17F Signaling

It is well known in the art that immunological mechanisms studied in animal models, particularly murine models, may be and often are, translatable to the human immune system. As such, although many of the Examples disclosed herein demonstrate the ability of IL-17F signaling antagonists to inhibit IL-17F bioactivities in animal models, in addition to human samples, the disclosed methods for diagnosing, prognosing, and monitoring disorders related to IL-17F signaling will be particularly useful for diagnosing, prognosing and monitoring such disorders in humans.

The present invention provides methods for diagnosing, prognosing, and monitoring the progress of disorders related to IL-17F signaling in a. subject (e.g., that directly or indirectly involve increases in the bioactivity of IL-17F) by detecting an upregulation of IL-17F activity, e.g., by detecting the upregulation of IL-17F, including but not limited to the use of such methods in human subjects. Due to the direct relationship between IL-21 and IL-17F, the invention also provides methods for diagnosing, prognosing, and monitoring the progress of disorders associated with IL-21 binding to and activation of IL-21R in a subject (e.g., that directly or indirectly involve increases in the bioactivity of IL-21) by detecting an upregulation of IL-17F activity, e.g., by detecting the upregulation of IL-17F, including but not limited to the use of such methods in human subjects. These methods may be performed by utilizing prepackaged diagnostic kits comprising at least one of the group comprising an IL-17F, IL-17R, or IL-17RC polynucleotide or fragments thereof, an IL-17F, IL-17R, or IL-17RC polypeptide or fragments thereof (including fusion proteins thereof), or antibodies to an IL-17F, IL-17R, or IL-17RC polypeptide or derivatives thereof, or modulators of IL-17F, IL-17R, or IL-17RC polynucleotides and/or polypeptides as described herein, which may be conveniently used, for example, in a clinical setting. A skilled artisan will recognize that other indirect methods may be used to confirm the upregulation of, e.g., IL-17F, such as counting the number of immune cells, e.g., neutrophils.

"Diagnostic" or "diagnosing" means identifying the presence or absence of a pathologic condition. Diagnostic methods include detecting upregulation of IL-17F signaling (and/or IL-21 signaling) by determining a test amount of the gene products (e.g., mRNA, cDNA, or polypeptide, including fragments thereof) of IL-17F, IL-17R, and/or IL-17RC in a biological sample from a subject (human or nonhuman mammal), and comparing the test amount with a normal amount or range (i.e., an amount or range from an individual(s) known not to suffer from disorders related to IL-17F signaling). Although a particular diagnostic method may not provide a definitive diagnosis of disorders related IL-17F signaling, it suffices if the method provides a positive indication that aids in diagnosis.

The present invention also provides methods for prognosing such disorders by detecting the upregulation of IL-17F activity, e.g., by detecting upregulation of IL-17F, IL-17R, or IL-17RC. "Prognostic" or "prognosing" means predicting the probable development and/or severity of a pathologic condition. Prognostic methods include determining the test amount of a gene product of IL-17F, IL-17R, or IL-17RC in a biological sample from a subject, and comparing the test amount to a prognostic amount or range (i.e., an amount or range from individuals with varying severities of disorders related to IL-17F signaling and/or disorders associated with IL-21 signaling) for the gene product of IL-17F, IL-17R, or IL-17RC, respectively. Various amounts of the IL-17F, IL-17R, or IL-17RC gene product in a test sample are consistent with certain prognoses for disorders related to IL-17F signaling and/or disorders associated with IL-21 signaling. The detection of an amount of IL-17F, IL-17R, or IL-17RC gene product at a particular prognostic level provides a prognosis for the subject.

The present invention also provides methods for monitoring the progress or course of such disorders related to IL-17F signaling (and/or disorders associated with IL-21 signaling) by detecting the upregulation of IL-17F activity, e.g., by detecting upregulation of TL-17F, IL-17R, or IL-17RC. Monitoring methods include determining the test amounts of a gene product of IL-17F, IL-17R, or IL-17RC in biological samples taken from a subject at a first and second time, and comparing the amounts. A change in amount of an IL-17F, IL-17R, or IL-17RC gene product between the first and second times indicates a change in the course of IL-17F signaling-related disorders (and/or IL-21 signaling-associated disorders), with a decrease in amount indicating remission of such disorders, and an increase in amount indicating progression of such disorders. Such monitoring assays are also useful for evaluating the efficacy of a particular therapeutic intervention in patients being treated for autoimmune disorders.

Increased IL-17F signaling in methods outlined above may be detected in a variety of biological samples, including bodily fluids (e.g., whole blood, plasma, and urine), cells (e.g., whole cells, cell fractions, and cell extracts), and other tissues. Biological samples also include sections of tissue, such as biopsies and frozen sections taken for histological purposes. Preferred biological samples include blood, plasma, lymph, tissue biopsies, urine, CSF (cerebrospinal fluid), synovial fluid, and BAL (bronchoalveolar lavage). It will be appreciated that analysis of a biological sample need not necessarily require removal of cells or tissue from the subject. For example, appropriately labeled agents that bind IL-17F signaling gene products (e.g., antibodies, nucleic acids) can be administered to a subject and visualized (when bound to the target) using standard imaging technology (e.g., CAT, NMR (MRI), and PET).

In the diagnostic and prognostic assays of the present invention, the IL-17F, IL-17R, or IL-17RC gene product is detected and quantified to yield a test amount. The test amount is then compared with a normal amount or range. An amount significantly above the normal amount or range is a positive sign in the diagnosis of disorders related to IL-17F signaling (and/or disorders associated with IL-21 binding to and activation of IL-21R). Particular methods of detection and quantitation of IL-17F, IL-17R, or IL-17RC gene products are described below.

Normal amounts or baseline levels of IL-17F, IL-17R, or IL-17RC gene products may be determined for any particular sample type and population. Generally, baseline (normal) levels of IL-17F, IL-17R, or IL-17RC protein or mRNA are determined by measuring respective amounts of IL-17F, IL-17R, or IL-17RC protein or mRNA in a biological sample type from normal (i.e., healthy) subjects. Alternatively, normal values of IL-17F, IL-17R, or IL-17RC gene products may be determined by measuring the amount in healthy cells or tissues taken from the same subject from which the diseased (or possibly diseased) test cells or tissues were taken. The amount of IL-17F, IL-17R, or IL-17RC gene products (either the normal amount or the test amount) may be determined or expressed on a per cell, per total protein, or per volume basis. To determine the cell amount of a sample, one can measure the level of a constitutively expressed gene product or other gene product expressed at known levels in cells of the type from which the biological sample was taken.

It will be appreciated that the assay methods of the present invention do not necessarily require measurement of absolute values of IL-17F, IL-17R, or IL-17RC gene products because relative values are sufficient for many applications of these methods. It will also be appreciated that in addition to the quantity or abundance of IL-17F, IL-17R, or IL-17RC gene products, variant or abnormal IL-17F, IL-17R, or IL-17RC gene products or their expression patterns (e.g., mutated transcripts, truncated polypeptides) may be identified by comparison to normal gene products and expression patterns.

Whether the expression of a particular gene in two samples is significantly similar or significantly different, e.g., significantly above or significantly below a given level, depends on the gene itself and, *inter alia,* its variability in expression between different individuals or different samples. It is within the skill in the art to determine whether expression levels are significantly similar or different. Factors such as genetic variation, e.g., in IL-17F and/or IL-17A expression levels, between individuals, species, organs, tissues, or cells may be taken into consideration (when and where necessary) when determining whether the level of expression, e.g., of IL-17F and/or IL-17A, between two samples is significantly similar or significantly different, e.g., significantly above a given level. As a result of the natural heterogeneity in gene expression between individuals, species, organs, tissues, or cells, phrase such as "significantly similar" or "significantly above" cannot be defined as a precise percentage or value, but rather can be ascertained by one skilled in the art upon practicing the invention.

The diagnostic, prognostic, and monitoring assays of the present invention involve detecting and quantifying IL-17F, IL-17R, or IL-17RC gene products in biological samples. IL-17F, IL-17R, or IL-17RC gene products include mRNAs and polypeptides, and both can be measured using methods well known to those skilled in the art.

For example, mRNA can be directly detected and quantified using hybridization-based assays, such as Northern hybridization, *in situ* hybridization, dot and slot blots, and oligonucleotide arrays. Hybridization-based assays refer to assays in which a probe nucleic acid is hybridized to a target nucleic acid. In some formats, the target, the probe, or both are immobilized. The immobilized nucleic acid may be DNA, RNA, or another oligonucleotide or polynucleotide, and may comprise naturally or nonnaturally occurring nucleotides, nucleotide analogs, or backbones. Methods of selecting nucleic acid probe sequences for use in the present invention are based on the nucleic acid sequence of IL-17F, IL-17R, or IL-17RC and are well known in the art.

Alternatively, mRNA can be amplified before detection and quantitation.
Such amplification-based assays are well known in the art and include polymerase chain reaction (PCR), reverse-transcription-PCR (RT-PCR), PCR-enzyme-linked immunosorbent assay (PCR-ELISA), and ligase chain reaction (LCR). Primers and probes for producing and detecting amplified IL-17F, IL-17R, or IL-17RC gene products (e.g., mRNA or cDNA) may be readily designed and produced without undue experimentation by those of skill in the art based on the nucleic acid sequences of IL-17F, IL-17R, or IL-17RC, respectively. As a nonlimiting example, amplified IL-17F gene products may be directly analyzed, for example, by gel electrophoresis; by hybridization to a probe nucleic acid; by sequencing; by detection of a fluorescent, phosphorescent, or radioactive signal; or by any of a variety of well-known methods. In addition, methods are known to those of skill in the art for increasing the signal produced by amplification of target nucleic acid sequences. One of skill in the art will recognize that whichever amplification method is used, a variety of quantitative methods known in the art (e.g., quantitative PCR) may be used if quantitation of gene products is desired.

IL-17F, IL-17R, or IL-17RC polypeptides (or fragments thereof) may be detected using various well-known immunological assays employing the respective anti-IL-17F, anti-IL-17R, or anti-IL-17RC antibodies that may be generated as described above. Immunological assays refer to assays that utilize an antibody (e.g., polyclonal, monoclonal, chimeric, humanized, scFv, and/or fragments thereof) that specifically binds to, e.g., an IL-17F polypeptide (or a fragment thereof). Such well-known immunological assays suitable for the practice of the present invention include ELISA, radioimmunoassay (RIA), immunoprecipitation, immunofluorescence, fluorescence-activated cell sorting (FACS), and Western blotting. An IL-17F polypeptide may also be detected using a labeled IL-17R and/or IL-17RC polypeptide(s). Conversely, IL-17R or IL-17RC may be detected using a labeled IL-17F polypeptide.

One of skill in the art will understand that the aforementioned methods may be applied to disorders related to IL-17F signaling.

### Uses of Molecules Related to IL-17F Signaling in Therapy

The present inventors are the first to demonstrate, *inter alia,* the following: 1) binding of IL-17R and/or IL-17RC by IL-17F, or other IL-17F agonists, is correlated with increased neutrophil infiltration, cartilage destruction, etc.; 2) antibodies directed toward IL-17F may be used to detect IL-17F protein and to inhibit at least one IL-17F bioactivity; 3) siRNAs directed to IL-17R and IL-17RC may be used to decrease IL-17A and IL-17F bioactivity; 4) IL-17F protein may form an IL-17F homodimer and an IL-17A/IL-17F heterodimer, and thus, inhibitory antibodies directed toward IL-17F may also inhibit IL-17A bioactivity that is mediated by IL-17A/IL-17F heterodimers; 5) IL-21 acts synergistically with CD28 to upregulate IL-17A homodimers, IL-17F homodimers, and IL-17A/IL-17F heterodimers, and thus antibodies that inhibit IL-17F activity (e.g., IL-17F homodimer activity and/or IL-17A/IL-17F heterodimer activity) may regulate IL-21 signal; 6) natural and recombinant IL-17A homodimers, IL-17F homodimers, and IL-17A/IL-17F heterodimers may be isolated and purified; 7) IL-17F heterodimers possess IL-17F bioactivity; 8) antibodies against human IL-17F cross react with and partially neutralize primate IL-17F; and 9) both IL-17F and IL-17A are increased in lesional tissues from human patients with psoriasis and involved tissues in human patients with Crohn's disease and ulcerative colitis. Although some animal models have been used to identify some of the above correlations, it is well known in the art that immunological mechanisms studied in animal models may be, and often are, translatable to the human immune system. Additionally, the antibodies of the invention were used to isolate natural IL-17F in its homodimeric and heterodimeric forms from primary human T cells, while the experiments involving IL-17F regulation of Aggrecanase and profiling the expression levels of IL-17A and IL-17F in psoriatic lesions and involved tissues in inflammatory bowel disease were undertaken in human cells and tissues. As such, the disclosed methods for using molecules related to IL-17F signaling, e.g., IL-17F agonists or IL-17F signaling antagonists, to treat disorders related to IL-17F signaling and/or disorders associated with IL-21 signaling, will be particularly useful for treating such disorders in humans.

The IL-17F signaling-related molecules disclosed herein, including modulators of IL-17F, IL-17R, or IL-17RC polynucleotide and/or polypeptide activity identified using the methods described above, may be used *in vitro, ex vivo,* or incorporated into pharmaceutical compositions and administered to individuals *in vivo* to treat, for example, disorders related to IL-17F signaling and/or IL-21 signaling, by administration of an IL-17F signaling antagonist (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or anti-IL-17RC antibodies; and/or antagonistic small molecules, etc.). Several pharmacogenomic approaches to be considered in determining whether to administer IL-17F signaling-related molecules are well known to one of skill in the art and include genome-wide association, candidate gene approach, and gene expression profiling. A pharmaceutical composition of the invention is formulated to be compatible with its intended route of administration (e.g., oral compositions generally include an inert diluent or an edible carrier). Other nonlimiting examples of routes of administration include parenteral (e.g., intravenous), intradermal, subcutaneous, oral (e.g., inhalation), transdermal (topical), transmucosal, and rectal administration. The pharmaceutical compositions compatible with each intended route are well known in the art.

IL-17F agonists or IL-17F signaling antagonists may be used as pharmaceutical compositions when combined with a pharmaceutically acceptable carrier. Such a composition may contain, in addition to an IL-17F signaling-related molecules (e.g., IL-17F agonists or IL-17F signaling antagonists) and carrier, various diluents, fillers, salts, buffers, stabilizers, solubilizers, and other materials well known in the art. The term "pharmaceutically acceptable" means a nontoxic material that does not interfere with the effectiveness of the biological activity of the active ingredient(s). The characteristics of the carrier will depend on the route of administration.

The pharmaceutical composition of the invention may also contain cytokines, lymphokines, or other hematopoietic factors such as M-CSF, GM-CSF, IL-1, IL-2, IL-3, IL-4, IL-5, IL-6, IL-7, IL-8, IL-9, IL-10, IL-11, IL-12, IL-14, IL-15, G-CSF, stem cell factor, and erythropoietin. The pharmaceutical composition may also include anticytokine antibodies as described in more detail below. The pharmaceutical composition may contain thrombolytic or antithrombotic factors such as plasminogen activator and Factor VIII. The pharmaceutical composition may further contain other anti-inflammatory agents as described in more detail below. Such additional factors and/or agents may be included in the pharmaceutical composition to produce a synergistic effect with IL-17F agonists or IL-17F signaling antagonists, or to minimize side effects caused by the IL-17F agonists or IL-17F signaling antagonists. Conversely IL-17F agonists or IL-17F signaling antagonists may be included in formulations of the particular cytokine, lymphokine, other hematopoietic factor, thrombolytic or antithrombotic factor, or anti-inflammatory agent to minimize side effects of the cytokine, lymphokine, other hematopoietic factor, thrombolytic or antithrombotic factor, or anti-inflammatory agent.

The pharmaceutical composition of the invention may be in the form of a liposome in which IL-17F agonists or IL-17F signaling antagonists are combined, in addition to other pharmaceutically acceptable carriers, with amphipathic agents such as lipids that exist in aggregated form as micelles, insoluble monolayers, liquid crystals, or lamellar layers in aqueous solution. Suitable lipids for liposomal formulation include, without limitation, monoglycerides, diglycerides, sulfatides, lysolecithin, phospholipids, saponin, bile acids, etc.

As used herein, the term "therapeutically effective amount" means the total amount of each active component of the pharmaceutical composition or method that is sufficient to show a meaningful patient benefit, e.g., amelioration of symptoms of, healing of, or increase in rate of healing of such conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

In practicing the method of treatment or use of the present invention, a therapeutically effective amount of an IL-17F agonist or IL-17F signaling antagonist is administered to a subject, e.g., a mammal (e.g., a human). An IL-17F signaling-related molecule may be administered in accordance with the method of the invention either alone or in combination with other therapies, such as treatments employing cytokines, lymphokines or other hematopoietic factors, or anti-inflammatory agents. When coadministered with one or more agents, IL-17F signaling antagonists may be administered either simultaneously with the second agent, or sequentially. If administered sequentially, the attending physician will decide on the appropriate sequence of administering, e.g., an IL-17R and/or IL-17RC polypeptide (or fusion protein thereof) and/or inhibiting antibody in combination with other agents.

When a therapeutically effective amount of an IL-17F agonist or IL-17F signaling antagonist is administered orally, the binding agent will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% binding agent, and preferably from about 25 to 90% binding agent. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol, or polyethylene glycol. When administered in liquid form, the pharmaceutical composition contains from about 0.5 to 90% by weight of the binding agent, and preferably from about 1 to 50% by weight of the binding agent.

When a therapeutically effective amount of an IL-17F agonist or IL-17F signaling antagonist is administered by intravenous, cutaneous or subcutaneous injection, the IL-17F agonist or IL-17F signaling antagonist will be in the form of a pyrogen-free, parenterally acceptable aqueous solution. The preparation of such parenterally acceptable protein solutions, having due regard to pH, isotonicity, stability, and the like, is within the skill of those in the art. A preferred pharmaceutical composition for intravenous, cutaneous, or subcutaneous injection should contain, in addition to the IL-17F agonist or IL-17F signaling antagonist, an isotonic vehicle such as sodium chloride injection, Ringer's injection, dextrose injection, dextrose and sodium chloride injection, lactated Ringer's injection, or other vehicle as known in the art. The pharmaceutical composition of the present invention may also contain stabilizers, preservatives, buffers, antioxidants, or other additive known to those of skill in the art.

The amount of an IL-17F agonist or IL-17F signaling antagonist in the pharmaceutical composition of the present invention will depend upon the nature and severity of the condition being treated, and on the nature of prior treatments that the patient has undergone. Ultimately, the attending physician will decide the amount of IL-17F agonist or IL-17F signaling antagonist with which to treat each individual patient. Initially, the attending physician will administer low doses of IL-17F agonist or IL-17F signaling antagonist and observe the patient's response. Larger doses of IL-17F agonist or IL-17F signaling antagonist may be administered until the optimal therapeutic effect is obtained for the patient, and at that point the dosage is not generally increased further. It is contemplated that the various pharmaceutical compositions used to practice the method of the present invention should contain about 0.1 µg to about 100 mg of IL-17F agonist or IL-17F signaling antagonist, e.g., IL-17R and/or IL-17RC (including fusion proteins thereof), per kg body weight.

The duration of intravenous (i.v.) therapy using a pharmaceutical composition of the present invention will vary, depending on the severity of the disease being treated and the condition and potential idiosyncratic response of each individual patient. It is contemplated that the duration of each application of the IL-17F agonist or IL-17F signaling antagonist may be in the range of 12 to 24 hours of continuous i.v. administration. Also contemplated is subcutaneous (s.c.) therapy using a pharmaceutical composition of the present invention. These therapies can be administered daily, weekly, or, more preferably, biweekly, or monthly. It is also contemplated that where the IL-17F agonist or IL-17F signaling antagonist is a small molecule (e.g., for oral delivery), the therapies may be administered daily, twice a day, three times a day, etc. Ultimately the attending physician will decide on the appropriate duration of i.v. or s.c. therapy, or therapy with a small molecule, and the timing of administration of the therapy, using the pharmaceutical composition of the present invention.

The polynucleotides and proteins of the present invention are expected to exhibit one or more of the uses or biological activities (including those associated with assays cited herein) identified below. Uses or activities described for proteins of the present invention may be provided by administration or use of such proteins or by administration or use of polynucleotides encoding such proteins (such as, for example, in gene therapies or vectors suitable for introduction of DNA).

### Uses of IL-17F Signaling Antagonists to Decrease Inflammation

In one aspect, the invention features a method of decreasing an inflammatory response, e.g., due to IL-21 signaling. The method may comprise contacting a population of cells with an IL-17F signaling antagonist (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) in an amount sufficient to inhibit the IL-17F activity of the cell or population. Antagonists to IL-17F signaling may also be administered to subjects for whom suppression of IL-17F signaling (and/or IL-21 signaling) is desired. These conditions include, but are not limited to, inflammatory disorders, e.g., autoimmune diseases (e.g., arthritis (including rheumatoid arthritis), psoriasis, systemic lupus erythematosus, multiple sclerosis), respiratory diseases (e.g., COPD, cystic fibrosis, asthma, allergy), transplant rejection (including solid organ transplant rejection), and inflammatory bowel diseases (e.g., ulcerative colitis, Crohn's disease).

These methods are based, at least in part, on the finding that interfering with IL-17F signaling, e.g., by using an interfering anti-IL-17F antibody, decreases IL-17F-associated inflammatory responses, e.g., cytokine production by primary fibroblast-like synoviocytes (Example 4.2). Accordingly, IL-17F signaling antagonists, i.e., molecules that inhibit IL-17F activity (e.g., anti-IL-17F antibodies) may be used to decrease inflammation *in vivo,* e.g., for treating or preventing disorders related to IL-17F signaling and/or disorders related to IL-21 signaling.

The methods of using IL-17F signaling antagonists may also be used inhibit IL-17F inflammatory activity and thus, can be used to treat or prevent a variety of immune disorders. Nonlimiting examples of the disorders that can be treated or prevented include, but are not limited to, transplant rejection, autoimmune diseases (including, for example, diabetes mellitus, arthritis (including rheumatoid arthritis, juvenile rheumatoid arthritis, osteoarthritis, psoriatic arthritis, reactive arthritis), multiple sclerosis, encephalomyelitis, myasthenia gravis, systemic lupus erythematosus (SLE), autoimmune thyroiditis, dermatitis (including atopic dermatitis and eczematous dermatitis), Reiter's syndrome, psoriasis, Sjögren's syndrome, Crohn's disease, aphthous ulcer, iritis, conjunctivitis, keratoconjunctivitis, ulcerative colitis, spondyloarthropathy, ankylosing spondylitis, intrinsic asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior,
and interstitial lung fibrosis), graft-versus-host disease, pulmonary exacerbation (e.g., due to bacterial infection), and allergy, such as atopic allergy. Preferred disorders that can be treated using methods which comprise the administration of IL-17F signaling antagonists, e.g., an inhibitory IL-17F antibody, include, but are not limited to, inflammatory disorders, e.g., autoimmune diseases (e.g., arthritis (including rheumatoid arthritis), psoriasis, systemic lupus erythematosus, multiple sclerosis), respiratory diseases (e.g., COPD, cystic fibrosis, asthma, allergy), transplant rejection (including solid organ transplant rejection), and inflammatory bowel diseases (e.g., ulcerative colitis, Crohn's disease).

Using IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.), it is possible to modulate immune responses in a number of ways. Downregulation may be in the form of inhibiting or blocking an inflammatory response already in progress, or may involve preventing the induction of an inflammatory response.

In one embodiment, IL-17F signaling antagonists, including pharmaceutical compositions thereof, are administered in combination therapy, i.e., combined with other agents, e.g., therapeutic agents, that are useful for treating pathological conditions or disorders, such as immune disorders and inflammatory diseases. The term "in combination" in this context means that the agents are given substantially contemporaneously, either simultaneously or sequentially. If given sequentially, at the onset of administration of the second compound, the first of the two compounds is preferably still detectable at effective concentrations at the site of treatment.

For example, the combination therapy can include one or more IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) coformulated with, and/or coadministered with, one or more additional therapeutic agents, e.g., one or more cytokine and growth factor inhibitors, immunosuppressants, anti-inflammatory agents, metabolic inhibitors, enzyme inhibitors, and/or cytotoxic or cytostatic agents, as described in more detail below. Furthermore, one or more IL-17F signaling antagonists described herein may be used in combination with two or more of the therapeutic agents described herein. Such combination therapies may advantageously utilize lower dosages of the administered therapeutic agents, thus avoiding possible toxicities or complications associated with the various monotherapies. Moreover, the therapeutic agents disclosed herein act on pathways that differ from the IL-17F receptor signaling pathway, and thus are expected to enhance and/or synergize with the effects of the IL-17F signaling antagonists.

Preferred therapeutic agents used in combination with an IL-17F signaling antagonist are those agents that interfere at different stages in an inflammatory response. In one embodiment, one or more IL-17F signaling antagonists described herein may be coformulated with, and/or coadministered with, one or more additional agents such as other cytokine or growth factor antagonists (e.g., soluble receptors, peptide inhibitors, small molecules, ligand fusions); or antibodies or antigen binding fragments thereof that bind to other targets (e.g., antibodies that bind to other cytokines or growth factors, their receptors, or other cell surface molecules); and anti-inflammatory cytokines or agonists thereof. Nonlimiting examples of the agents that can be used in combination with the IL-17F signaling antagonists described herein, include, but are not limited to, antagonists of one or more interleukins (ILs) or their receptors, e.g., antagonists of IL-1, IL-2, IL-6, IL-7, IL-8, IL-12, IL-13, IL-15, IL-16, IL-18, IL-21 and IL-22; antagonists of cytokines or growth factors or their receptors, such as tumor necrosis factor (TNF), LT, EMAP-II, GM-CSF, FGF and PDGF. IL-17F signaling antagonists can also be combined with inhibitors of, e.g., antibodies to, cell surface molecules such as CD2, CD3, CD4, CD8, CD20 (e.g., the CD20 inhibitor rituximab (RITUXAN^{®})), CD25, CD28, CD30, CD40, CD45, CD69, CD80 (B7.1), CD86 (B7.2), CD90, or their ligands, including CD154 (gp39 or CD40L), or LFA-1/ICAM-1 and VLA-4/VCAM-1 (Yusuf-Makagiansar et al. (2002) Med. Res. Rev. 22:146-67). Preferred antagonists that can be used in combination with IL-17F signaling antagonists described herein include antagonists of IL-1, IL-12, TNFα, IL-15, IL-18, and IL-22.

Examples of those agents include IL-12 antagonists, such as chimeric, humanized, human or in *vitro*-generated antibodies (or antigen binding fragments thereof) that bind to IL-12 (preferably human IL-12), e.g., the antibody disclosed in WO 00/56772; IL-12 receptor inhibitors, e.g., antibodies to human IL-12 receptor; and soluble fragments of the IL-12 receptor, e.g., human IL-12 receptor. Examples ofIL-15 antagonists include antibodies (or antigen binding fragments thereof) against IL-15 or its receptor, e.g., chimeric, humanized, human or *in* vitro-generated antibodies to human IL-15 or its receptor, soluble fragments of the IL-15 receptor, and IL-15-binding proteins. Examples of IL-18 antagonists include antibodies, e.g., chimeric, humanized, human or in vitro-generated antibodies (or antigen binding fragments thereof), to human IL-18, soluble fragments of the IL-18 receptor, and IL-18 binding proteins (IL-18BP). Examples of IL-1 antagonists include Interleukin-1-converting enzyme (ICE) inhibitors, such as Vx740, IL-1 antagonists, e.g., IL-1RA (anikinra, KINERET^{™}, Amgen), sIL1RII (Immunex), and anti-IL-1 receptor antibodies (or antigen binding fragments thereof).

Examples of TNF antagonists include chimeric, humanized, human or *in* vitro-generated antibodies (or antigen binding fragments thereof) to TNF (e.g., human TNFα), such as (HUMIRA^{™}, D2E7, human TNFα antibody), CDP-571/CDP-870/BAY-10-3356 (humanized anti-TNFα antibody; Celltech/Pharmacia), cA2 (chimeric anti-TNFα antibody; REMICADE^{®}, Centocor); anti-TNF antibody fragments (e.g., CPD870); soluble fragments of the TNF receptors, e.g., p55 or p75 human TNF receptors or derivatives thereof, e.g., 75 kdTNFR-IgG (75 kD TNF receptor-IgG fusion protein, ENBREL^{™}; Immunex), p55 kdTNFR-IgG (55 kD TNF receptor-IgG fusion protein (LENERCEPT^{®})); enzyme antagonists, e.g., TNFα converting enzyme (TACE) inhibitors (e.g., an alpha-sulfonyl hydroxamic acid derivative, and N-hydroxyformamide TACE inhibitor GW 3333, -005, or -022); and TNF-bp/s-TNFR (soluble TNF binding protein). Preferred TNF antagonists are soluble fragments of the TNF receptors, e.g., p55 or p75 human TNF receptors or derivatives thereof, e.g., 75 kdTNFR-IgG, and TNFα converting enzyme (TACE) inhibitors.

In other embodiments, the IL-17F signaling antagonists described herein may be administered in combination with one or more of the following: IL-13 antagonists, e.g., soluble IL-13 receptors (sIL-13) and/or antibodies against IL-13; IL-2 antagonists, e.g., DAB 486-IL-2 and/or DAB 389-IL-2 (IL-2 fusion proteins, Seragen), and/or antibodies to IL-2R, e.g., anti-Tac (humanized anti-IL-2R, Protein Design Labs). Yet another combination includes IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.), antagonistic small molecules, and/or inhibitory antibodies in combination with nondepleting anti-CD4 inhibitors (IDEC-CE9.1/SB 210396; nondepleting primatized anti-CD4 antibody; IDEC/SmithKline). Yet other preferred combinations include antagonists of the costimulatory pathway CD80 (B7.1) or CD86 (B7.2), including antibodies, soluble receptors or antagonistic ligands; as well as p-selectin glycoprotein ligand (PSGL), anti-inflammatory cytokines, e.g., IL-4 (DNAX/Schering); IL-10 (SCH 52000; recombinant IL-10 DNAX/Schering); IL-13 and TGF-β, and agonists thereof (e.g., agonist antibodies).

In other embodiments, one or more IL-17F signaling antagonists can be coformulated with, and/or coadministered with, one or more anti-inflammatory drugs, immunosuppressants, or metabolic or enzymatic inhibitors. Nonlimiting examples of the drugs or inhibitors that can be used in combination with the IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) described herein, include, but are not limited to, one or more of: nonsteroidal anti-inflammatory drug(s) (NSAIDs), e.g., ibuprofen, tenidap, naproxen, meloxicam, piroxicam, diclofenac, and indomethacin; sulfasalazine; corticosteroids such as prednisolone; cytokine suppressive anti-inflammatory drug(s) (CSAIDs); inhibitors of nucleotide biosynthesis, e.g., inhibitors of purine biosynthesis, folate antagonists (e.g., methotrexate (N-[4-[[(2,4-diamino-6-pteridinyl)methyl] methylamino] benzoyl]-L-glutamic acid); and inhibitors of pyrimidine biosynthesis, e.g., dihydroorotate dehydrogenase (DHODH) inhibitors. Preferred therapeutic agents for use in combination with IL-17F signaling antagonists include NSAIDs, CSAIDs, (DHODH) inhibitors (e.g., leflunomide), and folate antagonists (e.g., methotrexate).

Examples of additional inhibitors include one or more of: corticosteroids (oral, inhaled and local injection); immunosuppresants, e.g., cyclosporin, tacrolimus (FK-506); and mTOR inhibitors, e.g., sirolimus (rapamycin - RAPAMUNE^{™} or rapamycin derivatives, e.g., soluble rapamycin derivatives (e.g., ester rapamycin derivatives, e.g., CCI-779); agents which interfere with signaling by proinflammatory cytokines such as TNFα or IL-1 (e.g. IRAK, NIK, IKK, p38 or MAP kinase inhibitors); COX2 inhibitors, e.g., celecoxib, rofecoxib, and variants thereof; phosphodiesterase inhibitors, e.g., R973401 (phosphodiesterase Type IV inhibitor); phospholipase inhibitors, e.g., inhibitors of cytosolic phospholipase 2 (cPLA2) (e.g., trifluoromethyl ketone analogs); inhibitors of vascular endothelial cell growth factor or growth factor receptor, e.g., VEGF inhibitor and/or VEGF-R inhibitor; and inhibitors of angiogenesis. Preferred therapeutic agents for use in combination with IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) are immunosuppresants, e.g., cyclosporin, tacrolimus (FK-506); mTOR inhibitors, e.g., sirolimus (rapamycin) or rapamycin derivatives, e.g., soluble rapamycin derivatives (e.g., ester rapamycin derivatives, e.g., CCI-779); COX2 inhibitors, e.g., celecoxib and variants thereof; and phospholipase inhibitors, e.g., inhibitors of cytosolic phospholipase 2 (cPLA2), e.g., trifluoromethyl ketone analogs.

Additional examples of therapeutic agents that can be combined with an IL-17F signaling antagonist include one or more of: 6-mercaptopurines (6-MP); azathioprine sulphasalazine; mesalazine; olsalazine; chloroquine/ hydroxychloroquine (PLAQUENIL^{®}); pencillamine; aurothiornalate (intramuscular and oral); azathioprine; colchicine; beta-2 adrenoreceptor agonists (salbutamol, terbutaline, salmeteral); xanthines (theophylline, arninophylline); cromoglycate; nedocromil; ketotifen; ipratropium and oxitropium; mycophenolate mofetil; adenosine agonists; antithrombotic agents; complement inhibitors; and adrenergic agents.

The use of the IL-17F signaling antagonists disclosed herein in combination with other therapeutic agents to treat or prevent specific disorders related to IL-17F signaling is discussed in further detail below.

Nonlimiting examples of agents for treating or preventing arthritic disorders (e.g., rheumatoid arthritis, inflammatory arthritis, rheumatoid arthritis, juvenile rheumatoid arthritis, osteoarthritis and psoriatic arthritis), with which IL-17F signaling antagonists may be combined include one or more of the following: IL-12 antagonists as described herein; NSAIDs; CSAIDs; TNFs, e.g., TNFα, antagonists as described herein; nondepleting anti-CD4 antibodies as described herein; IL-2 antagonists as described herein; anti-inflammatory cytokines, e.g., IL-4, IL-10, IL- 13 and TGFα, or agonists thereof; IL-1 or IL-1 receptor antagonists as described herein; phosphodiesterase inhibitors as described herein; Cox-2 inhibitors as described herein; iloprost: methotrexate; thalidomide and thalidomide-related drugs (e.g., Celgen); leflunomide; inhibitor of plasminogen activation, e.g., tranexamic acid; cytokine inhibitor, e.g., T-614; prostaglandin E1; azathioprine; an inhibitor of interleukin-1 converting enzyme (ICE); zap-70 and/or lck inhibitor (inhibitor of the tyrosine kinase zap-70 or lck); an inhibitor of vascular endothelial cell growth factor or vascular endothelial cell growth factor receptor as described herein; an inhibitor of angiogenesis as described herein; corticosteroid anti-inflammatory drugs (e.g., SB203580); TNF-convertase inhibitors; IL-11; IL-13; IL-17 inhibitors; gold; penicillamine; chloroquine; hydroxychloroquine; chlorambucil; cyclophosphamide; cyclosporine; total lymphoid irradiation; antithymocyte globulin; CD5-toxins; orally administered peptides and collagen; lobenzarit disodium; cytokine regulating agents (CRAs) HP228 and HP466 (Houghten Pharmaceuticals, Inc.); ICAM-1 antisense phosphorothioate oligodeoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline (MINOCIN^{®}); anti-IL2R antibodies; marine and botanical lipids (fish and plant seed fatty acids); auranofm; phenylbutazone; meclofenamic acid; flufenamic acid; intravenous immune globulin; zileuton; mycophenolic acid (RS-61443); tacrolimus (FK-506); sirolimus (rapamycin); amiprilose (therafectin); cladribine (2-chlorodeoxyadenosine); and azaribine. Preferred combinations include one or more IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) in combination with methotrexate or leflunomide, and in moderate or severe rheumatoid arthritis cases, cyclosporine.

Preferred examples of inhibitors to use in combination with IL-17F signaling antagonists to treat arthritic disorders include TNF antagonists (e.g., chimeric, humanized, human or *in vitro*-generated antibodies, or antigen binding fragments thereof, that bind to TNF; soluble fragments of a TNF receptor, e.g., p55 or p75 human TNF receptor or derivatives thereof, e.g., 75 kdTNFR-IgG (75 kD TNF receptor-IgG fusion protein, ENBREL^{™}), p55 kD TNF receptor-IgG fusion protein; TNF enzyme antagonists, e.g., TNFα converting enzyme (TACE) inhibitors); antagonists of IL-12, IL-15, IL-18, IL-22; T cell and B cell-depleting agents (e.g., anti-CD4 or anti-CD22 antibodies); small molecule inhibitors, e.g., methotrexate and leflunomide; sirolimus (rapamycin) and analogs thereof, e.g., CCI-779; cox-2 and cPLA2 inhibitors; NSAIDs; p38 inhibitors, TPL-2, Mk-2 and NFkb inhibitors; RAGE or soluble RAGE; P-selectin or PSGL-1 inhibitors (e.g., small molecule inhibitors, antibodies thereto, e.g., antibodies to P-selectin); estrogen receptor beta (ERB) agonists or ERB-NFkb antagonists. Most preferred additional therapeutic agents that can be coadministered and/or coformulated with one or more IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) include one or more of: a soluble fragment of a TNF receptor, e.g., p55 or p75 human TNF receptor or derivatives thereof, e.g., 75 kdTNFR-IgG (75 kD TNF receptor-IgG fusion protein, ENBREL^{™}); methotrexate, leflunomide, or a sirolimus (rapamycin) or an analog thereof, e.g., CCI-779.

Nonlimiting examples of agents for treating or preventing multiple sclerosis with which IL-17F signaling antagonists can be combined include the following: interferons, e.g., interferon-alphala (e.g., AVONEX™; Biogen) and interferon-1b (BETASERON^{™} Chiron/Berlex); Copolymer 1 (Cop-1; COPAXONE^{™} Teva Pharmaceutical Industries, Inc.); hyperbaric oxygen; intravenous immunoglobulin; cladribine; TNF antagonists as described herein; corticosteroids; prednisolone; methylprednisolone; azathioprine; cyclophosphamide; cyclosporine; cyclosporine A, methotrexate; 4-aminopyridine; and tizanidine. Additional antagonists that can be used in combination with IL-17F signaling antagonists include antibodies to or antagonists of other human cytokines or growth factors, for example, TNF, LT, IL-1, IL-2, IL-6, IL-7, IL-8, IL-12 IL-15, IL-16, IL-18, EMAP-11, GM-CSF, FGF, and PDGF. IL-17F signaling antagonists as described herein can be combined with antibodies to cell surface molecules such as CD2, CD3, CD4; CD8, CD25, CD28, CD30, CD40, CD45, CD69, CD80, CD86, CD90 or their ligands. The IL-17F signaling antagonists may also be combined with agents, such as methotrexate, cyclosporine, FK506, rapamycin, mycophenolate mofetil, leflunomide, NSAIDs, for example, ibuprofen, corticosteroids such as prednisolone, phosphodiesterase inhibitors, adenosine agonists, antithrombotic agents, complement inhibitors, adrenergic agents, agents which interfere with signaling by proinflammatory cytokines as described herein, IL-Ib converting enzyme inhibitors (e.g., Vx740), anti-P7s, PSGL, TACE inhibitors, T-cell signaling inhibitors such as kinase inhibitors, metalloproteinase inhibitors, sulfasalazine, azathloprine, 6-mercaptopurines, angiotensin converting enzyme inhibitors, soluble cytokine receptors and derivatives thereof, as described herein, and anti-inflammatory cytokines (e.g. IL-4, IL-10, IL-13 and TGF).

Preferred examples of therapeutic agents for multiple sclerosis with which the IL-17F signaling antagonists can be combined include interferon-β, for example, IFNβ-1a and IFNβ-1b; copaxone, corticosteroids, IL- I inhibitors, TNF inhibitors, antibodies to CD40 ligand and CD80, IL-12 antagonists.

Nonlimiting examples of agents for treating or preventing inflammatory bowel disease (e.g., Crohn's disease, ulcerative colitis) with which a IL-17F signaling antagonist (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) can be combined include the following: budenoside; epidermal growth factor; corticosteroids; cyclosporine; sulfasalazine; aminosalicylates; 6-mercaptopurine; azathioprine; , metronidazole; lipoxygenase inhibitors; mesalamine; olsalazine; balsalazide; antioxidants; thromboxane inhibitors; IL-1 receptor antagonists; anti-IL-1 monoclonal antibodies; anti-IL-6 monoclonal antibodies; growth factors; elastase inhibitors; pyridinyl-imidazole compounds; TNF antagonists as described herein; IL-4, IL-10, IL-13 and/or TGFβ cytokines or agonists thereof (e.g., agonist antibodies); IL-11; glucuronide- or dextran-conjugated prodrugs of prednisolone, dexamethasone or budesonide; ICAM-1 antisense phosphorothioate oligodeoxynucleotides (ISIS 2302; Isis Pharmaceuticals, Inc.); soluble complement receptor 1 (TP10; T Cell Sciences, Inc.); slow-release mesalazine; methotrexate; antagonists of platelet activating factor (PAF); ciprofloxacin; and lignocaine.

In one embodiment, an IL-17F signaling antagonist (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) can be used in combination with one or more antibodies directed at other targets involved in regulating immune responses, e.g., transplant rejection. Nonlimiting examples of agents for treating or preventing immune responses with which an IL-17F signaling antagonist of the invention can be combined include the following: antibodies against other cell surface molecules, including but not limited to CD25 (interleukin-2 receptor-a), CD1 1a (LFA-1), CD54 (ICAM-1), CD4, CD45, CD28/CTLA4 (CD80 (B7.1), e.g., CTLA4 Ig-abatacept (ORENCIA^{®})), ICOSL, ICOS and/or CD86 (B7.2). In yet another embodiment, an IL-17F signaling antagonist is used in combination with one or more general immunosuppressive agents, such as cyclosporin A or FK506.

In other embodiments, IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) are used as vaccine adjuvants against autoimmune disorders, inflammatory diseases, etc. The combination of adjuvants for treatment of these types of disorders are suitable for use in combination with a wide variety of antigens from targeted self-antigens, i.e., autoantigens, involved in autoimmunity, e.g., myelin basic protein; inflammatory self-antigens, e.g., amyloid peptide protein, or transplant antigens, e.g., alloantigens. The antigen may comprise peptides or polypeptides derived from proteins, as well as fragments of any of the following: saccharides, proteins, polynucleotides or oligonucleotides, autoantigens, amyloid peptide protein, transplant antigens, allergens, or other macromolecular components. In some instances, more than one antigen is included in the antigenic composition.

For example, desirable vaccines for moderating responses to allergens in a vertebrate host, which contain the adjuvant combinations of this invention, include those containing an allergen or fragment thereof. Examples of such allergens are described in U.S. Patent No. 5,830,877 and published International Patent Application No. WO 99/51259, which are hereby incorporated by reference in their entireties, and include pollen, insect venoms, animal dander, fungal spores and drugs (such as penicillin). The vaccines interfere with the production of IgE antibodies, a known cause of allergic reactions. In another example, desirable vaccines for preventing or treating disease characterized by amyloid deposition in a vertebrate host, which contain the adjuvant combinations of this invention, include those containing portions of amyloid peptide protein (APP). This disease is referred to variously as Alzheimer's disease, amyloidosis or amyloidogenic disease. Thus, the vaccines of this invention include the adjuvant combinations of this invention plus Aβ peptide, as well as fragments of Aβ peptide and antibodies to Aβ peptide or fragments thereof.

Methods of: 1) downregulating antigen presenting cell function; and 2) combination therapy for managing immunosuppression are well known in the art (see, e.g., Xiao et al. (2003) BioDrugs 17:103-11; Kuwana (2002) Hum. Immunol. 63:1156-63; Lu et al. (2002) Transplantation 73:S19-S22; Rifle et al. (2002) Transplantation 73:S1-S2; Mancini et al. (2004) Crit. Care. Nurs. Q. 27:61-64).

Another aspect of the present invention accordingly relates to kits for carrying out the administration of IL-17F signaling antagonists (e.g., IL-17F, IL-17R, and/or IL-17RC inhibitory polynucleotides; soluble IL-17R and/or IL-17RC polypeptides (including fragments and/or fusion proteins thereof); inhibitory anti-IL-17F, anti-IL-17R, or IL-17RC antibodies; and/or antagonistic small molecules, etc.) with other therapeutic compounds. In one embodiment, the kit comprises one or more binding agents formulated in a pharmaceutical carrier, and at least one agent, e.g., therapeutic agent, formulated as appropriate, in one or more separate pharmaceutical preparations.

The entire contents of all references, patents, and published patent applications cited throughout this application are hereby incorporated by reference herein.

### EXAMPLES

The following Examples provide illustrative embodiments of the invention and do not in any way limit the invention. One of ordinary skill in the art will recognize that numerous other embodiments are encompassed within the scope of the invention.

The Examples do not include detailed descriptions of conventional methods, such methods employed in the construction of vectors, the insertion of genes encoding the polypeptides into such vectors and plasmids, the introduction of such vectors and plasmids into host cells, and the expression of polypeptides from such vectors and plasmids in host cells. Such methods are well known to those of ordinary skill in the art.

### Example 1: IL-17F-mediated inflammatory responses and implications in inflammatory disorders, e.g., rheumatoid arthritis, inflammatory respiratory disorders, and inflammatory bowel disease

### Example 1.1: Human IL-17F administration in naïve mice induces neutrophil influx into the peritoneum

The observation that IL-17F treatment results in increased proteoglycan breakdown and decreased proteoglycan synthesis by articular cartilage (Hymowitz et al. (2001) EMBO J. 20:5332-41) suggests a role for IL-17F signaling in the development of inflammatory diseases of joint tissue. Indeed, synovial fluid samples from patients with rheumatoid arthritis and osteoarthritis show degradation of proteoglycans including, e.g., aggrecan, keratin, and collagen (*see, e.g.,* Witter et al. (1987) Arth. Rheum. 30:519-29 and Yagi et al. (2005) J. Orthop. Res. 23(5):1128-38), and *in vitro* arthritis models mimic this phenomenon by displaying matrix and proteoglycan degradation (Neidhart et al. (2000) Arth. Rheum. 43:1719-28).

Additionally, the increased expression of IL-17F observed in BAL samples isolated from patients suffering from asthma (Kawaguchi et al. (2002) J. Immunol.167:4430-35) and colon samples isolated from patients suffering from inflammatory bowel diseases, e.g., ulcerative colitis or Crohn's disease (Gurney et al. (2003) GTCBIO Conference: Cytokines and Beyond) suggests an additional role for IL-17F signaling in inflammatory disorders of lung and bowel tissues. To investigate the role of IL-17F signaling in inflammatory responses, naïve mice were injected intraperitoneally with PBS (as a control) or 100 µg human IL-17F (SEQ ID NO:2). Hours after treatment, samples from peripheral blood (PB; 1, 2, 4, 6, 8, and 10 h) and peritoneal cavities (PEC; 2, 4, 6, 8, and 10 h) were taken, and the absolute neutrophil count (ANC) in each sample was determined. The data in **Table 2** reflects the ability of IL-17F to increase neutrophil counts in blood and peritoneum. This effect may explain the neutrophilia seen in patients suffering from rheumatoid arthritis and chronic obstructive pulmonary diseases (COPD).

**Table 2. Absolute neutrophil counts (ANC; mean ± SEM) in peripheral blood (PB) and peritoneal cavity (PEC) samples isolated from mice injected with PBS (n = 5) or IL-17F (n = 5). Asterisk denotes a p-value <0.05 compared to control samples.**

| Hours post injection | PB ANC x10³/uL | | PEC ANC x10⁵/mL | |
|---|---|---|---|---|
| | PBS | IL-17F | PBS | IL-17F |
| 1 | 1.24 ± 0.59 | 1.97 ± 0.45 | - | - |
| 2 | 0.15 ± 0.10 | 1.07 ± 0.25* | 0.08 ± 0.05 | 2.28 ± 2.52 |
| 4 | 0.15 ± 0.08 | 0.75 ± 0.13* | 0.00 ± 0.01 | 0.30 ± 0.15* |
| 6 | 0.25 ± 0.13 | 0.93 ± 0.4* | 0.04 ± 0.06 | 2.03 ± 1.35* |
| 8 | 0.22 ± 0.06 | 0.31 ± 0.08 | 0.02 ± 0.02 | 1.20 ± 2.11 |
| 10 | 0.11 ± 0.09 | 0.48 ± 0.36 | 0.02 ± 0.03 | 2.06 ± 1.53* |

### Example 1.2: IL-17F signaling plays a role in inflammatory joint disorders

To further assess the involvement of IL-17F signaling in inflammation, particularly inflammatory responses implicated in joint diseases (e.g., arthritis), the ability of IL-17F to activate a primary factor involved in the transcription of inflammatory cytokines, i.e., NF-κB, in primary chondrocytes was determined. Primary human or porcine chondrocytes were infected (100 MOI) with adenovirus expressing an NF-κB reporter gene system, which detects activation of endogenous NF-κB (i.e., translocation of NF-κB from the cytoplasm to the nucleus) by measuring the expression of a luciferase gene that is controlled by an NF-κB-responsive promoter (BD Mercury Pathway Profiling systems, BD Biosciences, Palo Alto, CA). After 48-72 hours, infected chondrocytes were cultured with varying concentrations of IL-17A (SEQ ID NO:4) or IL-17F. After four hours of incubation with IL-17A or IL-17F, cells were lysed in 25 µl lysis buffer (Promega, Madison, WI) for 20 min at RT, and activation of NF-κB was measured using an automated luminometer. The data show that IL-17F activated NF-κB in primary human chondrocytes **(****Figure 1A****)** and primary porcine chondrocytes **(****Figure 1B****)** in a dose-dependent manner. Finally, the amount of IL-17F required to activate NF-κB to levels above background was similar to the amount of IL-17A required to activate NF-κB to levels above background **(****Figure 1A****).**

The ability of IL-17F to activate the cytokine transcription factor, NF-κB, in primary chondrocytes suggests that the role IL-17F plays in inflammation of joint tissue, e.g., during the development of arthritis, involves the induction of inflammatory cytokines. To test the effect of IL-17F on inflammatory cytokine production by joint tissues, human fibroblast-like synoviocytes isolated from two patients diagnosed with rheumatoid arthritis (RA) were plated to semi-confluence in 24 well plates and cultured in the absence or presence of 150 ng/ml IL-17F. Supernatants were collected at 48 h and cytokine production assessed using a multiplex cytokine system (Pierce-Bio, Rockford, IL). As shown in **Figure 2****,** IL-17F induced the production of inflammatory cytokines such as IL-6 and EL-8, and chemokines, such as MCP-1 and GRO-α, by human fibroblast-like synoviocytes isolated from RA patients.

The ability of IL-17F to activate NF-κB in primary chondrocytes and induce production of inflammatory cytokines and chemokines, particularly chemokines involved in neutrophil recruitment, by human fibroblast-like synoviocytes from RA patients supports a role for IL-17F in mediating inflammatory responses by joint tissue. These data, taken together with data demonstrating increased IL-17F expression in the paws of mice suffering from collagen induced arthritis compared to control animals (data not shown) and the presence of neutrophils within degenerated articular cartilage and joint space (data not shown), suggests that IL-17F mediates inflammatory joint diseases (e.g., rheumatoid arthritis) by inducing cytokine and chemokine production, which subsequently recruits, to the site of inflammation, immune cells (e.g., neutrophils) that cause damage to surrounding tissues.

### Example 1.3: Primary mouse lung fibroblasts respond to IL-17F by upregulating production of inflammatory chemokines and cytokines

To test the role of IL-17F in the development of inflammatory respiratory diseases, murine lung fibroblast (MFL) cells were grown to semi-confluence on 24 well plates and treated with IL-17F (50 ng/ml). Supernatants were collected at 48 h, and cytokine production was assessed using a multiplex cytokine system (Pierce-Bio, Rockford, IL). **Figure 3** shows that IL-17F induced the MFL production of inflammatory cytokines, such as IL-6, and chemokines, such as JE (CCL2) and KC. These results suggest IL-17F contributes to inflammatory responses implicated in inflammatory disorders of the lung (e.g., COPD, asthma, allergy, cystic fibrosis) by inducing the production of inflammatory cytokines and leukocyte chemoattractants.

### Example 2: Characterization of IL-17F receptors Example 2.1: IL-17F binds to IL-17R and IL-17RC

As IL-17F shares the greatest homology with IL-17A within the IL-17 family, and as it has been suggested that IL-17A and IL-17F signal via the IL-17 receptor, the ability of IL-17F to bind to the receptor for IL-17A (i.e., IL-17R; SEQ ID NO:6) was determined. The ability of IL-17F to bind to IL-17RC, an IL-17 receptor whose ligand to date has not been identified, was also tested.

ELISA plates were incubated with 1.5 µg/ml human IL-17R-Ig (SEQ ID NO:34) or 1.5 µg/ml human IL-17RC-Ig (SEQ ID NO:35) overnight. Plates were washed with PBS/1%BSA and incubated with serial dilutions of biotin-conjugated IL-17A or biotin-conjugated IL-17F for 2 h at room temperature (RT). After washing, saturating concentrations of avidin-horseradish peroxidase (HRP) were added, and plates were incubated for an additional 1 h at RT. Unbound avidin-HRP was washed using PBS/1% BSA, and the ELISA was developed using TBM. Bound IL-17A or IL-17F was detected by measuring the absorbance at 405 nm.

**Figures 4A** and **4B** demonstrate binding of IL-17F to both IL-17R and IL-17RC, respectively, with IL-17F having a greater affinity for IL-17R compared to its affinity for IL-17RC (EC₅₀ value for IL-17R:IL-17F = 1.23 µg/ml; EC₅₀ value for IL-17RC:IL-17F =15 µg/ml). However, although IL-17F bound to IL-17R, its affinity for the receptor was lower than that of the affinity of IL-17A for the same receptor (EC₅₀ values for IL-17R: IL-17F =1.23 µg/ml, IL-17R:IL-17A = 0.35 µg/ml; **Figure 4A****).**

### Example 2.2: Anti-IL-17R antibody and IL-17RC-Ig fusion protein, but not IL-17R-Ig fusion protein, block IL-17F activity

To further characterize IL-17F receptor binding, human fibroblast cells (10⁴ cells/well) were stimulated with 0.5 ng/ml IL-17A or 20 ng/ml IL-17F in the presence of increasing concentrations of an IL-17R-Ig fusion protein, an IL-17RC-Ig fusion protein or an anti-IL-17R antibody. After 24 h, the GRO-α concentrations of collected supernatants were determined using a commercially available ELISA (R&D, Minneapolis, MN). Concentrations of GRO-α were determined based on a standard curve.

**Figure 5** demonstrates increased GRO-α production by human fibroblast cells when incubated with IL-17A **(A)** or IL-17F **(B),** and further corroborates the findings of Example 1.2 (demonstrating increased inflammatory cytokine production by human fibroblast-like synoviocytes cultured with IL-17F). **Figure 5A** additionally shows that all three receptor antagonists, i.e., IL-17R-Ig (h17R.Fc), IL-17RC-Ig (h17RH2.Fc) and anti-IL-17R antibody (ahIL17R), blocked the ability of IL-17A to induce GRO-α. These data suggest that IL-17A binds to and requires both IL-17R and IL-17RC receptors for IL-17A signaling. In contrast, only the anti-IL-17R antibody and the IL-17RC-Ig fusion protein, and not the IL-17R-Ig fusion protein, notably blocked IL-17F activity **(****Figure 5B****).** The data presented in **Figure 5B** suggest that IL-17F binds to IL-17R (see **Figure 4****),** but does not require this receptor for IL-17F-mediated signaling. Altogether these data suggest IL-17A and IL-17F may use different receptors to mediate their activity on human fibroblast cells.

### Example 3: Generation and characterization of anti-IL-17F antibodies

### Example 3.1: Generation of anti-IL-17F antibodies

A group of five mice (Jackson Labs, Maine) were injected with 2 µg of cDNA encoding human IL-17F. Purified plasmid cDNA was precipitated onto gold beads to a concentration of 1 µg cDNA/0.5 mg gold. The gold beads and precipitated cDNA were delivered, monthly in two nonoverlapping shots, intradermally in the abdomen of 11-week old female Balb/c mice using the Helios charged gene. These animals were immunized every four weeks and spleens removed at end of this period. Reimmunizations were performed using purified IL-17F protein in addition to IL-17F cDNA. Spleens were processed to obtain a lymphocyte suspension and the resulting suspension was fused with the myeloma cell line 653/P3 using 50% (w/v) polyethylene glycol 1500 by an established procedure (Oi and Herzenberg (1980) in Selected Methods in Cellular Immunology, Mishel and Schigi, eds. W. J. Freeman Co., San Francisco, CA, p. 351). The fused cells were plated in 96-well microtiter plates at a density of 2 x 10⁵ cells/well, and after 24 hr were subjected to HAT selection. Hybridoma cells secreting putative anti-IL-17F antibodies were identified by solid and solution phase ELISA. Wells containing hybridoma positive for the above assays were expanded, cloned by limiting dilution and cryopreserved. Isotypes of antibodies were determined using solid phase ELISA. Purified human IL-17F-Ig was used to coat 96-well microtiter plates and detected by different isotype-specific biotin-conjugated goat anti-mouse IgG (Zymed, South San Francisco, CA). Streptavidin conjugated with horseradish peroxidase (HRP) was added and specifically bound enzyme measured using a colorimetric substrate.

### Example 3.2: Some anti-IL-17F antibodies inhibit IL-17F binding to IL-17R

To assess the ability of the anti-IL-17F antibodies to block binding of IL-17F to IL-17R, inhibition assays were performed by modifying the ELISA described in Example 2.1. Briefly, serial dilutions of anti-IL-17F antibodies were preincubated with 7 µg/ml IL-17F for 1 h at RT. Each cytokine:antibody mixture was then added to separate wells of an ELISA plate previously coated with 100 µl/well of 1.5 µg/ml IL-17R-Ig. The mixture was incubated in the wells for 1 h at RT. After washing the plate with PBS/1% BSA, saturating concentrations of avidin-HRP were added, and the plate was incubated for an additional 1 h at RT. Unbound avidin-HRP was washed away using PBS/1% BSA. The assay was developed using TMB. **Figure 6** demonstrates that five out of six antibodies tested, i.e., anti-IL-17F-01, anti-IL-17F-02, anti-IL-17F-06, anti-IL-17F-07, and (albeit to a lesser degree) anti-IL-17F-05 were able to block binding ofIL-17F to IL-17R. In contrast, anti-IL-17F-03 antibody did not inhibit IL-17F binding to IL-17R **(****Figure 6****).**

### Example 3.3: Some anti-IL-17F antibodies inhibit IL-17F binding to IL-17RC

To assess the ability of anti-IL-17F antibodies to block binding of IL-17F to IL-17RC, inhibition assays using the modified ELISA, as described above, were performed using plates previously coated with 1.5 µg/ml IL-17RC-Ig and IL-17F at a concentration of 20 µg/ml. **Figure 7** demonstrates that two out of 6 antibodies tested (anti-IL-17F-01 and anti-IL-17F-07) were able to inhibit binding of IL-17F to IL-17RC. In contrast anti-IL-17F-02, anti-IL-17F-03, anti-IL-17F-05 and anti-IL-17F-06 antibodies did not inhibit IL-17F binding to IL-17RC **(****Figure 7****).** Taking **Figures 6** and **7** together, the data not only suggest that anti-IL-17F antibodies bind to distinct sites on IL-17F, but also that anti-IL-17F-02, and anti-IL-17F-06 antibodies bind to and/or inhibit binding to a site on IL-17F unique for the IL-17F:IL-17R interaction while anti-IL-17F-01 and anti-IL-17F-07 antibodies bind to and/or inhibit binding to a site on IL-17F shared between IL-17R and IL-17RC. Consequently, these six antibodies may be used to define distinct sites, i.e., epitopes, on IL-17F.

### Example 4: Anti-IL-17F antibodies inhibit IL-17F bioactivities

### Example 4.1: Anti-IL-17F antibodies inhibit IL-17F-mediated cytokine production

To determine whether any of the anti-IL-17F antibodies described in Example 3 inhibit IL-17F bioactivity, human fibroblast cells (10⁴ cells/well) were stimulated with human 20 ng/ml IL-17F in the presence of increasing concentrations of a control antibody (mIgG1) or antibodies to IL-17F, i.e., anti-IL-17F-01, anti-IL-17F-02, anti-IL-17F-03, anti-IL-17F-05, anti-IL-17F-06, or anti-IL-17F-07. After 24 h, supernatants were collected and GRO-α concentrations determined using commercially available ELISA. Concentrations of GRO-α produced were determined based on a standard curve.

**Figure 8** demonstrates that anti-IL-17F-01, anti-IL-17F-02, and anti-IL-17F-07 antibodies inhibited human IL-17F-mediated GRO-α production by human fibroblast cells. The results, presented in **Figures 6****,** **7****,** and **8****,** suggest a model whereby IL-17F signaling is initiated by IL-17F binding to IL-17R, which results in the subsequent recruitment and required signaling through IL-17RC.

### Example 4.2: Potential uses of anti-IL-17F antibodies in the treatment of inflammatory disorders

To determine the potential of anti-IL-17F antibodies, in particular anti-IL-17F-07 antibody, as a therapeutic in the treatment of disorders related to IL-17F signaling (e.g., autoimmune diseases, respiratory diseases, inflammatory bowel diseases, etc.), porcine primary chondrocytes infected with a NF-κB reporter vector were incubated for 48 h with 100 ng/ml IL-17A or 500 ng/ml IL-17F preincubated for 1 h at RT in the absence or presence of one of the following: 20 µg/ml IL-17R-Ig fusion protein (IL17R/Fc), 10 µg/ml anti-IL-17F-07 antibody (antiIL17F), or 10 µg/ml control antibody (mouseIgG). **Figure 9** demonstrates that while incubation of the IL-17R-Ig fusion protein inhibited IL-17A-mediated activation of NF-κB, it had no effect on IL-17F-mediated activation of NF-κB. In contrast, anti-IL-17F-07 antibody was able to inhibit IL-17F-mediated activation of NF-κB, but had no effect on IL-17A-mediated activation of NF-κB **(****Figure 9****).**

To assess whether inhibited NF-κB activation in the presence of anti-IL-17F antibodies correlated with inhibited cytokine production, human fibroblast-like synoviocytes incubated with IL-17F, as described in Example 1.2, were incubated with an isotype control antibody, anti-IL-17F-01 antibody, or anti-IL-17F-07 antibody. Concentrations of IL-6, IL-8, or GRO-α were assessed as described in Example 1.2. **Figure 10** demonstrates that the ability of anti-IL-17F antibodies to inhibit IL-17F activation of NF-κB correlated with a decreased production of IL-6, IL-8, and GRO-α by primary fibroblast-like synoviocytes isolated from two patients with rheumatoid arthritis. These data suggest that antagonists ofIL-17F signaling, including, but not limited to, inhibitory antibodies directed toward IL-17F, may be used to reduce inflammatory responses. In particular, inhibitors of IL-17F may be used in the treatment of inflammatory responses associated with disorders related to IL-17F signaling, i.e., IL-17F-associated disorders.

### Example 5: Antibodies directed toward IL-17F and IL-17A may be used to detect and purify recombinant and natural IL-17F homodimers, IL-17A homodimers, and IL-17A/IL-17F heterodimers

### Example 5.1: Detection of recombinant IL-17A/IL-17A, IL-17F/IL-17F and IL-17A/IL-17F by ELISA

cDNAs encoding for human IL-17A, human IL-17F or both human IL-17A and human IL-17F were used to modify 293 cells. Expression of these cDNAs resulted in the production of IL-17A/IL-17A, IL-17F/IL-17F or IL-17A/IL-17F dimers. The conditioned media derived from these cells, i.e., the recombinant cytokines, and either commercially available antibodies or antibodies as described above, were used to develop ELISA formats for the detection of IL-17A protein, IL-17F protein, or IL-17A/IL-17F heterodimers. For the detection of IL-17A protein, i.e., as an IL-17A homodimer or an IL-17A/IL-17F heterodimer, anti-IL-17A antibody was used as a capture antibody and biotin labeled anti-IL-17A antibody was used as the detection antibody (both antibodies are available from R&D Systems, Minneapolis, MN). For the detection of IL-17F protein, i.e., as an IL-17F homodimer or an IL-17A/IL-17F heterodimer, anti-IL-17F-01 antibody (as described above) and biotin-labeled anti-IL-17F-07 antibody (as described above) were used as capture and detection antibodies, respectively. For the detection of IL-17A/IL-17F heterodimers, an anti-IL-17A antibody (R&D Systems) was used as a capture antibody and biotin labeled anti-IL-17F-07 antibody was used as a detection antibody. The IL-17A and IL-17F antibodies are not cross-reactive (data not shown).

ELISAs were performed according to a well-known protocol. Briefly, ELISA plates were incubated with 2 µg/ml of capture antibody overnight. The plates were washed with PBS/1%BSA to remove excess capture antibody and incubated with serial dilutions of the conditioned media, (i.e., recombinant IL-17A/IL-17A, IL-17F/IL-17F, IL-17A/IL-17F cytokines) for 2 h at RT. After washing unbound cytokine, 0.07-0.5 µg/ml biotin-conjugated developing antibody was added and plates were incubated for 2 h at RT. Plates were washed to remove unbound developing antibody, saturating concentrations of avidin-horseradish peroxidase (HRP) were added, and plates were incubated for 1 h at RT. Unbound avidin-HRP was washed away using PBS/1% BSA. The assay was developed using TBM.

**Figure 11** demonstrates the detection of recombinant IL-17A and IL-17F homodimers, as well as the detection of recombinant IL-17A/IL-17F heterodimers. When capture and detection antibodies are both directed toward one cytokine, (i.e., IL-17A or IL-17F), both homodimers and heterodimers of that cytokine were detected **(****Figures 11A** and **11B****).** In contrast, when an anti-IL-17A antibody and an anti-IL-17F antibody are used as capture and detection antibodies, respectively, only IL-17A/IL-17F heterodimers are detected **(****Figure 11C****).** These data suggest that the IL-17A/IL-17F antibody pair may be used to detect and potentially purify natural (i.e., nonrecombinant) IL-17A/IL-17F heterodimers in conditioned media derived from primary cells. Additionally, these results suggest that anti-IL-17F-07 antibodies, and likely anti-IL-17F-01 antibodies and other anti-Il-17F antibodies, as described herein, may also be directed against IL-17A/IL-17F heterodimers.

### Example 5.2: Detection of natural IL-17A homodimers, natural IL-17F homodimers, and natural IL-17A/IL-17F heterodimers

Human CD4⁺ T cells (5 x 10⁵ cells/ml) were activated with anti-CD3-coupled beads (5 µg/10⁷ beads), and increasing concentrations of anti-CD28 antibodies (R&D, Minneapolis, MN), and in the absence or presence of IL-21 (60 ng/ml) or IL-23 (0.5 ng/ml). Supernatants were collected 72 hours after primary activation and the concentration of IL-17A or IL-17F was determined by ELISA for IL-17A protein, or IL-17F protein, respectively, as described above (Example 5.1). **Figure 12** demonstrates that IL-21 or IL-23 maybe used to enhance IL-17A or IL-17F production by T cells undergoing primary activation. IL-2, IL-7, and IL-15 also induced IL-17A and IL-17F production upon CD3/CD28 stimulation (data not shown).

Human CD4⁺ T cells (5 x 10⁵ cells/ml) were activated with anti-CD3-coupled beads (5 µg/10⁷ beads) and soluble anti-CD28 antibodies for 48 hours. Activated T cells were harvested, rested overnight, and reactivated in the presence of bead-bound anti-CD3, anti-CD28 antibodies, IL-21 (60 ng/ml) or IL-23 (0.5 ng/ml). Supernatants were collected 72 hours after secondary activation and production IL-17A or IL-17F was determined by ELISA for IL-17A protein, or IL-17F protein, respectively, as described above. **Figure 13** demonstrates that IL-21 or IL-23 synergizes with CD28 costimulation to enhance IL-17A or IL-17F production by T cells undergoing secondary activation.

Human CD4⁺ T cells (2 x 10⁶ cells/ml) were subjected to primary activation with anti-CD3-coupled beads (5 µg/10⁷ beads) and soluble anti-CD28 antibody (0.5 µg/ml). After 48 h of primary activation, conditioned media (CM1) was collected and cells rested overnight. The next day, cells were counted and restimulated at 2 x 10⁶ cells/ml as described for the primary activation above and in the presence of 60 ng/ml IL-21. After 72 h ofrestimulation, conditioned media (CM2) was collected. The presence or absence of IL-17A homodimers, IL-17F homodimers, and IL-17A/IL-17F heterodimers in neat and 1:10 diluted CM1 and CM2 was assessed using the ELISA formats described in Example 5.1.

The data indicate little to no detection of IL-17A homodimers or IL-17A/IL-17F heterodimers in CM1 media, i.e., media of T cells that underwent primary activation **(****Figures 14A** and **14C****).** In contrast, conditioned media of restimulated T cells (CM2) comprised not only IL-17A and IL-17F homodimers but also IL-17A/IL-17F heterodimers **(****Figures 14A, 14B****,** and **14C****).** These data indicate that T cells, the "natural" source of IL-17 cytokines, express both homodimers and heterodimers of IL-17A and IL-17F. These results also indicate that antibodies directed against IL-17F can recognize and react with both IL-17F homodimers and IL-17A/IL-17F heterodimers, and that such antibodies may be used to isolate and inhibit the biological activity of IL-17F homodimers and/or IL-17A/IL-17F heterodimers.

### Example 5.3: Immunoprecipitation of natural IL-17A homodimers, IL-17F homodimers, and IL-17A/IIL-17F heterodimers from T cells

Conditioned media (CM2) derived from T cells undergoing secondary activation in the presence of IL-21, as described in Example 5.2, was mixed with 20 µg/ml murine anti-human IL-17A-02 (Wyeth) or murine anti-human IL-17F-01 (Wyeth) monoclonal antibodies for 1 h at 4°C under gentle rotation. Antibody complexes from each mixture were separately immunoprecipitated with 50 µl hydrated protein A-sepharose overnight at 4°C under gentle rotation. The immunoprecipitated pellets were then sequentially washed with PBS/1% Tween 20, PBS/0.1% Tween 20, and PBS/0.05% Tween 20. The immunoprecipitated pellets were resuspended in nonreducing sample buffer and loaded onto a 10% Tricine gel for Western blot analysis with either anti-human IL-17A biotin conjugation (R&D, Minneapolis, MN) or rabbit anti-human IL-17F antibodies (Wyeth).

IL-17F homodimers (35 kDa) were immunoprecipitated using murine anti-human IL-17F-01 antibody and detected via Western blot analysis with a rabbit anti-human IL-17F polyclonal antibody from 500 µl of CM2. The IL-17F/IL-17A heterodimers (32 kDa) were immunoprecipitated using murine anti-human IL-17A-02 antibody and detected via Western blot analysis with a rabbit anti-human IL-17F polyclonal antibody from 500 µl of CM2 **(****Figure 15****).** Neither the monoclonal nor the polyclonal antibody cross-reacted with IL-17A (data not shown). Similarly, IL-17A homodimers (31 kDa) and IL-17F/A heterodimers were immunoprecipitated using murine anti-human IL-17A-02 antibody and detected via Western blot analysis using a polyclonal goat anti-IL-17A biotinylated antibody from 700 µl of CM2. The IL-17F/IL-17A heterodimers were immunoprecipitated using murine anti-human IL-17F-01 antibody and detected via Western blot analysis using a polyclonal goat anti-IL-17A biotinylated antibody from 700 µl of CM2 **(****Figure 16****).** The polyclonal antibody cross-reacts with IL-17F homodimer at high protein concentrations (data not shown).

As controls (lane 2 of **Figures 15** and **16****),** IL-17F homodimers were , purified from concentrated conditioned media overexpressing His tagged human IL-17F. Briefly, concentrated conditioned media was diluted 1:1 with 100 mM Tris pH 8/1M NaCl/10 mM imidazole and loaded onto a Nickel-NTA Fast Flow column (Qiagen, Valencia, CA). The homodimer was step eluted with 250 mM imidazole buffer. The protein was dialyzed against PBS-NSO. The homodimer was then digested with EK (enterokinase) for 4 hours at room temp to remove the His₆ tag. The digested protein was diluted 1:1 with 50 mM sodium phosphate pH 8/20 mM imidazole/300 mM NaCl and bound to a Nickel-NTA column (Qiagen, Valencia, CA). The protein minus the tag was eluted with 40 mM imidazole buffer, and was dialyzed against PBS NSO. Purified IL-17A homodimers for use as controls (lane 5 of **Figure 15****)** were purchased from R&D Systems (Minneapolis, MN).

### Example 5.4: Immunoprecipitation of recombinant IL-17A homodimers, IL-17F homodimers, and IL-17F/IL-17A heterodimers from transfected COS cells

Experiments were conducted to determine whether recombinant human IL-17F and IL-17A would form heterodimers upon expression in COS cells, and whether anti-IL-17F and anti-IL-17A antibodies were capable of immunoprecipitating and detecting IL-17F/IL-17A heterodimers. COS cell cultures were transfected with IL-17F cDNA, IL-17A cDNA, or both IL-17F and IL-17A cDNA, and the transfected cell cultures were allowed to secrete the resultant protein(s) into the media. Conditioned media derived from either expression of IL-17F or IL-17A or the coexpression of IL-17F and IL-17A was mixed with 20 µg/ml murine anti-human IL-17A-02 (Wyeth) or murine anti-human IL-17F-01 (Wyeth) monoclonal antibodies for 1 hour at 4°C under gentle rotation. Antibody complexes from each mixture were separately immunoprecipitated with 50 µl-hydrated protein A-sepharose overnight at 4°C under gentle rotation. The immunoprecipitated pellets were then sequentially washed with PBS/1% Tween 20, PBS/0.1% Tween 20, and PBS/0.05% Tween 20. The immunoprecipitated pellets were resuspended in nonreducing sample buffer and loaded onto a 10% Tricine gel for Western blot analysis with either goat anti-human IL-17A (R&D, Minneapolis, MN) or rabbit anti-human IL-17F antibodies (Wyeth).

IL-17F homodimers (35 kDa) and IL-17F/IL-17A heterodimers were immunoprecipitated using murine anti-human IL-17F-01 antibody and detected via Western blot analysis with a rabbit anti-human IL-17F polyclonal antibody from 50 µl of CM2 **(****Figure 17A****;** lanes 3 and 4, respectively). The IL-17F/IL-17A heterodimers (32 kDa) were also immunoprecipitated using murine anti-human IL-17A-02 antibody and detected via Western blot analysis with a rabbit anti-human IL-17F polyclonal antibody from 50 µl of CM2 **(****Figure 17A****;** lane 9). The IL-17A antibody used for immunoprecipitation in lanes 8-10 of Figure 17A appeared to cross-react with IL-17F at high protein concentrations, since a band corresponding to IL-17F homodimer is detected by the anti-IL-17F antibody probe in lane 10. As shown in **Figure 17B****,** the IL-17A homodimers (31 kDa) and IL-17F/IL-17A heterodimers were immunoprecipitated using murine anti-human IL-17A-02 antibody and detected via Western blot analysis using a polyclonal goat anti-human IL-17A antibody from 50 µl of CM2 **(****Figure 17B****;** lanes 3 and 4, respectively). Also, the IL-17F/IL-17A heterodimers were immunoprecipitated using murine anti-human IL-17F-01 antibody and detected via Western blot analysis using a polyclonal goat anti-human IL-17A antibody from 50 µl of CM2 **(****Figure 17B****;** lane 6). As opposed to the IL-17A antibody used for immunoprecipitation in **Figure 17A****,** the IL-17F antibody used for immunoprecipitation in lanes 5-7 of **Figure 17B** did not significantly cross-react with IL-17A, since almost no band corresponding to IL-17A homodimer is detected by the anti-IL-17A antibody probe in lane 5.

As controls (lanes 6-7 of **Figure 17A****),** IL-17F homodimers were purified as described in Example 5.3. Purified IL-17A homodimers for use as controls (lane 5 of **Figure 17A****)** were purchased from R&D Systems (Minneapolis, MN). Control purified IL-17F homodimers migrate slightly faster than IL-17F homodimers immunoprecipitated from conditioned media. This is likely due to differences in glycosylation and/or the lack of an epitope tag on the IL-17F proteins in the purified samples.

### Example 5.5: Purification of recombinant IL-17A/IL-17F heterodimers

Two methods of purifying IL-17A/IL-17F heterodimers are provided herein. In the first method, COS cells were cotransfected with His₆-tagged IL-17F (SEQ ID NO:36) and untagged IL-17A. Sodium chloride and imidazole were added to the conditioned media to final concentrations of 500 mM and 6 mM, respectively. The conditioned media was then loaded onto a Nickel NTA column (Qiagen, Valencia, CA). Thus, only IL-17F homodimer and IL-17F/IL-17A heterodimer, which contain a His tag, were captured on the nickel column. The IL-17F homodimer and IL-17F/IL-17A heterodimer were then separated with an imidazole gradient, and the IL-17F/IL-17A heterodimer was then digested with EK to remove the His₆ tag. The protein was dialyzed against PBS. Edman sequencing was done to verify that the IL-17F and IL-17A protein was detected in the IL-17F/A heterodimer sample. N-terminal sequence results confirmed the existence of heterodimers, i.e., the first five amino acids for IL-17F were shown to be RKIPK (SEQ ID NO:37), and for IL-17A were shown to be IVKAG (SEQ ID NO:38).

The second method used a dual column purification scheme, which is shown in the flow diagram set forth in **Figure 18****.** Flag-tagged human IL-17A (SEQ ID NO:39) and a His₆-tagged human IL-17F (SEQ ID NO:36) were cloned into separate pSMED2 vectors (Wyeth) and coexpressed in HEK293 cells by lipofection. Briefly, cells were seeded in two 175-cm² flasks 24 h prior to transfection. For each flask, 24 µg pSMED2/Flag-IL-17A + 24 µg pSMED2/His6-IL-17F was mixed with 120 µl TRANSIT® reagent-LT1 (Mirus, Madison, WI) in 2 ml serum-free media, and added to a flask containing cells (90% confluent) and 25 ml DMEM media containing 10% heat-inactivated fetal bovine serum. One day post-transfection, media was removed, the cells rinsed 1x with serum-free media, and fresh serum-free media was added (40 ml/flask). Conditioned media was harvested 48 h later, filtered (0.45 µ) to remove cells, and frozen at -20°C. Protein expression was confirmed by Western analysis using specific antibodies.

The conditioned media was batch-bound to anti-Flag M2 affinity resin (Sigma, St. Louis, MO) at 4°C for 2 hours. The bound proteins, IL-17A homodimer and IL-17F/IL-17A heterodimer, were eluted using 50 mM Tris pH 8/500 mM NaCl/200 µg/ml Flag peptide (Sigma, St. Louis, MO). The flag elution was then batch-bound to Nickel-NTA resin (Qiagen, Valencia, CA) overnight at 4°C. The bound protein, IL-17F/IL-17A heterodimer, was eluted using 50 mM Tris pH 8/1 M NaCl/500 mM imidazole.

Following purification of recombinant IL-17F/IL-17A heterodimers substantially free from IL-17F and IL-17A homodimers, the activity of the heterodimers was tested on BJ cells by measuring the ability of the heterodimer to stimulate GRO-α levels in the BJ cell culture media. Thus, BJ cultures were stimulated with various concentrations of IL-17F homodimers, IL-17A homodimers, or the purified recombinant IL-17F/IL-17A heterodimers. After 24 h, supernatants from the BJ cultures were collected and GRO-α concentrations determined using commercially available ELISA. Briefly, BJ cells were seeded at 5 x 10³ cells/well in flat-bottom 96-well plates and supplied with 15 µl of media containing IL-17A homodimers, IL-17F homodimers, or IL-17F/IL-17A heterodimers. Plates were then incubated for 16-24 hours at 37°C, after which the supernatants were removed and the concentration of GRO-α determined using standard sandwich ELISA with antibodies to GRO-α (R & D Systems, Minneapolis, MN). Concentrations of GRO-α produced were determined based on a standard curve. The results are shown in **Figure 19A****.** Similar to both IL-17F and IL-17A homodimers, the IL-17A/IL-17F heterodimer is capable of stimulating IL-17 GRO-α concentrations in BJ cells. Moreover, as shown in **Figure 19B****,** treatment of BJ cultures with anti-IL-17A antibodies, or anti-IL-17A in combination with anti-IL-17F antibodies abrogated the ability of IL-17F/IL-17A heterodimers to stimulate GRO-a levels.

### Example 5.6: Mass spectrometry analysis of IL-17A homodimers, IL-17F homodimers, and IL-17A/IL-17F heterodimers

To provide direct evidence of a disulfide linkage between two IL-17 monomers, the presence of disulfide linkages was verified and intermolecular disulfide-linked peptides (IL-17A homodimers, IL-17F homodimers, and IL-17A/IL-17F heterodimers) were identified by tandem mass spectrometric analysis. Tryptic cleavage and reverse phase high performance liquid chromatography (rpHPLC) were used to isolate disulfide-linked peptides, which were then analyzed by nanoLC-MS/MS. Briefly, 1-2 µg of purified, nonreduced recombinant IL-17A homodimer, IL-17F homodimer and IL-17A/IL-17F. heterodimer were run on 10% Bis-Tris gels (Invitrogen, Carlsbad, CA), and stained with the IMPERIAL^{™} Protein Stain Solution (Pierce, Rockford, IL). Positively stained bands were excised and manually trypsin digested (Promega, Madison, WI) for mass spectrometric analysis. For the digestion, the excised gel slices were dehydrated in acetonitrile (ACN), rehydrated and washed in 25 mM sodium phosphate buffer (pH 6.0), and dehydrated again in ACN. The protease trypsin (0.5 µg of trypsin dissolved in 25 mM sodium phosphate buffer) was added, driven into the gel pieces by rehydration, and incubated for 4 h at 37°C.
The resulting peptides were further extracted from the gel with three successive washes using aliquots of 60% ACN/1% formic acid (FA) and 90% ACN/5% FA. These extracts were combined and evaporated, and the final sample reconstituted in 2% ACN and 0.1 % FA.

The digested samples were pressure-loaded onto a C18 PICOFRIT^{®} microcapillary column (New Objective, Woburn, MA) packed with Magic C18 beads (5 µm, 75 µm x 11 cm, Michrom BioResources, Auburn, CA). The column was then coupled to a linear ion trap mass spectrometer (LTQ, ThermoFinnigan, San Jose, CA). The HPLC gradient increased linearly from 4-60% ACN using Solvent B as a modifier (Solvent A, 2% ACN and 0.1% FA; Solvent B, 90% ACN and 0.1 % FA) over 70 min with a flow rate of 250 nl/min. Mass spectra were collected using LTQ at tandem mass spectrometry mode (referred to as MS/MS), in which each MS acquisition was followed by six MS/MS acquisitions of the first six most intense peptide ions in the prior MS spectrum. In some cases, MS/MS acquisitions were followed by two MS/MS/MS acquisitions of the first two most intense peptide ions of the prior MS/MS acquisitions. The peptide masses were recorded by scanning an m/z (mass-to-charge ratio) range from 375 to 1500. The dynamic exclusion in the acquisition software provided by the manufacturer was also employed to increase the number of peptide ions of interest to be analyzed. The MS/MS data were manually interpreted.

At the MS level, the observed m/z of the peptide fragments ([M+3H]³⁺=919.7 for IL-17A homodimer; [M+3H]³⁺=1196.9 for IL-17F homodimer; [M+3H]³⁺=1138.1 or 807.9 for IL-17F/IL-17A heterodimer) matched the calculated candidate disulfide-linked peptides. Further, the peptide sequence according to the MS/MS data of these targeted masses was obtained, and it was confirmed that they were the expected cysteine(C)-containing peptides derived from IL-17A and/or IL-17F for the disulfide bond formation as shown in **Figure 20****.** In addition, MS³ data was acquired for the IL-17A homodimer peptide ([M+3H]³⁺=907.7 and [M+3H]³⁺=843.3) due to the poor fragmentation at the MS/MS level. This demonstrates that IL-17A homodimers, IL-17F homodimers, and IL-17F/IL-17A heterodimers exist as dimers via disulfide bond linkage. In conclusion, the disulfide linkage patterns of the two homodimers and the heterodimer were resolved, which were consistent to be C1 and C4 between each monomer. Similar approaches were used in this study to demonstrate the involvement of other cysteines (C2/C3 and C5/C6) for the intra-molecular disulfide bond formation.

### Example 6: Antibodies against human IL-17F inhibit primate IL-17F bioactivity

To determine whether the anti-IL-17F antibodies were capable of cross-reacting with primate IL-17F, various concentrations of macaque IL-17F conditioned media or purified human IL-17F were used to stimulate BJ cells in the presence or absence of 100 µg/ml anti-IL-17F-01 or anti-IL-17F-07 antibodies. Macaque IL-17F conditioned media for use in this experiment was produced by expressing macaque IL-17F cDNA (SEQ ID NO:40) subcloned into pCRII in HEK293 cells, and harvesting the conditioned media containing the macaque IL-17F homodimers. After 16-24 hours of treatment with either human or macaque IL-17F, the GRO-α concentrations of supernatants collected from the treated cultures was determined using a commercially available ELISA (R&D, Minneapolis, MN) as described in Example 5.5. Concentrations of GRO-α were determined based on a standard curve.

**Figure 21** demonstrates increased GRO-α production by BJ cells incubated with macaque IL-17F conditioned media or human IL-17F protein. As described previously (see **Figure 2** and **Figure 8****),** human IL-17F stimulates GRO-α levels, and this stimulation is inhibited by both anti-IL-17F-01 and anti-IL-17F-07 antibodies **(****Figure 21A****).** As shown in **Figure 21B****,** macaque IL-17F also stimulates production of GRO-α in BJ cells. Both anti-IL-17F-01 and anti-IL-17F-07 antibodies are capable of inhibiting the ability of macaque IL-17F conditioned media to stimulate GRO-α cytokine levels. Thus, antagonist antibodies to human IL-17F can cross-react with primate IL-17F to inhibit primate IL-17F bioactivity.

### Example 7: IL-17F stimulation of Aggrecanase 1 levels in human chondrocytes is reduced by cotreatment with IL-17F antibodies

To determine whether IL-17F may be involved in the inflammatory response accompanying, e.g., rheumatoid arthritis, nonarthritic human cartilage was obtained from National Disease Research Interchange (NDRI, Philadelphia, PA), and chondrocytes were isolated by serial enzymatic digestion of pronase (1 mg/ml, 37°C for 30 min) and collagenase P (1 mg/ml, 37°C overnight) within 48 hour postmortem. Upon isolation, cells were resuspended in Dulbecco's modified Eagle's medium (DMEM)/F-12 media with 2 µM L-glutamine, and 100 U/ml penicillin/100 µg/ml streptomycin containing 10% fetal bovine serum (FBS). Cells were plated in 24-well plates at 1 x 10⁶ cells/well. Media was changed to serum-free media after 72 h and the chondrocytes were stimulated with human IL-17F in the presence or absence of anti-IL-17F-07 or its isotype control (IgG) for 6 h at 37°C. Cells were harvested immediately in RLT buffer (Qiagen, Valencia, CA, RNEASY^{®} Kit) with β-mercaptoethanol and stored at -80°C until ready for the RNA isolation. RNA was prepared using RNEASY^{®} Mini Kit, and DNase treatment was performed on the RNEASY^{®} column for 15 min at room temperature as per the manufacturer's protocol. ADAMTS-4 (aggrecanase-1) mRNA expression levels were monitored by TAQMAN^{®} PCR analysis (Applied Biosystems, Foster City, CA). Briefly, 100 ng of isolated RNA was used for QPCR reaction with ADAMTS-4 probe/primers obtained from Applied Biosystems. The expression of ADAMTS-4 was normalized to the expression of GAPDH (10 ng of isolated RNA was used with GAPDH primers obtained from Applied Biosystems) for each sample. A standard curve for sample extrapolation was prepared using 0.16 ng to 100 ng of Universal Reference Total RNA (Clontech, Palo Alto, CA) that consists of pools of different tissues.

The results, presented as TAQMAN^{®} units, are shown in **Figure 22****.** Thus, IL-17F treatment increases the expression of Aggrecanase 1 in human chondrocytes, and treatment with anti-IL-17F-07 antibodies abrogates this stimulation. These data suggest that IL-17F involvement in inflammation and joint degradation, such as occurs during autoimmune arthritis, e.g., reactive and rheumatoid arthritis, may be mitigated by treatment with anti-IL-17F antibodies.

### Example 8: Regulation of IL-17F and IL-17A bioactivity by siRNA knockdown of receptors IL-17R and IL-17RC in BJ cells

Experiments were designed to determine whether a reduction in the level of IL-17R and/or IL-17RC transcripts would reduce the bioactivity of IL-17F and/or IL-17A. BJ cells were seeded 24 h prior to transfection in 96-well plates at 9x10³ cells/100 µl medium/well. Cells were transfected with chemically synthesized RNAi reagents (Dharmacon, Lafayette, CO) using DHARMA*FECT*^{®} 1 (Dharmacon, Lafayette, CO) at 0.3 µl/well, and individual or pooled siRNAs at 10 nM or lower (see SEQ ID NOs:17-32). Following siRNA transfection, the cells were incubated with transfection complexes for 18 h. The transfection medium was then replaced by regular culture medium and incubated for an additional 6 h. The regular culture medium was then replaced with 150 µl of culture medium containing IL-17A at 1 ng/ml or IL-17F at 50 ng/ml. Following 16 h of incubation with the designated cytokine, the culture supernatants were collected for analysis by standard sandwich ELISA of the ability of IL-17F and/or IL-17A to induce levels of IL-6, IL-8, and GRO-α (see **Figure 2****).** Matched antibody pairs for hGRO-α, hIL-6 and hIL-8 were purchased from R&D Systems (Minneapolis, MN).

To measure decreases in IL-17R and IL-17RC receptor levels following siRNA treatment, the TURBOCAPTURE^{®} mRNA kit (Qiagen, Valencia, CA) was used to isolate mRNA from BJ fibroblast cells according to manufacturer's instructions. A one-step RT qPCR MASTERMIX PLUS^{®} (Eurogentec, San Diego, CA) TAQMAN^{®} (Applied Biosystems) protocol was used whereby 10 µl of mRNA per sample was used in 25 µl TAQMAN^{®} PCR reactions performed on an ABI PRISM^{®} 7700 DNA Sequence Detector (Applied Biosystems). The conditions for TAQMAN^{®} PCR were as follows: 30 min at 48°C, 10 min at 95°C, then 40 cycles each of 15 s at 95°C and 1 min at 60°C on MICROAMP OPTICAL^{®} (Applied Biosystems) 96-well plates, covered with MICROAMP OPTICAL^{®} caps. Each plate contained triplicates of the test cDNA templates or no-template controls for each reaction mix. The expression for each mouse gene was normalized to human β2 microglobulin gene expression. The TAQMAN^{®} gene expression assay probe-primer sets for IL-17R and IL-17RC were acquired from Applied Biosystems.

The results presented in **Figure 23** depict the percent reduction in GRO-α levels (normalized to β2-microglobulin) following siRNA treatment of BJ cultures. siRNA treatment of BJ cultures decreased IL-17R and IL-17RC transcript levels by about 80% **(****Figure 23A** and **Figure 23B** **-** "Taqman"). While decreases in both IL-17R and IL-17RC levels reduced the ability of IL-17A and IL-17F to stimulate GRO-α levels **(****Figure 23A** and **Figure 23B****),** reduction in IL-17RC levels **(****Figure 23B****)** had a more pronounced effect than reduction in IL-17R levels **(****Figure 23A****)** on both IL-17A and IL-17F bioactivity. Interestingly, the reduction in IL-17RC had a greater effect on the ability of IL-17A to stimulate GRO-α levels **(****Figure 23B****).** Preferred examples of siRNAs that target IL-17R and IL-17RC are disclosed in **Figure 23C** (see also SEQ ID NOs: 17-32). These data suggest that both IL-17A and IL-17F can signal through IL-17R and IL-17RC, and that IL-17RC may be a preferred receptor for both molecules in relation to GRO-α stimulation.

### Example 9: IL-17A and IL-17F are upregulated in afflicted tissues from human patients with psoriasis, Crohn's disease, and ulcerative colitis

Psoriatic tissue biopsy samples were collected from patients enrolled in Wyeth-sponsored Clinical Study #3067K6-207 (A Randomized, Double-blind, Placebo-controlled, Exploratory Pharmacogenomic Study of Recombinant Human Interleukin Eleven, rhIL-11, in Patients with Active Psoriasis). Baseline (visit 2) psoriatic lesional and nonlesional skin biopsies from 48 patients were flash frozen in liquid nitrogen immediately after collection and shipped to the Wyeth Clinical Pharmacogenomics Laboratory in Andover, MA.

Crohn's disease samples were collected from patients enrolled in Wyeth-sponsored Clinical Study #3067K6-204 (A Multicenter, Randomized, Double-blind, Placebo-controlled, Safety and Exploratory Pharmacogenomic Study of Orally Administered Recombinant Human Interleukin Eleven (RhIL-11) for the Treatment of Patients with Active Crohn's Disease), and ulcerative colitis samples were collected from patients enrolled in Clinical Study #3067K5-114 (A Multicenter, Randomized, Double-blind, Placebo-controlled, Dose-escalating, Safety and Exploratory Pharmacogenomic Study of Orally Administered Recombinant Human Interleukin Eleven (RhIL-11) in Patients with Mild to Moderate Left-sided Ulcerative Colitis). Baseline (visit 2) paired involved and noninvolved tissue biopsies from #3067K6-204 patients (16 patients), and baseline (visit 1) paired tissue biopsies from the same anatomical area of sigmoid and left colon from #3067K5-114 patients (12 patients) were flash frozen in liquid nitrogen immediately after collection and shipped to the Wyeth Clinical Pharmacogenomics Laboratory in Andover, MA.

Tissue was homogenized using a polytron, RNA was isolated from the supernatant of the lysate using RNEASY® Mini Kit (Qiagen, Valencia, CA), and treated with DNase (Qiagen RNase-free DNase Kit). The DNase-treated RNA preparation was further purified using a Phase Lock Gel column (Brinkman, Westbury NY), phenol:chloroform:IAA (isoamyl alcohol) extracted (Ambion, Austin, TX), and concentrated using an RNEASY^{®} mini column. SPECTRAMAX^{®} (Molecular Devices, Sunnyvale, CA) was used to quantify RNA, and RNA quality was assessed by Agilent Bioanalyzer gel (Model 2100; Agilent Technologies, Palo Alto, CA). Conversion of 2 µg of total RNA from the above preparations to cDNA was accomplished using the Applied Biosystems High Capacity cDNA Archive Kit (Applied Biosystems) by following the manufacturer's instructions. Plates containing completed reactions were stored at temperatures of-20°C (short term) or-80°C (long term).

Applied Biosystem's Assays-on-Demand (AOD) gene-specific primer-probe pairs are prevalidated, QC tested and optimized for use on any ABI PRISM^{®} sequence detection system (all from Applied Biosystems). According to the manufacturer's AOD protocol, a master mix was prepared using TAQMAN^{®} Universal PCR Master Mix (Applied Biosystems) containing IL-17F or IL-17A primers, and aliquoted into a 96-well plate for a final volume of 50 µl/well. Duplicate wells for serially diluted standards and cDNA samples (50 ng/well) were assayed on an ABI PRISM^{®} 7700 Sequence detector (Sequence Detector Software v1.7) using universal thermal cycling conditions of 50°C for 2 min, 95°C for 10 min, 95°C for 15 s (40 cycles), and extension at 60°C for 1 min.

Relative quantification of RNA transcript levels of IL-17F and IL-17A was performed following the manufacturer's guidelines described for the ABI PRISM^{®} 7700 Sequence Detection System (Applied Biosystems). Specifically, standard curves were calculated for target standards and endogenous control, input values determined for target and endogenous controls using standard curves' slope and y-intercept, and target input values were normalized to endogenous control. Fold-change in IL-17F and IL-17A expression was calculated using the 50 ng standard as a calibrator, and relative concentration of sample was obtained by multiplying fold-change by calibrator, then averaging.

To utilize the standard curve method, a tissue was empirically determined to express the target gene using TAQMAN^{®} AOD (Applied Biosystems). Total RNA from over 10 candidate target-positive tissues was obtained from Wyeth (Cambridge/Andover) and outside vendors. Multiple 2 µg aliquots from a single RNA preparation were converted to cDNA (as described above), pooled, stored as aliquots at -80°C, and assayed for expression of target gene by TAQMAN^{®} (Applied Biosystems). Cycle threshold (Ct) values of≥35 were considered below the limits of detection. For standard curve development, the goal was to achieve a Ct value between 18 and 25 for 100 ng of cDNA; this allowed for appropriate standard curve dynamic range. Preparations of positive control tissue meeting these requirements were used to generate the standard curve for each assay. Standard curves consisted of two-fold serial dilutions of total cDNA from 100 ng/well to 1.5 ng/well. Standard curves were performed on each plate for every assay and were used for sample quantification and assay performance monitoring. Due to 96-well plate space constraints, standard curve dilution points of 25 ng and 3 ng were omitted when running samples. Inter-plate % CV was < 3% for psoriasis samples, and <5% for Crohn's disease and ulcerative colitis samples.

Genes that are expressed at similar levels in all samples (i.e., treated and untreated, lesional and nonlesional, etc.) were selected to serve as endogenous controls in the relative standard curve method. From a list of candidate endogenous controls, it was determined that the gene designated *ZNF592* (GenBank Accession No. NM_014630) produced acceptable standard curves and did not vary significantly in lesional and nonlesional tissues, and involved and noninvolved tissues (p<0.05 for psoriasis and p<0.09 for Crohn's disease and ulcerative colitis samples). All study samples were normalized to *ZNF592* levels in determining relative concentration values.

A paired Student's t-test (pairing lesional and nonlesional samples, or involved and noninvolved tissues, from each patient) was used to assess the significance of the association between IL-17F and IL-17A expression levels and lesional (psoriasis) or inflammatory phenotype (Crohn's disease or ulcerative colitis). Fold-changes for the psoriasis study were calculated by dividing lesional relative concentration values by nonlesional relative concentration values. Fold-changes for the Crohn's disease and ulcerative colitis (IBDs) studies were obtained by dividing involved relative concentration values by noninvolved relative concentration values. Summary fold-changes were calculated by averaging fold-changes from all patients for IL-17F of IL-17A levels.

The results of these studies are shown in Figure 24 and Figure 25. As shown in Figure 24, both IL-17F and IL-17A expression levels are significantly increased in lesional tissues from afflicted patients, suggesting that IL-17F and IL-17A are involved in psoriasis *in vivo.* As shown in Figure 25, both IL-17A and IL-17F are increased in the involved tissues from patients afflicted with Crohn's disease, as well as those from patients afflicted with ulcerative colitis. The considerable heterogeneity among patients with Crohn's disease and ulcerative colitis, coupled with the relatively small sample size, mitigated against identifying a statistically significant association of IL-17A and IL-17F with the involved phenotype. However, clustering tools showed that both IL-17A and IL-17F were well correlated with the involved phenotype in the Crohn's disease sample set (r=0.65) (data not shown). These data suggest that elevated levels of IL-17A and IL-17F in involved tissues may play a role in the inflammatory conditions associated with IBDs *in vivo.*

### Example 10: LN cells from ovalbumin immunized mice produce IL-17F

8-week-old C57BL/6 mice were immunized in the flanks with 100 µg ovalbumin protein emulsified in complete Freund's adjuvant. Seven days later, inguinal lymph nodes were harvested. Lymph nodes were dissociated and the cells were restimulated with 50 ng phorbol ester 12-tetradecanoylphorbol-13 acetate, 1 µg/ml ionomycin, and 1 µg/ml GOLGIPLUG^{™} for 12 hours. Cells were then harvested, stained for surface CD4 using anti-mouse CD4 PerCP Cy5.5 (Pharmagen, San Diego, CA). Cells were fixed and permeabilized with CYTOFIX/CYTOPERM^{™} (BD Biosciences, San. Diego, CA) after which cells were stained with 4 µg/ml rat IgGl ALEXA FLUOR^{®} 647 conjugate (Invitrogen, Carlsbad, CA) or with 4 µg/ml rat anti-IL-17F (clone 15-1) ALEXA FLUOR^{®} 647 (Invitrogen, Carlsbad, CA) for 30 min. Cells were then washed twice with PERM/WASH^{™} (BD Biosciences, San. Diego, CA), and analyzed using FACSCALIBUR^{™} (BD Biosciences, San. Diego, CA). Rat anti-IL-17F (clone 15-1) ALEXA FLUOR^{®} 647 (Invitrogen, Carlsbad, CA) was prepared using an ALEXA FLUOR^{®} 647 conjugation kit from Invitrogen. The results are shown in Figure 26. Thus, *in vivo* CD4+ T cells from the lymph nodes of ovalbumin-immunized mice produce IL-17F protein.

### Example 11: Conclusion and Discussion

Among several findings, these data indicate that IL-21 and IL-23 induced IL-17A and IL-17F upon TCR/CD28 costimulation, and that IL-23 and IL-21 synergize with costimulation for IL-17A and IL-17F production. Both IL-23 and IL-21 are equally effective in IL-17 induction. These data suggest that IL-17A, and particularly IL-17F (since it is produced at 10-20 fold higher levels compared to IL-17A) may mediate some of the proinflammatory effects attributed to IL-21, and that inhibition of IL-17F (either as an IL-17F homodimer or an IL-17F heterodimer) may have similar therapeutic effects as blocking IL-21 signaling (see, e.g., U.S. Patent Application Nos. 60/599,086 and 60/639,176). The similarities between the effects of IL-17F signaling and IL-21 signaling lead to a strong conclusion that inhibition of IL-17F signaling may be as therapeutically valuable as inhibiting IL-21 signaling. Additionally, the results show for the first time that T cells express IL-17A/IL-17F heterodimers, as well as IL-17A and IL-17F homodimers; the results also show that such cytokines may be isolated and purified in their natural and recombinant forms. The data presented herein also shows that anti-IL-17F antibodies, fusion proteins comprised of IL-17F, and siRNA targeting IL-17R and IL-17RC reduce IL-17F bioactivity. Further, the results show that IL-17F treatment increases Aggrecanase expression in human chondrocytes, which can be reduced by anti-IL-17F antibodies, and that IL-17F and IL-17A are elevated in psoriatic lesions and tissues involved in IBD from human biopsies.

IL-17A and IL-17F are novel proinflammatory cytokines produced by activated T cells. These cytokines share a high degree of amino acid identity, including conserved cysteines that exhibit structural features of a cysteine knot motif Both cytokines have been proposed to share receptor chains and exhibit similar biological functions. Members of the IL-17 cytokine family have been implicated in diseases mediated by abnormal immune responses such as rheumatoid arthritis, inflammatory bowel disorders (IBDs) and asthma. Due to the similarities enumerated above, IL-17A and IL-17F produced by human T cells upon activation were characterized. CD4+ T cells were activated with anti-CD3 in the presence or absence of CD28 costimulation, γ-common cytokines (IL-2, IL-4, IL-7, IL-15, IL-21) or IL-23. Optimal production of IL-17A and IL-17F required TCR as well as CD28 costimulation. Additionally, CD28 and IL-21 act synergistically in IL-17A and IL-17F production, suggesting IL-17A and IL-17F may mediate proinflammatory effects attributed to IL-21 signaling. Under all activating conditions, protein levels of IL-17F were 10-20 fold above those obtained for IL-17A. Interestingly, in addition to IL-17A homodimers and IL-17F homodimers, T cells also produced IL-17A/IL-17F heterodimers. These findings suggest that multiple forms of these cytokines are present *in vivo,* with each form accounting for distinct biological functions, e.g., that the IL-17A/IL-17F heterodimer may constitute a new cytokine target in the treatment of inflammatory diseases.

## Claims

1. A composition comprising a therapeutically effective amount of an IL-17F signalling antagonist for use in the treatment of a subject at risk for, or diagnosed with, a disorder related to increased IL- 17F signalling.

2. The composition of claim 1, wherein the IL-17F signalling antagonist is selected from the group consisting of IL-17F inhibitory polynucleotides, IL- 17R inhibitory polynucleotides, IL- 17RC inhibitory polynucleotides, soluble polypeptides comprising IL- 17R or IL- 17F binding fragments thereof, soluble polypeptides comprising IL-17RC or IL-17F binding fragments thereof, inhibitory anti-IL-17F antibodies, inhibitory anti-IL-17R antibodies, inhibitory IL- 17RC antibodies, and antagonistic small molecules.

3. The composition of claim 2, wherein the IL-17F signalling antagonist is an IL-17R inhibitory polynucleotide.

4. The composition of claim 2, wherein the IL- 17F signalling antagonist is an IL- 17RC inhibitory polynucleotide.

5. The composition of claim 3, wherein the inhibitory polynucleotide is an siRNA selected from the group consisting of the nucleotide sequences set forth in SEQ ID NOs:17-24.

6. The composition of claim 4, wherein the inhibitory polynucleotide is an siRNA selected from the group consisting of the nucleotide sequences set forth in SEQ ID NOs:25-32.

7. The composition of claim 2, wherein the IL- 17F signalling antagonist is a soluble polypeptide comprising IL- 17R or IL- 17F binding fragments thereof.

8. The composition of claim2, wherein the IL- 17F signalling antagonist is a soluble polypeptide comprising IL- 17RC or IL- 17F binding fragments thereof.

9. The composition of claim 7, wherein the soluble polypeptide has the amino acid sequence set forth in SEQ ID NO:34.

10. The composition of claim 8, wherein the soluble polypeptide has the amino acid sequence set forth in SEQ ID NO:35.

11. The composition of claim 2, wherein the EL-17F inhibitory polynucleotide comprises the nucleotide sequence set forth in, or a nucleotide sequence complementary to the nucleotide sequence set forth in, SEQ ID NO:1 or a fragment of SEQ ID NO:1, or an RNA equivalent thereof, and wherein expression of the inhibitory polynucleotide in a cell results in the decreased expression of IL-17F.

12. The composition of claim 2, wherein the IL-17R inhibitory polynucleotide comprises the nucleotide sequence set forth in, or a nucleotide sequence complementary to the nucleotide sequence set forth in, SEQ ID NO:5 or a fragment of SEQ ID NO:5, or an RNA equivalent thereof, and wherein expression of the inhibitory polynucleotide in a cell results in the decreased expression of IL-17R.

13. The composition of claim 2, wherein the BL-17RC inhibitory polynucleotide comprises a nucleotide sequence selected from the group consisting of the nucleotide sequences set forth in, or a nucleotide sequence complementary to a nucleotide sequence selected from the group consisting of the nucleotide sequences set forth in, SEQ ID NO:7, SEQ ID NO:9, SEQ ID NO: 11, SEQ ID NO: 13, and SEQ ID NO: 15 or a fragment of a nucleotide sequence selected from the group consisting of the nucleotide sequences set forth in SEQ ID NO:7, SEQ ID NO:9, SEQ ID NO: 11, SEQ ID NO: 13, and SEQ ID NO: 15, or an RNA equivalent thereof, and wherein expression of the inhibitory polynucleotide in a cell results in the decreased expression of IL- 17RC.

14. The composition of claim 1, wherein the disorder related to increased BL-17F signalling is an inflammatory disorder.

15. The composition of claim 14, wherein the inflammatory disorder is selected from the group consisting of an autoimmune disease, a respiratory disease, and an inflammatory bowel disease.

16. The composition of claim 15, wherein the inflammatory disorder is an autoimmune disease, and the autoimmune disease is selected from the group consisting of arthritis, psoriasis, systemic lupus erythematosus, and multiple sclerosis.

17. The composition of claim 16, wherein the autoimmune disease is rheumatoid arthritis.

18. The composition of claim 15, wherein the inflammatory disorder is a respiratory disease, and the respiratory disease is cystic fibrosis.

19. The composition of claim 15, wherein the inflammatory disorder is an inflammatory bowel disease.

20. The composition of claim 1, further comprising administering to the subject a therapeutically effective amount of at least one additional therapeutic agent.

21. The composition of claim 20, wherein the at least one additional therapeutic agent is selected from the group consisting of cytokine inhibitors, growth factor inhibitors, immunosuppressants, anti-inflammatory agents, metabolic inhibitors, enzyme inhibitors, cytotoxic agents, and cytostatic agents.

22. The composition of claim 20, wherein the at least one additional therapeutic agent is selected from the group consisting of TNF antagonists, anti-TNF agents, IL- 12 antagonists, IL- 15 antagonists, IL- 17 antagonists, IL- 18 antagonists, IL-22 antagonists, T cell-depleting agents, B cell-depleting agents, cyclosporin, FK-506, CCI-779, etanercept, infliximab, rituximab, adalimumab, prednisolone, azathioprine, gold, sulphasalazine, chloroquine, hydroxychloroquine, minocycline, anakinra, abatacept, methotrexate,
leflunomide, rapamycin, rapamycin analogs, Cox-2 inhibitors, cPLA2 inhibitors, NSABDs, p38 inhibitors, antagonists of B7.1, B7.2, ICOSL, ICOS and/or CD28, and agonists of CTLA4.


SEQUENCE LISTING

<110>  Carreno, Beatriz M.
       Collins, Mary
       Wright, Jill F.
       Wolfman, Neil M.
       Arai, Maya
       Jacobs, Kenneth
       Lu, Zhijian
       Guo, Yongjing
       Qiu, Yongchang

<120>  INTERLEUKIN-17F ANTIBODIES AND OTHER IL-17F SIGNALING ANTAGONISTS
        AND USES THEREFOR

<130>  01997.039301

<150>  60/653,260
<151>  2005-02-14

<150>  60/667,492
<151>  2005-04-01

<160>  40

<170>  PatentIn version 3.3

<210>  1
<211>  492
<212>  DNA
<213>  Homo sapiens


<220>
<221>  CDS
<222>  (1)..(492)

<400>  1
atg aca gtg aag acc ctg cat ggc cca gcc atg gtc aag tac ttg ctg        48
Met Thr Val Lys Thr Leu His Gly Pro Ala Met Val Lys Tyr Leu Leu
1               5                   10                  15

ctg tcg ata ttg ggg ctt gcc ttt ctg agt gag gcg gca gct cgg aaa        96
Leu Ser Ile Leu Gly Leu Ala Phe Leu Ser Glu Ala Ala Ala Arg Lys
            20                  25                  30

atc ccc aaa gta gga cat act ttt ttc caa aag cct gag agt tgc ccg       144
Ile Pro Lys Val Gly His Thr Phe Phe Gln Lys Pro Glu Ser Cys Pro
        35                  40                  45

cct gtg cca gga ggt agt atg aag ctt gac att ggc atc atc aat gaa       192
Pro Val Pro Gly Gly Ser Met Lys Leu Asp Ile Gly Ile Ile Asn Glu
    50                  55                  60

aac cag cgc gtt tcc atg tca cgt aac atc gag agc cgc tcc acc tcc       240
Asn Gln Arg Val Ser Met Ser Arg Asn Ile Glu Ser Arg Ser Thr Ser
65                  70                  75                  80

ccc tgg aat tac act gtc act tgg gac ccc aac cgg tac ccc tcg gaa       288
Pro Trp Asn Tyr Thr Val Thr Trp Asp Pro Asn Arg Tyr Pro Ser Glu
                85                  90                  95

gtt gta cag gcc cag tgt agg aac ttg ggc tgc atc aat gct caa gga       336
Val Val Gln Ala Gln Cys Arg Asn Leu Gly Cys Ile Asn Ala Gln Gly
            100                 105                 110

aag gaa gac atc tcc atg aat tcc gtt ccc atc cag caa gag acc ctg       384
Lys Glu Asp Ile Ser Met Asn Ser Val Pro Ile Gln Gln Glu Thr Leu
        115                 120                 125

gtc gtc cgg agg aag cac caa ggc tgc tct gtt tct ttc cag ttg gag       432
Val Val Arg Arg Lys His Gln Gly Cys Ser Val Ser Phe Gln Leu Glu
    130                 135                 140

aag gtg ctg gtg act gtt ggc tgc acc tgc gtc acc cct gtc atc cac       480
Lys Val Leu Val Thr Val Gly Cys Thr Cys Val Thr Pro Val Ile His
145                 150                 155                 160

cat gtg cag taa                                                          492
His Val Gln

<210>  2
<211>  163
<212>  PRT
<213>  Homo sapiens

<400>  2

Met Thr Val Lys Thr Leu His Gly Pro Ala Met Val Lys Tyr Leu Leu
1               5                   10                  15

Leu Ser Ile Leu Gly Leu Ala Phe Leu Ser Glu Ala Ala Ala Arg Lys
            20                  25                  30

Ile Pro Lys Val Gly His Thr Phe Phe Gln Lys Pro Glu Ser Cys Pro
        35                  40                  45

Pro Val Pro Gly Gly Ser Met Lys Leu Asp Ile Gly Ile Ile Asn Glu
    50                  55                  60

Asn Gln Arg Val Ser Met Ser Arg Asn Ile Glu Ser Arg Ser Thr Ser
65                  70                  75                  80

Pro Trp Asn Tyr Thr Val Thr Trp Asp Pro Asn Arg Tyr Pro Ser Glu
                85                  90                  95

Val Val Gln Ala Gln Cys Arg Asn Leu Gly Cys Ile Asn Ala Gln Gly
            100                 105                 110

Lys Glu Asp Ile Ser Met Asn Ser Val Pro Ile Gln Gln Glu Thr Leu
        115                 120                 125

Val Val Arg Arg Lys His Gln Gly Cys Ser Val Ser Phe Gln Leu Glu
    130                 135                 140

Lys Val Leu Val Thr Val Gly Cys Thr Cys Val Thr Pro Val Ile His
145                 150                 155                 160

His Val Gln

<210>  3
<211>  1883
<212>  DNA
<213>  Homo sapiens

<220>
<221>  CDS
<222>  (54)..(521)

<400>  3
   gaattccggc aggcacaaac tcatccatcc ccagttgatt ggaagaaaca acg atg        56
                                                              Met
                                                              1
   act cct ggg aag acc tca ttg gtg tca ctg cta ctg ctg ctg agc ctg      104
   Thr Pro Gly Lys Thr Ser Leu Val Ser Leu Leu Leu Leu Leu Ser Leu
               5                   10                  15
   gag gcc ata gtg aag gca gga atc aca atc cca cga aat cca gga tgc      152
   Glu Ala Ile Val Lys Ala Gly Ile Thr Ile Pro Arg Asn Pro Gly Cys
           20                  25                  30
   cca aat tct gag gac aag aac ttc ccc cgg act gtg atg gtc aac ctg      200
   Pro Asn Ser Glu Asp Lys Asn Phe Pro Arg Thr Val Met Val Asn Leu
       35                  40                  45
   aac atc cat aac cgg aat acc aat acc aat ccc aaa agg tcc tca gat      248
   Asn Ile His Asn Arg Asn Thr Asn Thr Asn Pro Lys Arg Ser Ser Asp
   50                  55                  60                  65
   tac tac aac cga tcc acc tca cct tgg aat ctc cac cgc aat gag gac      296
   Tyr Tyr Asn Arg Ser Thr Ser Pro Trp Asn Leu His Arg Asn Glu Asp
                   70                  75                  80
   cct gag aga tat ccc tct gtg atc tgg gag gca aag tgc cgc cac ttg      344
   Pro Glu Arg Tyr Pro Ser Val Ile Trp Glu Ala Lys Cys Arg His Leu
               85                  90                  95
   ggc tgc atc aac gct gat ggg aac gtg gac tac cac atg aac tct gtc      392
   Gly Cys Ile Asn Ala Asp Gly Asn Val Asp Tyr His Met Asn Ser Val
           100                 105                 110
   ccc atc cag caa gag atc ctg gtc ctg cgc agg gag cct cca cac tgc      440
   Pro Ile Gln Gln Glu Ile Leu Val Leu Arg Arg Glu Pro Pro His Cys
       115                 120                 125
   ccc aac tcc ttc cgg ctg gag aag ata ctg gtg tcc gtg ggc tgc acc      488
   Pro Asn Ser Phe Arg Leu Glu Lys Ile Leu Val Ser Val Gly Cys Thr
   130                 135                 140                 145
   tgt gtc acc ccg att gtc cac cat gtg gcc taa gagctctggg gagcccacac      541
   Cys Val Thr Pro Ile Val His His Val Ala
                   150                 155
   tccccaaagc agttagacta tggagagccg acccagcccc tcaggaaccc tcatccttca      601
   aagacagcct catttcggac taaactcatt agagttctta aggcagtttg tccaattaaa      661
   gcttcagagg taacacttgg ccaagatatg agatctgaat tacctttccc tctttccaag      721
   aaggaaggtt tgactgagta ccaatttgct tcttgtttac ttttttaagg gctttaagtt      781
   atttatgtat ttaatatgcc ctgagataac tttggggtat aagattccat tttaatgaat      841
   tacctacttt attttgtttg tctttttaaa gaagataaga ttctgggctt gggaatttta      901
   ttatttaaaa ggtaaaacct gtatttattt gagctattta aggatctatt tatgtttaag      961
   tatttagaaa aaggtgaaaa agcactatta tcagttctgc ctaggtaaat gtaagataga     1021
   attaaatggc agtgcaaaat ttctgagtct ttacaacata cggatatagt atttcctcct     1081
   ctttgttttt aaaagttata acatggctga aaagaaagat taaacctact ttcatatgta     1141
   ttaatttaaa ttttgcaatt tgttgaggtt ttacaagaga tacagcaagt ctaactctct     1201
   gttccattaa acccttataa taaaatcctt ctgtaataat aaagtttcaa aagaaaatgt     1261
   ttatttgttc tcattaaatg tattttagca aactcagctc ttccctattg ggaagagtta     1321
   tgcaaattct cctataagca aaacaaagca tgtctttgag taacaatgac ctggaaatac     1381
   ccaaaattcc aagttctcga tttcacatgc cttcaagact gaacaccgac taaggttttc     1441
   atactattag ccaatgctgt agacagaagc attttgatag gaatagagca aataagataa     1501
   tggccctgag gaatggcatg tcattattaa agatcatatg gggaaaatga aaccctcccc     1561
   aaaatacaag aagttctggg aggagacatt gtcttcagac tacaatgtcc agtttctccc     1621
   ctagactcag gcttcctttg gagattaagg cccctcagag atcaacagac caacattttt     1681
   ctcttcctca agcaacactc ctagggcctg gcttctgtct gatcaaggca ccacacaacc     1741
   cagaaaggag ctgatggggc agaatgaact ttaagtatga gaaaagttca gcccaagtaa     1801
   aataaaaact caatcacatt caattccaga gtagtttcaa gtttcacatc gtaaccattt 1861
   tcgcccggaa ttcaaaaaaa aa 1883

<210> 4
<211> 155
<212> PRT
<213> Homo sapiens

<400> 4

Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155

<210> 5
<211> 3420
<212> DNA
<213> Homo sapiens

<220>
<221> CDS
<222> (134)..(2734)

<400> 5
   ggctggaagc cggaagcgag caaagtggag ccgactcgaa ctccaccggc acgagggcgg 60
   aaaagaaagc ctcagaacgt tcgctcgctg cgtccccagc cggggccgag ccctccgcga 120
   cgccacccgg gcc atg ggg gcc gca cgc agc ccg ccg tcc gct gtc ccg 169
   Met Gly Ala Ala Arg Ser Pro Pro Ser Ala Val Pro
   1 5 10
   ggg ccc ctg ctg ggg ctg ctc ctg ctg ctc ctg ggc gtg ctg gcc ccg 217
   Gly Pro Leu Leu Gly Leu Leu Leu Leu Leu Leu Gly Val Leu Ala Pro
   15 20 25
   ggt ggc gcc tcc ctg cga ctc ctg gac cac cgg gcg ctg gtc tgc tcc 265
   Gly Gly Ala Ser Leu Arg Leu Leu Asp His Arg Ala Leu Val Cys Ser
   30                  35                  40
   cag ccg ggg cta aac tgc acg gtc aag aat agt acc tgc ctg gat gac      313
   Gln Pro Gly Leu Asn Cys Thr Val Lys Asn Ser Thr Cys Leu Asp Asp
   45                  50                  55                  60
   agc tgg att cac cct cga aac ctg acc ccc tcc tcc cca aag gac ctg      361
   Ser Trp Ile His Pro Arg Asn Leu Thr Pro Ser Ser Pro Lys Asp Leu
                   65                  70                  75
   cag atc cag ctg cac ttt gcc cac acc caa caa gga gac ctg ttc ccc      409
   Gln Ile Gln Leu His Phe Ala His Thr Gln Gln Gly Asp Leu Phe Pro
               80                  85                  90
   gtg gct cac atc gaa tgg aca ctg cag aca gac gcc agc atc ctg tac      457
   Val Ala His Ile Glu Trp Thr Leu Gln Thr Asp Ala Ser Ile Leu Tyr
           95                  100                 105
   ctc gag ggt gca gag tta tct gtc ctg cag ctg aac acc aat gaa cgt      505
   Leu Glu Gly Ala Glu Leu Ser Val Leu Gln Leu Asn Thr Asn Glu Arg
       110                 115                 120
   ttg tgc gtc agg ttt gag ttt ctg tcc aaa ctg agg cat cac cac agg      553
   Leu Cys Val Arg Phe Glu Phe Leu Ser Lys Leu Arg His His His Arg
   125                 130                 135                 140
   cgg tgg cgt ttt acc ttc agc cac ttt gtg gtt gac cct gac cag gaa      601
   Arg Trp Arg Phe Thr Phe Ser His Phe Val Val Asp Pro Asp Gln Glu
                   145                 150                 155
   tat gag gtg acc gtt cac cac ctg ccc aag ccc atc cct gat ggg gac      649
   Tyr Glu Val Thr Val His His Leu Pro Lys Pro Ile Pro Asp Gly Asp
               160                 165                 170
   cca aac cac cag tcc aag aat ttc ctt gtg cct gac tgt gag cac gcc      697
   Pro Asn His Gln Ser Lys Asn Phe Leu Val Pro Asp Cys Glu His Ala
           175                 180                 185
   agg atg aag gta acc acg cca tgc atg agc tca ggc agc ctg tgg gac      745
   Arg Met Lys Val Thr Thr Pro Cys Met Ser Ser Gly Ser Leu Trp Asp
       190                 195                 200
   ccc aac atc acc gtg gag acc ctg gag gcc cac cag ctg cgt gtg agc      793
   Pro Asn Ile Thr Val Glu Thr Leu Glu Ala His Gln Leu Arg Val Ser
   205                 210                 215                 220
   ttc acc ctg tgg aac gaa tct acc cat tac cag atc ctg ctg acc agt      841
   Phe Thr Leu Trp Asn Glu Ser Thr His Tyr Gln Ile Leu Leu Thr Ser
                   225                 230                 235
   ttt ccg cac atg gag aac cac agt tgc ttt gag cac atg cac cac ata      889
   Phe Pro His Met Glu Asn His Ser Cys Phe Glu His Met His His Ile
               240                 245                 250
   cct gcg ccc aga cca gaa gag ttc cac cag cga tcc aac gtc aca ctc      937
   Pro Ala Pro Arg Pro Glu Glu Phe His Gln Arg Ser Asn Val Thr Leu
           255                 260                 265
   act cta cgc aac ctt aaa ggg tgc tgt cgc cac caa gtg cag atc cag      985
   Thr Leu Arg Asn Leu Lys Gly Cys Cys Arg His Gln Val Gln Ile Gln
       270                 275                 280
   ccc ttc ttc agc agc tgc ctc aat gac tgc ctc aga cac tcc gcg act     1033
   Pro Phe Phe Ser Ser Cys Leu Asn Asp Cys Leu Arg His Ser Ala Thr
   285                 290                 295                 300
   gtt tcc tgc cca gaa atg cca gac act cca gaa cca att ccg gac tac     1081
   Val Ser Cys Pro Glu Met Pro Asp Thr Pro Glu Pro Ile Pro Asp Tyr
                   305                 310                 315
   atg ccc ctg tgg gtg tac tgg ttc atc acg ggc atc tcc atc ctg ctg     1129
   Met Pro Leu Trp Val Tyr Trp Phe Ile Thr Gly Ile Ser Ile Leu Leu
               320                 325                 330
   gtg ggc tcc gtc atc ctg ctc atc gtc tgc atg acc tgg agg cta gct     1177
   Val Gly Ser Val Ile Leu Leu Ile Val Cys Met Thr Trp Arg Leu Ala
           335                 340                 345
   ggg cct gga agt gaa aaa tac agt gat gac acc aaa tac acc gat ggc 1225
   Gly Pro Gly Ser Glu Lys Tyr Ser Asp Asp Thr Lys Tyr Thr Asp Gly
   350 355 360
   ctg cct gcg gct gac ctg atc ccc cca ccg ctg aag ccc agg aag gtc      1273
   Leu Pro Ala Ala Asp Leu Ile Pro Pro Pro Leu Lys Pro Arg Lys Val
   365                 370                 375                 380
   tgg atc atc tac tca gcc gac cac ccc ctc tac gtg gac gtg gtc ctg      1321
   Trp Ile Ile Tyr Ser Ala Asp His Pro Leu Tyr Val Asp Val Val Leu
                   385                 390                 395
   aaa ttc gcc cag ttc ctg ctc acc gcc tgc ggc acg gaa gtg gcc ctg      1369
   Lys Phe Ala Gln Phe Leu Leu Thr Ala Cys Gly Thr Glu Val Ala Leu
               400                 405                 410
   gac ctg ctg gaa gag cag gcc atc tcg gag gca gga gtc atg acc tgg      1417
   Asp Leu Leu Glu Glu Gln Ala Ile Ser Glu Ala Gly Val Met Thr Trp
           415                 420                 425
   gtg ggc cgt cag aag cag gag atg gtg gag agc aac tct aag atc atc      1465
   Val Gly Arg Gln Lys Gln Glu Met Val Glu Ser Asn Ser Lys Ile Ile
       430                 435                 440
   gtc ctg tgc tcc cgc ggc acg cgc gcc aag tgg cag gcg ctc ctg ggc      1513
   Val Leu Cys Ser Arg Gly Thr Arg Ala Lys Trp Gln Ala Leu Leu Gly
   445                 450                 455                 460
   cgg ggg gcg cct gtg cgg ctg cgc tgc gac cac gga aag ccc gtg ggg      1561
   Arg Gly Ala Pro Val Arg Leu Arg Cys Asp His Gly Lys Pro Val Gly
                   465                 470                 475
   gac ctg ttc act gca gcc atg aac atg atc ctc ccg gac ttc aag agg      1609
   Asp Leu Phe Thr Ala Ala Met Asn Met Ile Leu Pro Asp Phe Lys Arg
               480                 485                 490
   cca gcc tgc ttc ggc acc tac gta gtc tgc tac ttc agc gag gtc agc      1657
   Pro Ala Cys Phe Gly Thr Tyr Val Val Cys Tyr Phe Ser Glu Val Ser
           495                 500                 505
   tgt gac ggc gac gtc ccc gac ctg ttc ggc gcg gcg ccg cgg tac ccg      1705
   Cys Asp Gly Asp Val Pro Asp Leu Phe Gly Ala Ala Pro Arg Tyr Pro
       510                 515                 520
   ctc atg gac agg ttc gag gag gtg tac ttc cgc atc cag gac ctg gag      1753
   Leu Met Asp Arg Phe Glu Glu Val Tyr Phe Arg Ile Gln Asp Leu Glu
   525                 530                 535                 540
   atg ttc cag ccg ggc cgc atg cac cgc gta ggg gag ctg tcg ggg gac      1801
   Met Phe Gln Pro Gly Arg Met His Arg Val Gly Glu Leu Ser Gly Asp
                   545                 550                 555
   aac tac ctg cgg agc ccg ggc ggc agg cag ctc cgc gcc gcc ctg gac      1849
   Asn Tyr Leu Arg Ser Pro Gly Gly Arg Gln Leu Arg Ala Ala Leu Asp
               560                 565                 570
   agg ttc cgg gac tgg cag gtc cgc tgt ccc gac tgg ttc gaa tgt gag      1897
   Arg Phe Arg Asp Trp Gln Val Arg Cys Pro Asp Trp Phe Glu Cys Glu
           575                 580                 585
   aac ctc tac tca gca gat gac cag gat gcc ccg tcc ctg gac gaa gag      1945
   Asn Leu Tyr Ser Ala Asp Asp Gln Asp Ala Pro Ser Leu Asp Glu Glu
       590                 595                 600
   gtg ttt gag gag cca ctg ctg cct ccg gga acc ggc atc gtg aag cgg      1993
   Val Phe Glu Glu Pro Leu Leu Pro Pro Gly Thr Gly Ile Val Lys Arg
   605                 610                 615                 620
   gcg ccc ctg gtg cgc gag cct ggc tcc cag gcc tgc ctg gcc ata gac      2041
   Ala Pro Leu Val Arg Glu Pro Gly Ser Gln Ala Cys Leu Ala Ile Asp
                   625                 630                 635
   ccg ctg gtc ggg gag gaa gga gga gca gca gtg gca aag ctg gaa cct      2089
   Pro Leu Val Gly Glu Glu Gly Gly Ala Ala Val Ala Lys Leu Glu Pro
               640                 645                 650
   cac ctg cag ccc cgg ggt cag cca gcg ccg cag ccc ctc cac acc ctg 2137
   His Leu Gln Pro Arg Gly Gln Pro Ala Pro Gln Pro Leu His Thr Leu
   655 660 665
   gtg ctc gcc gca gag gag ggg gcc ctg gtg gcc gcg gtg gag cct ggg 2185
   Val Leu Ala Ala Glu Glu Gly Ala Leu Val Ala Ala Val Glu Pro Gly
   670 675 680
   ccc ctg gct gac ggt gcc gca gtc cgg ctg gca ctg gcg ggg gag ggc 2233
   Pro Leu Ala Asp Gly Ala Ala Val Arg Leu Ala Leu Ala Gly Glu Gly
   685 690 695 700
   gag gcc tgc ccg ctg ctg ggc agc ccg ggc gct ggg cga aat agc gtc 2281
   Glu Ala Cys Pro Leu Leu Gly Ser Pro Gly Ala Gly Arg Asn Ser Val
   705 710 715
   ctc ttc ctc ccc gtg gac ccc gag gac tcg ccc ctt ggc agc agc acc 2329
   Leu Phe Leu Pro Val Asp Pro Glu Asp Ser Pro Leu Gly Ser Ser Thr
   720 725 730
   ccc atg gcg tct cct gac ctc ctt cca gag gac gtg agg gag cac ctc 2377
   Pro Met Ala Ser Pro Asp Leu Leu Pro Glu Asp Val Arg Glu His Leu
   735 740 745
   gaa ggc ttg atg ctc tcg ctc ttc gag cag agt ctg agc tgc cag gcc 2425
   Glu Gly Leu Met Leu Ser Leu Phe Glu Gln Ser Leu Ser Cys Gln Ala
   750 755 760
   cag ggg ggc tgc agt aga ccc gcc atg gtc ctc aca gac cca cac acg 2473
   Gln Gly Gly Cys Ser Arg Pro Ala Met Val Leu Thr Asp Pro His Thr
   765 770 775 780
   ccc tac gag gag gag cag cgg cag tca gtg cag tct gac cag ggc tac 2521
   Pro Tyr Glu Glu Glu Gln Arg Gln Ser Val Gln Ser Asp Gln Gly Tyr
   785 790 795
   atc tcc agg agc tcc ccg cag ccc ccc gag gga ctc acg gaa atg gag 2569
   Ile Ser Arg Ser Ser Pro Gln Pro Pro Glu Gly Leu Thr Glu Met Glu
   800 805 810
   gaa gag gag gaa gag gag cag gac cca ggg aag ccg gcc ctg cca ctc 2617
   Glu Glu Glu Glu Glu Glu Gln Asp Pro Gly Lys Pro Ala Leu Pro Leu
   815 820 825
   tct ccc gag gac ctg gag agc ctg agg agc ctc cag cgg cag ctg ctt 2665
   Ser Pro Glu Asp Leu Glu Ser Leu Arg Ser Leu Gln Arg Gln Leu Leu
   830 835 840
   ttc cgc cag ctg cag aag aac tcg ggc tgg gac acg atg ggg tca gag 2713
   Phe Arg Gln Leu Gln Lys Asn Ser Gly Trp Asp Thr Met Gly Ser Glu
   845 850 855 860
   tca gag ggg ccc agt gca tga gggcggctcc ccagggaccg cccagatccc 2764
   Ser Glu Gly Pro Ser Ala
   865
   agctttgaga gaggagtgtg tgtgcacgta ttcatctgtg tgtacatgtc tgcatgtgta 2824
   tatgttcgtg tgtgaaatgt aggctttaaa atgtaaatgt ctggatttta atcccaggca 2884
   tccctcctaa cttttctttg tgcagcggtc tggttatcgt ctatccccag gggaatccac 2944
   acagcccgct cccaggagct aatggtagag cgtccttgag gctccattat tcgttcattc 3004
   agcatttatt gtgcacctac tatgtggcgg gcatttggga taccaagata aattgcatgc 3064
   ggcatggccc cagccatgaa ggaacttaac cgctagtgcc gaggacacgt taaacgaaca 3124
   ggatgggccg ggcacggtgg ctcacgcctg taatcccagc acactgggag gccgaggcag 3184
   gtggatcact ctgaggtcag gagtttgagc cagcctggcc aacatggtga aaccccatct 3244
   ccactaaaaa tagaaaaatt agccgggcat ggtgacacat gcctgtagtc ctagctactt 3304
   gggaggctga ggcaggagaa ttgcttgaat ctgggaggca gaggttgcag tgagccgaga 3364
   ttgtgccatt gcactgcagc ctggatgaca gagcgagact ctatctcaaa aaaaaa 3420

<210>  6
<211>  866
<212>  PRT
<213>  Homo sapiens

<400>  6

Met Gly Ala Ala Arg Ser Pro Pro Ser Ala Val Pro Gly Pro Leu Leu
1               5                   10                  15

Gly Leu Leu Leu Leu Leu Leu Gly Val Leu Ala Pro Gly Gly Ala Ser
            20                  25                  30

Leu Arg Leu Leu Asp His Arg Ala Leu Val Cys Ser Gln Pro Gly Leu
        35                  40                  45

Asn Cys Thr Val Lys Asn Ser Thr Cys Leu Asp Asp Ser Trp Ile His
    50                  55                  60

Pro Arg Asn Leu Thr Pro Ser Ser Pro Lys Asp Leu Gln Ile Gln Leu
65                  70                  75                  80

His Phe Ala His Thr Gln Gln Gly Asp Leu Phe Pro Val Ala His Ile
                85                  90                  95

Glu Trp Thr Leu Gln Thr Asp Ala Ser Ile Leu Tyr Leu Glu Gly Ala
            100                 105                 110

Glu Leu Ser Val Leu Gln Leu Asn Thr Asn Glu Arg Leu Cys Val Arg
        115                 120                 125

Phe Glu Phe Leu Ser Lys Leu Arg His His His Arg Arg Trp Arg Phe
    130                 135                 140

Thr Phe Ser His Phe Val Val Asp Pro Asp Gln Glu Tyr Glu Val Thr
145                 150                 155                 160

Val His His Leu Pro Lys Pro Ile Pro Asp Gly Asp Pro Asn His Gln
                165                 170                 175

Ser Lys Asn Phe Leu Val Pro Asp Cys Glu His Ala Arg Met Lys Val
            180                 185                 190

Thr Thr Pro Cys Met Ser Ser Gly Ser Leu Trp Asp Pro Asn Ile Thr
        195                 200                 205

Val Glu Thr Leu Glu Ala His Gln Leu Arg Val Ser Phe Thr Leu Trp
    210                 215                 220

Asn Glu Ser Thr His Tyr Gln Ile Leu Leu Thr Ser Phe Pro His Met
225                 230                 235                 240

Glu Asn His Ser Cys Phe Glu His Met His His Ile Pro Ala Pro Arg
                245                 250                 255

Pro Glu Glu Phe His Gln Arg Ser Asn Val Thr Leu Thr Leu Arg Asn
            260                 265                 270

Leu Lys Gly Cys Cys Arg His Gln Val Gln Ile Gln Pro Phe Phe Ser
        275                 280                 285

Ser Cys Leu Asn Asp Cys Leu Arg His Ser Ala Thr Val Ser Cys Pro
    290                 295                 300

Glu Met Pro Asp Thr Pro Glu Pro Ile Pro Asp Tyr Met Pro Leu Trp
305                 310                 315                 320

Val Tyr Trp Phe Ile Thr Gly Ile Ser Ile Leu Leu Val Gly Ser Val
                325                 330                 335

Ile Leu Leu Ile Val Cys Met Thr Trp Arg Leu Ala Gly Pro Gly Ser
            340                 345                 350

Glu Lys Tyr Ser Asp Asp Thr Lys Tyr Thr Asp Gly Leu Pro Ala Ala
        355                 360                 365

Asp Leu Ile Pro Pro Pro Leu Lys Pro Arg Lys Val Trp Ile Ile Tyr
    370                 375                 380

Ser Ala Asp His Pro Leu Tyr Val Asp Val Val Leu Lys Phe Ala Gln
385                 390                 395                 400

Phe Leu Leu Thr Ala Cys Gly Thr Glu Val Ala Leu Asp Leu Leu Glu
                405                 410                 415

Glu Gln Ala Ile Ser Glu Ala Gly Val Met Thr Trp Val Gly Arg Gln
            420                 425                 430

Lys Gln Glu Met Val Glu Ser Asn Ser Lys Ile Ile Val Leu Cys Ser
        435                 440                 445

Arg Gly Thr Arg Ala Lys Trp Gln Ala Leu Leu Gly Arg Gly Ala Pro
    450                 455                 460

Val Arg Leu Arg Cys Asp His Gly Lys Pro Val Gly Asp Leu Phe Thr
465                 470                 475                 480

Ala Ala Met Asn Met Ile Leu Pro Asp Phe Lys Arg Pro Ala Cys Phe
                485                 490                 495

Gly Thr Tyr Val Val Cys Tyr Phe Ser Glu Val Ser Cys Asp Gly Asp
            500                 505                 510

Val Pro Asp Leu Phe Gly Ala Ala Pro Arg Tyr Pro Leu Met Asp Arg
        515                 520                 525

Phe Glu Glu Val Tyr Phe Arg Ile Gln Asp Leu Glu Met Phe Gln Pro
    530                 535                 540

Gly Arg Met His Arg Val Gly Glu Leu Ser Gly Asp Asn Tyr Leu Arg
545                 550                 555                 560

Ser Pro Gly Gly Arg Gln Leu Arg Ala Ala Leu Asp Arg Phe Arg Asp
                565                 570                 575

Trp Gln Val Arg Cys Pro Asp Trp Phe Glu Cys Glu Asn Leu Tyr Ser

            580                 585                 590

Ala Asp Asp Gln Asp Ala Pro Ser Leu Asp Glu Glu Val Phe Glu Glu
        595                 600                 605

Pro Leu Leu Pro Pro Gly Thr Gly Ile Val Lys Arg Ala Pro Leu Val
    610                 615                 620

Arg Glu Pro Gly Ser Gln Ala Cys Leu Ala Ile Asp Pro Leu Val Gly
625                 630                 635                 640

Glu Glu Gly Gly Ala Ala Val Ala Lys Leu Glu Pro His Leu Gln Pro
                645                 650                 655

Arg Gly Gln Pro Ala Pro Gln Pro Leu His Thr Leu Val Leu Ala Ala
            660                 665                 670

Glu Glu Gly Ala Leu Val Ala Ala Val Glu Pro Gly Pro Leu Ala Asp
        675                 680                 685

Gly Ala Ala Val Arg Leu Ala Leu Ala Gly Glu Gly Glu Ala Cys Pro
    690                 695                 700

Leu Leu Gly Ser Pro Gly Ala Gly Arg Asn Ser Val Leu Phe Leu Pro
705                 710                 715                 720

Val Asp Pro Glu Asp Ser Pro Leu Gly Ser Ser Thr Pro Met Ala Ser
                725                 730                 735

Pro Asp Leu Leu Pro Glu Asp Val Arg Glu His Leu Glu Gly Leu Met
            740                 745                 750

Leu Ser Leu Phe Glu Gln Ser Leu Ser Cys Gln Ala Gln Gly Gly Cys
        755                 760                 765

Ser Arg Pro Ala Met Val Leu Thr Asp Pro His Thr Pro Tyr Glu Glu
    770                 775                 780

Glu Gln Arg Gln Ser Val Gln Ser Asp Gln Gly Tyr Ile Ser Arg Ser
785                 790                 795                 800

Ser Pro Gln Pro Pro Glu Gly Leu Thr Glu Met Glu Glu Glu Glu Glu
                805                 810                 815

Glu Glu Gln Asp Pro Gly Lys Pro Ala Leu Pro Leu Ser Pro Glu Asp
            820                 825                 830

Leu Glu Ser Leu Arg Ser Leu Gln Arg Gln Leu Leu Phe Arg Gln Leu
        835                 840                 845

Gln Lys Asn Ser Gly Trp Asp Thr Met Gly Ser Glu Ser Glu Gly Pro
    850                 855                 860

Ser Ala
865

<210> 7
<211> 2691

<212>  DNA
<213>  Homo sapiens


<220>
<221>  CDS
<222>  (219)..(2594)

<400>  7
aaaacgaaag cactccgtgc tggaagtagg aggagagtca ggactcccag gacagagagt      60

gcacaaacta cccagcacag ccccctccgc cccctctgga ggctgaagag ggattccagc     120

ccctgccacc cacagacacg ggctgactgg ggtgtctgcc ccccttgggg gggggcagca     180

cagggcctca ggcctgggtg ccacctggca cctagaag atg cct gtg ccc tgg ttc     236
                                          Met Pro Val Pro Trp Phe
                                          1               5

ttg ctg tcc ttg gca ctg ggc cga agc cca gtg gtc ctt tct ctg gag       284
Leu Leu Ser Leu Ala Leu Gly Arg Ser Pro Val Val Leu Ser Leu Glu
            10                  15                  20

agg ctt gtg ggg cct cag gac gct acc cac tgc tct ccg gtg agt ctg       332
Arg Leu Val Gly Pro Gln Asp Ala Thr His Cys Ser Pro Val Ser Leu
        25                  30                  35

gaa ccc tgg gga gac gag gaa agg ctc agg gtt cag ttt ttg gct cag       380
Glu Pro Trp Gly Asp Glu Glu Arg Leu Arg Val Gln Phe Leu Ala Gln
    40                  45                  50

caa agc ctt agc ctg gct cct gtc act gct gcc act gcc aga act gcc       428
Gln Ser Leu Ser Leu Ala Pro Val Thr Ala Ala Thr Ala Arg Thr Ala
55                  60                  65                  70

ctg tct ggt ctg tct ggt gct gat ggt aga aga gaa gaa cgg gga agg       476
Leu Ser Gly Leu Ser Gly Ala Asp Gly Arg Arg Glu Glu Arg Gly Arg
                75                  80                  85

ggc aag agc tgg gtc tgt ctt tct ctg gga ggg tct ggg aat acg gag       524
Gly Lys Ser Trp Val Cys Leu Ser Leu Gly Gly Ser Gly Asn Thr Glu
            90                  95                  100

ccc cag aaa aag ggc ctc tcc tgc cgc ctc tgg gac agt gac ata ctc       572
Pro Gln Lys Lys Gly Leu Ser Cys Arg Leu Trp Asp Ser Asp Ile Leu
        105                 110                 115

tgc ctg cct ggg gac atc gtg cct gct ccg ggc ccc gtg ctg gcg cct       620
Cys Leu Pro Gly Asp Ile Val Pro Ala Pro Gly Pro Val Leu Ala Pro
    120                 125                 130

acg cac ctg cag aca gag ctg gtg ctg agg tgc cag aag gag acc gac       668
Thr His Leu Gln Thr Glu Leu Val Leu Arg Cys Gln Lys Glu Thr Asp
135                 140                 145                 150

tgt gac ctc tgt ctg cgt gtg gct gtc cac ttg gcc gtg cat ggg cac       716
Cys Asp Leu Cys Leu Arg Val Ala Val His Leu Ala Val His Gly His
                155                 160                 165

tgg gaa gag cct gaa gat gag gaa aag ttt gga gga gca gct gac tca       764
Trp Glu Glu Pro Glu Asp Glu Glu Lys Phe Gly Gly Ala Ala Asp Ser
            170                 175                 180

ggg gtg gag gag cct agg aat gcc tct ctc cag gcc caa gtc gtg ctc       812
Gly Val Glu Glu Pro Arg Asn Ala Ser Leu Gln Ala Gln Val Val Leu
        185                 190                 195

tcc ttc cag gcc tac cct act gcc cgc tgc gtc ctg ctg gag gtg caa       860
Ser Phe Gln Ala Tyr Pro Thr Ala Arg Cys Val Leu Leu Glu Val Gln
    200                 205                 210

gtg cct gct gcc ctt gtg cag ttt ggt cag tct gtg ggc tct gtg gta       908
Val Pro Ala Ala Leu Val Gln Phe Gly Gln Ser Val Gly Ser Val Val
215                 220                 225                 230

tat gac tgc ttc gag gct gcc cta ggg agt gag gta cga atc tgg tcc       956
Tyr Asp Cys Phe Glu Ala Ala Leu Gly Ser Glu Val Arg Ile Trp Ser

                235                 240                 245

tat act cag ccc agg tac gag aag gaa ctc aac cac aca cag cag ctg 1004
Tyr Thr Gln Pro Arg Tyr Glu Lys Glu Leu Asn His Thr Gln Gln Leu
            250                 255                 260

cct gac tgc agg ggg ctc gaa gtc tgg aac agc atc ccg agc tgc tgg 1052
Pro Asp Cys Arg Gly Leu Glu Val Trp Asn Ser Ile Pro Ser Cys Trp
        265                 270                 275

gcc ctg ccc tgg ctc aac gtg tca gca gat ggt gac aac gtg cat ctg 1100
Ala Leu Pro Trp Leu Asn Val Ser Ala Asp Gly Asp Asn Val His Leu
    280                 285                 290

gtt ctg aat gtc tct gag gag cag cac ttc ggc ctc tcc ctg tac tgg 1148
Val Leu Asn Val Ser Glu Glu Gln His Phe Gly Leu Ser Leu Tyr Trp
295                 300                 305                 310

aat cag gtc cag ggc ccc cca aaa ccc cgg tgg cac aaa aac ctg act 1196
Asn Gln Val Gln Gly Pro Pro Lys Pro Arg Trp His Lys Asn Leu Thr
                315                 320                 325

gga ccg cag atc att acc ttg aac cac aca gac ctg gtt ccc tgc ctc 1244
Gly Pro Gln Ile Ile Thr Leu Asn His Thr Asp Leu Val Pro Cys Leu
            330                 335                 340

tgt att cag gtg tgg cct ctg gaa cct gac tcc gtt agg acg aac atc 1292
Cys Ile Gln Val Trp Pro Leu Glu Pro Asp Ser Val Arg Thr Asn Ile
        345                 350                 355

tgc ccc ttc agg gag gac ccc cgc gca cac cag aac ctc tgg caa gcc 1340
Cys Pro Phe Arg Glu Asp Pro Arg Ala His Gln Asn Leu Trp Gln Ala
    360                 365                 370

gcc cga ctg cga ctg ctg acc ctg cag agc tgg ctg ctg gac gca ccg 1388
Ala Arg Leu Arg Leu Leu Thr Leu Gln Ser Trp Leu Leu Asp Ala Pro
375                 380                 385                 390

tgc tcg ctg ccc gca gaa gcg gca ctg tgc tgg cgg gct ccg ggt ggg 1436
Cys Ser Leu Pro Ala Glu Ala Ala Leu Cys Trp Arg Ala Pro Gly Gly
                395                 400                 405

gac ccc tgc cag cca ctg gtc cca ccg ctt tcc tgg gag aac gtc act 1484
Asp Pro Cys Gln Pro Leu Val Pro Pro Leu Ser Trp Glu Asn Val Thr
            410                 415                 420

gtg gac aag gtt ctc gag ttc cca ttg ctg aaa ggc cac cct aac ctc 1532
Val Asp Lys Val Leu Glu Phe Pro Leu Leu Lys Gly His Pro Asn Leu
        425                 430                 435

tgt gtt cag gtg aac agc tcg gag aag ctg cag ctg cag gag tgc ttg 1580
Cys Val Gln Val Asn Ser Ser Glu Lys Leu Gln Leu Gln Glu Cys Leu
    440                 445                 450

tgg gct gac tcc ctg ggg cct ctc aaa gac gat gtg cta ctg ttg gag 1628
Trp Ala Asp Ser Leu Gly Pro Leu Lys Asp Asp Val Leu Leu Leu Glu
455                 460                 465                 470

aca cga ggc ccc cag gac aac aga tcc ctc tgt gcc ttg gaa ccc agt 1676
Thr Arg Gly Pro Gln Asp Asn Arg Ser Leu Cys Ala Leu Glu Pro Ser
                475                 480                 485

ggc tgt act tca cta ccc agc aaa gcc tcc acg agg gca gct cgc ctt 1724
Gly Cys Thr Ser Leu Pro Ser Lys Ala Ser Thr Arg Ala Ala Arg Leu
            490                 495                 500

gga gag tac tta cta caa gac ctg cag tca ggc cag tgt ctg cag cta 1772
Gly Glu Tyr Leu Leu Gln Asp Leu Gln Ser Gly Gln Cys Leu Gln Leu
        505                 510                 515

tgg gac gat gac ttg gga gcg cta tgg gcc tgc ccc atg gac aaa tac 1820
Trp Asp Asp Asp Leu Gly Ala Leu Trp Ala Cys Pro Met Asp Lys Tyr
    520                 525                 530

atc cac aag cgc tgg gcc ctc gtg tgg ctg gcc tgc cta ctc ttt gcc 1868
Ile His Lys Arg Trp Ala Leu Val Trp Leu Ala Cys Leu Leu Phe Ala
535                 540                 545                 550

gct gcg ctt tcc ctc atc ctc ctt ctc aaa aag gat cac gcg aaa ggg 1916
Ala Ala Leu Ser Leu Ile Leu Leu Leu Lys Lys Asp His Ala Lys Gly
                555                 560                 565

tgg ctg agg ctc ttg aaa cag gac gtc cgc tcg ggg gcg gcc gcc agg 1964
Trp Leu Arg Leu Leu Lys Gln Asp Val Arg Ser Gly Ala Ala Ala Arg
            570                 575                 580

ggc cgc gcg gct ctg ctc ctc tac tca gcc gat gac tcg ggt ttc gag 2012
Gly Arg Ala Ala Leu Leu Leu Tyr Ser Ala Asp Asp Ser Gly Phe Glu
        585                 590                 595

cgc ctg gtg ggc gcc ctg gcg tcg gcc ctg tgc cag ctg ccg ctg cgc 2060
Arg Leu Val Gly Ala Leu Ala Ser Ala Leu Cys Gln Leu Pro Leu Arg
    600                 605                 610

gtg gcc gta gac ctg tgg agc cgt cgt gaa ctg agc gcg cag ggg ccc 2108
Val Ala Val Asp Leu Trp Ser Arg Arg Glu Leu Ser Ala Gln Gly Pro
615                 620                 625                 630

gtg gct tgg ttt cac gcg cag cgg cgc cag acc ctg cag gag ggc ggc 2156
Val Ala Trp Phe His Ala Gln Arg Arg Gln Thr Leu Gln Glu Gly Gly
                635                 640                 645

gtg gtg gtc ttg ctc ttc tct ccc ggt gcg gtg gcg ctg tgc agc gag 2204
Val Val Val Leu Leu Phe Ser Pro Gly Ala Val Ala Leu Cys Ser Glu
            650                 655                 660

tgg cta cag gat ggg gtg tcc ggg ccc ggg gcg cac ggc ccg cac gac 2252
Trp Leu Gln Asp Gly Val Ser Gly Pro Gly Ala His Gly Pro His Asp
        665                 670                 675

gcc ttc cgc gcc tcg ctc agc tgc gtg ctg ccc gac ttc ttg cag ggc 2300
Ala Phe Arg Ala Ser Leu Ser Cys Val Leu Pro Asp Phe Leu Gln Gly
    680                 685                 690

cgg gcg ccc ggc agc tac gtg ggg gcc tgc ttc gac agg ctg ctc cac 2348
Arg Ala Pro Gly Ser Tyr Val Gly Ala Cys Phe Asp Arg Leu Leu His
695                 700                 705                 710

ccg gac gcc gta ccc gcc ctt ttc cgc acc gtg ccc gtc ttc aca ctg 2396
Pro Asp Ala Val Pro Ala Leu Phe Arg Thr Val Pro Val Phe Thr Leu
                715                 720                 725

ccc tcc caa ctg cca gac ttc ctg ggg gcc ctg cag cag cct cgc gcc 2444
Pro Ser Gln Leu Pro Asp Phe Leu Gly Ala Leu Gln Gln Pro Arg Ala
            730                 735                 740

ccg cgt tcc ggg cgg ctc caa gag aga gcg gag caa gtg tcc cgg gcc 2492
Pro Arg Ser Gly Arg Leu Gln Glu Arg Ala Glu Gln Val Ser Arg Ala
        745                 750                 755

ctt cag cca gcc ctg gat agc tac ttc cat ccc ccg ggg act ccc gcg 2540
Leu Gln Pro Ala Leu Asp Ser Tyr Phe His Pro Pro Gly Thr Pro Ala
    760                 765                 770

ccg gga cgc ggg gtg gga cca ggg gcg gga cct ggg gcg ggg gac ggg 2588
Pro Gly Arg Gly Val Gly Pro Gly Ala Gly Pro Gly Ala Gly Asp Gly
775                 780                 785                 790

act taa ataaaggcag acgctgtttt tctacccatg tggcccaaaa aaaaaaaaa 2644
Thr

aaaaaaaaaa aaaaaaaaa aaaaaaaaa aaaaaaaaa aaaaaaa 2691

<210> 8
<211> 791
<212> PRT
<213> Homo sapiens

<400> 8

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Val Ser Leu Glu Pro Trp Gly Asp Glu Glu Arg Leu Arg
        35                  40                  45

Val Gln Phe Leu Ala Gln Gln Ser Leu Ser Leu Ala Pro Val Thr Ala
    50                  55                  60

Ala Thr Ala Arg Thr Ala Leu Ser Gly Leu Ser Gly Ala Asp Gly Arg
65                  70                  75                  80

Arg Glu Glu Arg Gly Arg Gly Lys Ser Trp Val Cys Leu Ser Leu Gly
                85                  90                  95

Gly Ser Gly Asn Thr Glu Pro Gln Lys Lys Gly Leu Ser Cys Arg Leu
            100                 105                 110

Trp Asp Ser Asp Ile Leu Cys Leu Pro Gly Asp Ile Val Pro Ala Pro
        115                 120                 125

Gly Pro Val Leu Ala Pro Thr His Leu Gln Thr Glu Leu Val Leu Arg
    130                 135                 140

Cys Gln Lys Glu Thr Asp Cys Asp Leu Cys Leu Arg Val Ala Val His
145                 150                 155                 160

Leu Ala Val His Gly His Trp Glu Glu Pro Glu Asp Glu Glu Lys Phe
                165                 170                 175

Gly Gly Ala Ala Asp Ser Gly Val Glu Glu Pro Arg Asn Ala Ser Leu
            180                 185                 190

Gln Ala Gln Val Val Leu Ser Phe Gln Ala Tyr Pro Thr Ala Arg Cys
        195                 200                 205

Val Leu Leu Glu Val Gln Val Pro Ala Ala Leu Val Gln Phe Gly Gln
    210                 215                 220

Ser Val Gly Ser Val Val Tyr Asp Cys Phe Glu Ala Ala Leu Gly Ser
225                 230                 235                 240

Glu Val Arg Ile Trp Ser Tyr Thr Gln Pro Arg Tyr Glu Lys Glu Leu
                245                 250                 255

Asn His Thr Gln Gln Leu Pro Asp Cys Arg Gly Leu Glu Val Trp Asn
            260                 265                 270

Ser Ile Pro Ser Cys Trp Ala Leu Pro Trp Leu Asn Val Ser Ala Asp
        275                 280                 285

Gly Asp Asn Val His Leu Val Leu Asn Val Ser Glu Glu Gln His Phe
    290                 295                 300

Gly Leu Ser Leu Tyr Trp Asn Gln Val Gln Gly Pro Pro Lys Pro Arg
305                 310                 315                 320

Trp His Lys Asn Leu Thr Gly Pro Gln Ile Ile Thr Leu Asn His Thr
                325                 330                 335

Asp Leu Val Pro Cys Leu Cys Ile Gln Val Trp Pro Leu Glu Pro Asp
            340                 345                 350

Ser Val Arg Thr Asn Ile Cys Pro Phe Arg Glu Asp Pro Arg Ala His
        355                 360                 365

Gln Asn Leu Trp Gln Ala Ala Arg Leu Arg Leu Leu Thr Leu Gln Ser
    370                 375                 380

Trp Leu Leu Asp Ala Pro Cys Ser Leu Pro Ala Glu Ala Ala Leu Cys
385                 390                 395                 400

Trp Arg Ala Pro Gly Gly Asp Pro Cys Gln Pro Leu Val Pro Pro Leu
                405                 410                 415

Ser Trp Glu Asn Val Thr Val Asp Lys Val Leu Glu Phe Pro Leu Leu
            420                 425                 430

Lys Gly His Pro Asn Leu Cys Val Gln Val Asn Ser Ser Glu Lys Leu
        435                 440                 445

Gln Leu Gln Glu Cys Leu Trp Ala Asp Ser Leu Gly Pro Leu Lys Asp
    450                 455                 460

Asp Val Leu Leu Leu Glu Thr Arg Gly Pro Gln Asp Asn Arg Ser Leu
465                 470                 475                 480

Cys Ala Leu Glu Pro Ser Gly Cys Thr Ser Leu Pro Ser Lys Ala Ser
                485                 490                 495

Thr Arg Ala Ala Arg Leu Gly Glu Tyr Leu Leu Gln Asp Leu Gln Ser
            500                 505                 510

Gly Gln Cys Leu Gln Leu Trp Asp Asp Asp Leu Gly Ala Leu Trp Ala
        515                 520                 525

Cys Pro Met Asp Lys Tyr Ile His Lys Arg Trp Ala Leu Val Trp Leu
    530                 535                 540

Ala Cys Leu Leu Phe Ala Ala Ala Leu Ser Leu Ile Leu Leu Leu Lys
545                 550                 555                 560

Lys Asp His Ala Lys Gly Trp Leu Arg Leu Leu Lys Gln Asp Val Arg
                565                 570                 575

Ser Gly Ala Ala Ala Arg Gly Arg Ala Ala Leu Leu Leu Tyr Ser Ala
            580                 585                 590

Asp Asp Ser Gly Phe Glu Arg Leu Val Gly Ala Leu Ala Ser Ala Leu
        595                 600                 605

Cys Gln Leu Pro Leu Arg Val Ala Val Asp Leu Trp Ser Arg Arg Glu
    610                 615                 620

Leu Ser Ala Gln Gly Pro Val Ala Trp Phe His Ala Gln Arg Arg Gln
625                 630                 635                 640

Thr Leu Gln Glu Gly Gly Val Val Val Leu Leu Phe Ser Pro Gly Ala
                645                 650                 655

Val Ala Leu Cys Ser Glu Trp Leu Gln Asp Gly Val Ser Gly Pro Gly
            660                 665                 670

Ala His Gly Pro His Asp Ala Phe Arg Ala Ser Leu Ser Cys Val Leu
        675                 680                 685

Pro Asp Phe Leu Gln Gly Arg Ala Pro Gly Ser Tyr Val Gly Ala Cys
    690                 695                 700

Phe Asp Arg Leu Leu His Pro Asp Ala Val Pro Ala Leu Phe Arg Thr
705                 710                 715                 720

Val Pro Val Phe Thr Leu Pro Ser Gln Leu Pro Asp Phe Leu Gly Ala
                725                 730                 735

Leu Gln Gln Pro Arg Ala Pro Arg Ser Gly Arg Leu Gln Glu Arg Ala
            740                 745                 750

Glu Gln Val Ser Arg Ala Leu Gln Pro Ala Leu Asp Ser Tyr Phe His
        755                 760                 765

Pro Pro Gly Thr Pro Ala Pro Gly Arg Gly Val Gly Pro Gly Ala Gly
    770                 775                 780

Pro Gly Ala Gly Asp Gly Thr
785                 790

<210>  9
<211>  2478
<212>  DNA
<213>  Homo sapiens

<220>
<221>  CDS
<222>  (219)..(2381)

<400>  9
aaaacgaaag cactccgtgc tggaagtagg aggagagtca ggactcccag gacagagagt      60

gcacaaacta cccagcacag ccccctccgc cccctctgga ggctgaagag ggattccagc     120

ccctgccacc cacagacacg ggctgactgg ggtgtctgcc ccccttgggg gggggcagca     180

cagggcctca ggcctgggtg ccacctggca cctagaag atg cct gtg ccc tgg ttc     236
                                         Met Pro Val Pro Trp Phe
                                         1               5

ttg ctg tcc ttg gca ctg ggc cga agc cca gtg gtc ctt tct ctg gag     284
Leu Leu Ser Leu Ala Leu Gly Arg Ser Pro Val Val Leu Ser Leu Glu
            10                  15                  20

agg ctt gtg ggg cct cag gac gct acc cac tgc tct ccg ggc ctc tcc     332
Arg Leu Val Gly Pro Gln Asp Ala Thr His Cys Ser Pro Gly Leu Ser
        25                  30                  35

tgc cgc ctc tgg gac agt gac ata ctc tgc ctg cct ggg gac atc gtg     380
Cys Arg Leu Trp Asp Ser Asp Ile Leu Cys Leu Pro Gly Asp Ile Val
    40                  45                  50

cct gct ccg ggc ccc gtg ctg gcg cct acg cac ctg cag aca gag ctg 428
Pro Ala Pro Gly Pro Val Leu Ala Pro Thr His Leu Gln Thr Glu Leu
55                  60                  65                  70

gtg ctg agg tgc cag aag gag acc gac tgt gac ctc tgt ctg cgt gtg 476
Val Leu Arg Cys Gln Lys Glu Thr Asp Cys Asp Leu Cys Leu Arg Val
                75                  80                  85

gct gtc cac ttg gcc gtg cat ggg cac tgg gaa gag cct gaa gat gag 524
Ala Val His Leu Ala Val His Gly His Trp Glu Glu Pro Glu Asp Glu
            90                  95                  100

gaa aag ttt gga gga gca gct gac tca ggg gtg gag gag cct agg aat 572
Glu Lys Phe Gly Gly Ala Ala Asp Ser Gly Val Glu Glu Pro Arg Asn
        105                 110                 115

gcc tct ctc cag gcc caa gtc gtg ctc tcc ttc cag gcc tac cct act 620
Ala Ser Leu Gln Ala Gln Val Val Leu Ser Phe Gln Ala Tyr Pro Thr
    120                 125                 130

gcc cgc tgc gtc ctg ctg gag gtg caa gtg cct gct gcc ctt gtg cag 668
Ala Arg Cys Val Leu Leu Glu Val Gln Val Pro Ala Ala Leu Val Gln
135                 140                 145                 150

ttt ggt cag tct gtg ggc tct gtg gta tat gac tgc ttc gag gct gcc 716
Phe Gly Gln Ser Val Gly Ser Val Val Tyr Asp Cys Phe Glu Ala Ala
                155                 160                 165

cta ggg agt gag gta cga atc tgg tcc tat act cag ccc agg tac gag 764
Leu Gly Ser Glu Val Arg Ile Trp Ser Tyr Thr Gln Pro Arg Tyr Glu
            170                 175                 180

aag gaa ctc aac cac aca cag cag ctg cct gac tgc agg ggg ctc gaa 812
Lys Glu Leu Asn His Thr Gln Gln Leu Pro Asp Cys Arg Gly Leu Glu
        185                 190                 195

gtc tgg aac agc atc ccg agc tgc tgg gcc ctg ccc tgg ctc aac gtg 860
Val Trp Asn Ser Ile Pro Ser Cys Trp Ala Leu Pro Trp Leu Asn Val
    200                 205                 210

tca gca gat ggt gac aac gtg cat ctg gtt ctg aat gtc tct gag gag 908
Ser Ala Asp Gly Asp Asn Val His Leu Val Leu Asn Val Ser Glu Glu
215                 220                 225                 230

cag cac ttc ggc ctc tcc ctg tac tgg aat cag gtc cag ggc ccc cca 956
Gln His Phe Gly Leu Ser Leu Tyr Trp Asn Gln Val Gln Gly Pro Pro
                235                 240                 245

aaa ccc cgg tgg cac aaa aac ctg act gga ccg cag atc att acc ttg 1004
Lys Pro Arg Trp His Lys Asn Leu Thr Gly Pro Gln Ile Ile Thr Leu
            250                 255                 260

aac cac aca gac ctg gtt ccc tgc ctc tgt att cag gtg tgg cct ctg 1052
Asn His Thr Asp Leu Val Pro Cys Leu Cys Ile Gln Val Trp Pro Leu
        265                 270                 275

gaa cct gac tcc gtt agg acg aac atc tgc ccc ttc agg gag gac ccc 1100
Glu Pro Asp Ser Val Arg Thr Asn Ile Cys Pro Phe Arg Glu Asp Pro
    280                 285                 290

cgc gca cac cag aac ctc tgg caa gcc gcc cga ctg cga ctg ctg acc 1148
Arg Ala His Gln Asn Leu Trp Gln Ala Ala Arg Leu Arg Leu Leu Thr
295                 300                 305                 310

ctg cag agc tgg ctg ctg gac gca ccg tgc tcg ctg ccc gca gaa gcg 1196
Leu Gln Ser Trp Leu Leu Asp Ala Pro Cys Ser Leu Pro Ala Glu Ala
                315                 320                 325

gca ctg tgc tgg cgg gct ccg ggt ggg gac ccc tgc cag cca ctg gtc 1244
Ala Leu Cys Trp Arg Ala Pro Gly Gly Asp Pro Cys Gln Pro Leu Val
            330                 335                 340

cca ccg ctt tcc tgg gag aac gtc act gtg gac aag gtt ctc gag ttc 1292
Pro Pro Leu Ser Trp Glu Asn Val Thr Val Asp Lys Val Leu Glu Phe
        345                 350                 355

cca ttg ctg aaa ggc cac cct aac ctc tgt gtt cag gtg aac agc tcg 1340

Pro Leu Leu Lys Gly His Pro Asn Leu Cys Val Gln Val Asn Ser Ser
    360                 365                 370

gag aag ctg cag ctg cag gag tgc ttg tgg gct gac tcc ctg ggg cct      1388
Glu Lys Leu Gln Leu Gln Glu Cys Leu Trp Ala Asp Ser Leu Gly Pro
375                 380                 385                 390

ctc aaa gac gat gtg cta ctg ttg gag aca cga ggc ccc cag gac aac      1436
Leu Lys Asp Asp Val Leu Leu Leu Glu Thr Arg Gly Pro Gln Asp Asn
                395                 400                 405

aga tcc ctc tgt gcc ttg gaa ccc agt ggc tgt act tca cta ccc agc      1484
Arg Ser Leu Cys Ala Leu Glu Pro Ser Gly Cys Thr Ser Leu Pro Ser
            410                 415                 420

aaa gcc tcc acg agg gca gct cgc ctt gga gag tac tta cta caa gac      1532
Lys Ala Ser Thr Arg Ala Ala Arg Leu Gly Glu Tyr Leu Leu Gln Asp
        425                 430                 435

ctg cag tca ggc cag tgt ctg cag cta tgg gac gat gac ttg gga gcg      1580
Leu Gln Ser Gly Gln Cys Leu Gln Leu Trp Asp Asp Asp Leu Gly Ala
    440                 445                 450

cta tgg gcc tgc ccc atg gac aaa tac atc cac aag cgc tgg gcc ctc      1628
Leu Trp Ala Cys Pro Met Asp Lys Tyr Ile His Lys Arg Trp Ala Leu
455                 460                 465                 470

gtg tgg ctg gcc tgc cta ctc ttt gcc gct gcg ctt tcc ctc atc ctc      1676
Val Trp Leu Ala Cys Leu Leu Phe Ala Ala Ala Leu Ser Leu Ile Leu
                475                 480                 485

ctt ctc aaa aag gat cac gcg aaa ggg tgg ctg agg ctc ttg aaa cag      1724
Leu Leu Lys Lys Asp His Ala Lys Gly Trp Leu Arg Leu Leu Lys Gln
            490                 495                 500

gac gtc cgc tcg ggg gcg gcc gcc agg ggc cgc gcg gct ctg ctc ctc      1772
Asp Val Arg Ser Gly Ala Ala Ala Arg Gly Arg Ala Ala Leu Leu Leu
        505                 510                 515

tac tca gcc gat gac tcg ggt ttc gag cgc ctg gtg ggc gcc ctg gcg      1820
Tyr Ser Ala Asp Asp Ser Gly Phe Glu Arg Leu Val Gly Ala Leu Ala
    520                 525                 530

tcg gcc ctg tgc cag ctg ccg ctg cgc gtg gcc gta gac ctg tgg agc      1868
Ser Ala Leu Cys Gln Leu Pro Leu Arg Val Ala Val Asp Leu Trp Ser
535                 540                 545                 550

cgt cgt gaa ctg agc gcg cag ggg ccc gtg gct tgg ttt cac gcg cag      1916
Arg Arg Glu Leu Ser Ala Gln Gly Pro Val Ala Trp Phe His Ala Gln
                555                 560                 565

cgg cgc cag acc ctg cag gag ggc ggc gtg gtg gtc ttg ctc ttc tct      1964
Arg Arg Gln Thr Leu Gln Glu Gly Gly Val Val Val Leu Leu Phe Ser
            570                 575                 580

ccc ggt gcg gtg gcg ctg tgc agc gag tgg cta cag gat ggg gtg tcc      2012
Pro Gly Ala Val Ala Leu Cys Ser Glu Trp Leu Gln Asp Gly Val Ser
        585                 590                 595

ggg ccc ggg gcg cac ggc ccg cac gac gcc ttc cgc gcc tcg ctc agc      2060
Gly Pro Gly Ala His Gly Pro His Asp Ala Phe Arg Ala Ser Leu Ser
    600                 605                 610

tgc gtg ctg ccc gac ttc ttg cag ggc cgg gcg ccc ggc agc tac gtg      2108
Cys Val Leu Pro Asp Phe Leu Gln Gly Arg Ala Pro Gly Ser Tyr Val
615                 620                 625                 630

ggg gcc tgc ttc gac agg ctg ctc cac ccg gac gcc gta ccc gcc ctt      2156
Gly Ala Cys Phe Asp Arg Leu Leu His Pro Asp Ala Val Pro Ala Leu
                635                 640                 645

ttc cgc acc gtg ccc gtc ttc aca ctg ccc tcc caa ctg cca gac ttc      2204
Phe Arg Thr Val Pro Val Phe Thr Leu Pro Ser Gln Leu Pro Asp Phe
            650                 655                 660

ctg ggg gcc ctg cag cag cct cgc gcc ccg cgt tcc ggg cgg ctc caa      2252
Leu Gly Ala Leu Gln Gln Pro Arg Ala Pro Arg Ser Gly Arg Leu Gln

                665                 670                 675

gag aga gcg gag caa gtg tcc cgg gcc ctt cag cca gcc ctg gat agc      2300
Glu Arg Ala Glu Gln Val Ser Arg Ala Leu Gln Pro Ala Leu Asp Ser
        680                 685                 690

tac ttc cat ccc ccg ggg act ccc gcg ccg gga cgc ggg gtg gga cca      2348
Tyr Phe His Pro Pro Gly Thr Pro Ala Pro Gly Arg Gly Val Gly Pro
695                 700                 705                 710

ggg gcg gga cct ggg gcg ggg gac ggg act taa ataaaggcag acgctgtttt    2401
Gly Ala Gly Pro Gly Ala Gly Asp Gly Thr
                715                 720

tctacccatg tggcccaaaa aaaaaaaaaa aaaaaaaaaa aaaaaaaaaa aaaaaaaaaa    2461

aaaaaaaaaa aaaaaaa                                                   2478


<210>  10
<211>  720
<212>  PRT
<213>  Homo sapiens

<400>  10

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Gly Leu Ser Cys Arg Leu Trp Asp Ser Asp Ile Leu Cys
        35                  40                  45

Leu Pro Gly Asp Ile Val Pro Ala Pro Gly Pro Val Leu Ala Pro Thr
    50                  55                  60

His Leu Gln Thr Glu Leu Val Leu Arg Cys Gln Lys Glu Thr Asp Cys
65                  70                  75                  80

Asp Leu Cys Leu Arg Val Ala Val His Leu Ala Val His Gly His Trp
                85                  90                  95

Glu Glu Pro Glu Asp Glu Glu Lys Phe Gly Gly Ala Ala Asp Ser Gly
            100                 105                 110

Val Glu Glu Pro Arg Asn Ala Ser Leu Gln Ala Gln Val Val Leu Ser
        115                 120                 125

Phe Gln Ala Tyr Pro Thr Ala Arg Cys Val Leu Leu Glu Val Gln Val
    130                 135                 140

Pro Ala Ala Leu Val Gln Phe Gly Gln Ser Val Gly Ser Val Val Tyr
145                 150                 155                 160

Asp Cys Phe Glu Ala Ala Leu Gly Ser Glu Val Arg Ile Trp Ser Tyr
                165                 170                 175

Thr Gln Pro Arg Tyr Glu Lys Glu Leu Asn His Thr Gln Gln Leu Pro
            180                 185                 190

Asp Cys Arg Gly Leu Glu Val Trp Asn Ser Ile Pro Ser Cys Trp Ala
        195                 200                 205

Leu Pro Trp Leu Asn Val Ser Ala Asp Gly Asp Asn Val His Leu Val
    210                 215                 220

Leu Asn Val Ser Glu Glu Gln His Phe Gly Leu Ser Leu Tyr Trp Asn
225                 230                 235                 240

Gln Val Gln Gly Pro Pro Lys Pro Arg Trp His Lys Asn Leu Thr Gly
                245                 250                 255

Pro Gln Ile Ile Thr Leu Asn His Thr Asp Leu Val Pro Cys Leu Cys
            260                 265                 270

Ile Gln Val Trp Pro Leu Glu Pro Asp Ser Val Arg Thr Asn Ile Cys
        275                 280                 285

Pro Phe Arg Glu Asp Pro Arg Ala His Gln Asn Leu Trp Gln Ala Ala
    290                 295                 300

Arg Leu Arg Leu Leu Thr Leu Gln Ser Trp Leu Leu Asp Ala Pro Cys
305                 310                 315                 320

Ser Leu Pro Ala Glu Ala Ala Leu Cys Trp Arg Ala Pro Gly Gly Asp
                325                 330                 335

Pro Cys Gln Pro Leu Val Pro Pro Leu Ser Trp Glu Asn Val Thr Val
            340                 345                 350

Asp Lys Val Leu Glu Phe Pro Leu Leu Lys Gly His Pro Asn Leu Cys
        355                 360                 365

Val Gln Val Asn Ser Ser Glu Lys Leu Gln Leu Gln Glu Cys Leu Trp
    370                 375                 380

Ala Asp Ser Leu Gly Pro Leu Lys Asp Asp Val Leu Leu Leu Glu Thr
385                 390                 395                 400

Arg Gly Pro Gln Asp Asn Arg Ser Leu Cys Ala Leu Glu Pro Ser Gly
                405                 410                 415

Cys Thr Ser Leu Pro Ser Lys Ala Ser Thr Arg Ala Ala Arg Leu Gly
            420                 425                 430

Glu Tyr Leu Leu Gln Asp Leu Gln Ser Gly Gln Cys Leu Gln Leu Trp
        435                 440                 445

Asp Asp Asp Leu Gly Ala Leu Trp Ala Cys Pro Met Asp Lys Tyr Ile
    450                 455                 460

His Lys Arg Trp Ala Leu Val Trp Leu Ala Cys Leu Leu Phe Ala Ala
465                 470                 475                 480

Ala Leu Ser Leu Ile Leu Leu Leu Lys Lys Asp His Ala Lys Gly Trp
                485                 490                 495

Leu Arg Leu Leu Lys Gln Asp Val Arg Ser Gly Ala Ala Ala Arg Gly
            500                 505                 510

Arg Ala Ala Leu Leu Leu Tyr Ser Ala Asp Asp Ser Gly Phe Glu Arg
        515                 520                 525

Leu Val Gly Ala Leu Ala Ser Ala Leu Cys Gln Leu Pro Leu Arg Val
    530                 535                 540

Ala Val Asp Leu Trp Ser Arg Arg Glu Leu Ser Ala Gln Gly Pro Val
545                 550                 555                 560

Ala Trp Phe His Ala Gln Arg Arg Gln Thr Leu Gln Glu Gly Gly Val
                565                 570                 575

Val Val Leu Leu Phe Ser Pro Gly Ala Val Ala Leu Cys Ser Glu Trp
            580                 585                 590

Leu Gln Asp Gly Val Ser Gly Pro Gly Ala His Gly Pro His Asp Ala
        595                 600                 605

Phe Arg Ala Ser Leu Ser Cys Val Leu Pro Asp Phe Leu Gln Gly Arg
    610                 615                 620

Ala Pro Gly Ser Tyr Val Gly Ala Cys Phe Asp Arg Leu Leu His Pro
625                 630                 635                 640

Asp Ala Val Pro Ala Leu Phe Arg Thr Val Pro Val Phe Thr Leu Pro
                645                 650                 655

Ser Gln Leu Pro Asp Phe Leu Gly Ala Leu Gln Gln Pro Arg Ala Pro
            660                 665                 670

Arg Ser Gly Arg Leu Gln Glu Arg Ala Glu Gln Val Ser Arg Ala Leu
        675                 680                 685

Gln Pro Ala Leu Asp Ser Tyr Phe His Pro Pro Gly Thr Pro Ala Pro
    690                 695                 700

Gly Arg Gly Val Gly Pro Gly Ala Gly Pro Gly Ala Gly Asp Gly Thr
705                 710                 715                 720

<210>  11
<211>  2527
<212>  DNA
<213>  Homo sapiens

<220>
<221>  CDS
<222>  (219)..(1835)

<400>  11
aaaacgaaag cactccgtgc tggaagtagg aggagagtca ggactcccag gacagagagt      60

gcacaaacta cccagcacag ccccctccgc cccctctgga ggctgaagag ggattccagc     120

ccctgccacc cacagacacg ggctgactgg ggtgtctgcc ccccttgggg gggggcagca     180

cagggcctca ggcctgggtg ccacctggca cctagaag atg cct gtg ccc tgg ttc     236
                                          Met Pro Val Pro Trp Phe
                                          1               5

ttg ctg tcc ttg gca ctg ggc cga agc cca gtg gtc ctt tct ctg gag       284
Leu Leu Ser Leu Ala Leu Gly Arg Ser Pro Val Val Leu Ser Leu Glu
            10                  15                  20

agg ctt gtg ggg cct cag gac gct acc cac tgc tct ccg ggc ctc tcc 332
Arg Leu Val Gly Pro Gln Asp Ala Thr His Cys Ser Pro Gly Leu Ser
        25                  30                  35

tgc cgc ctc tgg gac agt gac ata ctc tgc ctg cct ggg gac atc gtg 380
Cys Arg Leu Trp Asp Ser Asp Ile Leu Cys Leu Pro Gly Asp Ile Val
    40                  45                  50

cct gct ccg ggc ccc gtg ctg gcg cct acg cac ctg cag aca gag ctg 428
Pro Ala Pro Gly Pro Val Leu Ala Pro Thr His Leu Gln Thr Glu Leu
55                  60                  65                  70

gtg ctg agg tgc cag aag gag acc gac tgt gac ctc tgt ctg cgt gtg 476
Val Leu Arg Cys Gln Lys Glu Thr Asp Cys Asp Leu Cys Leu Arg Val
                75                  80                  85

gct gtc cac ttg gcc gtg cat ggg cac tgg gaa gag cct gaa gat gag 524
Ala Val His Leu Ala Val His Gly His Trp Glu Glu Pro Glu Asp Glu
            90                  95                  100

gaa aag ttt gga gga gca gct gac tca ggg gtg gag gag cct agg aat 572
Glu Lys Phe Gly Gly Ala Ala Asp Ser Gly Val Glu Glu Pro Arg Asn
        105                 110                 115

gcc tct ctc cag gcc caa gtc gtg ctc tcc ttc cag gcc tac cct act 620
Ala Ser Leu Gln Ala Gln Val Val Leu Ser Phe Gln Ala Tyr Pro Thr
    120                 125                 130

gcc cgc tgc gtc ctg ctg gag gtg caa gtg cct gct gcc ctt gtg cag 668
Ala Arg Cys Val Leu Leu Glu Val Gln Val Pro Ala Ala Leu Val Gln
135                 140                 145                 150

ttt ggt cag tct gtg ggc tct gtg gta tat gac tgc ttc gag gct gcc 716
Phe Gly Gln Ser Val Gly Ser Val Val Tyr Asp Cys Phe Glu Ala Ala
                155                 160                 165

cta ggg agt gag gta cga atc tgg tcc tat act cag ccc agg tac gag 764
Leu Gly Ser Glu Val Arg Ile Trp Ser Tyr Thr Gln Pro Arg Tyr Glu
            170                 175                 180

aag gaa ctc aac cac aca cag cag ctg cct gcc ctg ccc tgg ctc aac 812
Lys Glu Leu Asn His Thr Gln Gln Leu Pro Ala Leu Pro Trp Leu Asn
        185                 190                 195

gtg tca gca gat ggt gac aac gtg cat ctg gtt ctg aat gtc tct gag 860
Val Ser Ala Asp Gly Asp Asn Val His Leu Val Leu Asn Val Ser Glu
    200                 205                 210

gag cag cac ttc ggc ctc tcc ctg tac tgg aat cag gtc cag ggc ccc 908
Glu Gln His Phe Gly Leu Ser Leu Tyr Trp Asn Gln Val Gln Gly Pro
215                 220                 225                 230

cca aaa ccc cgg tgg cac aaa aac ctg act gga ccg cag atc att acc 956
Pro Lys Pro Arg Trp His Lys Asn Leu Thr Gly Pro Gln Ile Ile Thr
                235                 240                 245

ttg aac cac aca gac ctg gtt ccc tgc ctc tgt att cag gtg tgg cct 1004
Leu Asn His Thr Asp Leu Val Pro Cys Leu Cys Ile Gln Val Trp Pro
            250                 255                 260

ctg gaa cct gac tcc gtt agg acg aac atc tgc ccc ttc agg gag gac 1052
Leu Glu Pro Asp Ser Val Arg Thr Asn Ile Cys Pro Phe Arg Glu Asp
        265                 270                 275

ccc cgc gca cac cag aac ctc tgg caa gcc gcc cga ctg cga ctg ctg 1100
Pro Arg Ala His Gln Asn Leu Trp Gln Ala Ala Arg Leu Arg Leu Leu
    280                 285                 290

acc ctg cag agc tgg ctg ctg gac gca ccg tgc tcg ctg ccc gca gaa 1148
Thr Leu Gln Ser Trp Leu Leu Asp Ala Pro Cys Ser Leu Pro Ala Glu
295                 300                 305                 310

gcg gca ctg tgc tgg cgg gct ccg ggt ggg gac ccc tgc cag cca ctg 1196
Ala Ala Leu Cys Trp Arg Ala Pro Gly Gly Asp Pro Cys Gln Pro Leu
                315                 320                 325

gtc cca ccg ctt tcc tgg gag aac gtc act gtg gac aag gtt ctc gag 1244

Val Pro Pro Leu Ser Trp Glu Asn Val Thr Val Asp Lys Val Leu Glu
            330                 335                 340

ttc cca ttg ctg aaa ggc cac cct aac ctc tgt gtt cag gtg aac agc      1292
Phe Pro Leu Leu Lys Gly His Pro Asn Leu Cys Val Gln Val Asn Ser
        345                 350                 355

tcg gag aag ctg cag ctg cag gag tgc ttg tgg gct gac tcc ctg ggg      1340
Ser Glu Lys Leu Gln Leu Gln Glu Cys Leu Trp Ala Asp Ser Leu Gly
    360                 365                 370

cct ctc aaa gac gat gtg cta ctg ttg gag aca cga ggc ccc cag gac      1388
Pro Leu Lys Asp Asp Val Leu Leu Leu Glu Thr Arg Gly Pro Gln Asp
375                 380                 385                 390

aac aga tcc ctc tgt gcc ttg gaa ccc agt ggc tgt act tca cta ccc      1436
Asn Arg Ser Leu Cys Ala Leu Glu Pro Ser Gly Cys Thr Ser Leu Pro
                395                 400                 405

agc aaa gcc tcc acg agg gca gct cgc ctt gga gag tac tta cta caa      1484
Ser Lys Ala Ser Thr Arg Ala Ala Arg Leu Gly Glu Tyr Leu Leu Gln
            410                 415                 420

gac ctg cag tca ggc cag tgt ctg cag cta tgg gac gat gac ttg gga      1532
Asp Leu Gln Ser Gly Gln Cys Leu Gln Leu Trp Asp Asp Asp Leu Gly
        425                 430                 435

gcg cta tgg gcc tgc ccc atg gac aaa tac atc cac aag cgc tgg gcc      1580
Ala Leu Trp Ala Cys Pro Met Asp Lys Tyr Ile His Lys Arg Trp Ala
    440                 445                 450

ctc gtg tgg ctg gcc tgc cta ctc ttt gcc gct gcg ctt tcc ctc atc      1628
Leu Val Trp Leu Ala Cys Leu Leu Phe Ala Ala Ala Leu Ser Leu Ile
455                 460                 465                 470

ctc ctt ctc aaa aag gat cac gcg aaa ggg tgg ctg agg ctc ttg aaa      1676
Leu Leu Leu Lys Lys Asp His Ala Lys Gly Trp Leu Arg Leu Leu Lys
                475                 480                 485

cag gac gtc cgc tcg ggg ggt gag tgg gag caa gcg ctg ggc gga ggg      1724
Gln Asp Val Arg Ser Gly Gly Glu Trp Glu Gln Ala Leu Gly Gly Gly
            490                 495                 500

ccg ccc ccg ggg agc cag gcc tgt gcc agc tca cct ctt ccc tcc cca      1772
Pro Pro Pro Gly Ser Gln Ala Cys Ala Ser Ser Pro Leu Pro Ser Pro
        505                 510                 515

tct gtt ttc tcc ggc agc ggc cgc cag ggg ccg cgc ggc tct gct cct      1820
Ser Val Phe Ser Gly Ser Gly Arg Gln Gly Pro Arg Gly Ser Ala Pro
    520                 525                 530

cta ctc agc cga tga ctcgggtttc gagcgcctgg tgggcgccct ggcgtcggcc      1875
Leu Leu Ser Arg
535

ctgtgccagc tgccgctgcg cgtggccgta gacctgtgga gccgtcgtga actgagcgcg      1935

caggggcccg tggcttggtt tcacgcgcag cggcgccaga ccctgcagga gggcggcgtg      1995

gtggtcttgc tcttctctcc cggtgcggtg gcgctgtgca gcgagtggct acaggatggg      2055

gtgtccgggc ccggggcgca cggcccgcac gacgccttcc gcgcctcgct cagctgcgtg      2115

ctgcccgact tcttgcaggg ccgggcgccc ggcagctacg tgggggcctg cttcgacagg      2175

ctgctccacc cggacgccgt acccgccctt ttccgcaccg tgcccgtctt cacactgccc      2235

tcccaactgc cagacttcct gggggccctg cagcagcctc gcgccccgcg ttccgggcgg      2295

ctccaagaga gagcggagca agtgtcccgg gcccttcagc cagccctgga tagctacttc      2355

catcccccgg ggactcccgc gccgggacgc ggggtgggac caggggcggg acctggggcg      2415

ggggacggga cttaaataaa ggcagacgct gtttttctac ccatgtggcc caaaaaaaaa      2475

aaaaaaaaaa aaaaaaaaaa aaaaaaaaaa aaaaaaaaaa aaaaaaaaaa aa              2527

<210>  12
<211>  538
<212>  PRT
<213>  Homo sapiens

<400>  12

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Gly Leu Ser Cys Arg Leu Trp Asp Ser Asp Ile Leu Cys
        35                  40                  45

Leu Pro Gly Asp Ile Val Pro Ala Pro Gly Pro Val Leu Ala Pro Thr
    50                  55                  60

His Leu Gln Thr Glu Leu Val Leu Arg Cys Gln Lys Glu Thr Asp Cys
65                  70                  75                  80

Asp Leu Cys Leu Arg Val Ala Val His Leu Ala Val His Gly His Trp
                85                  90                  95

Glu Glu Pro Glu Asp Glu Glu Lys Phe Gly Gly Ala Ala Asp Ser Gly
            100                 105                 110

Val Glu Glu Pro Arg Asn Ala Ser Leu Gln Ala Gln Val Val Leu Ser
        115                 120                 125

Phe Gln Ala Tyr Pro Thr Ala Arg Cys Val Leu Leu Glu Val Gln Val
    130                 135                 140

Pro Ala Ala Leu Val Gln Phe Gly Gln Ser Val Gly Ser Val Val Tyr
145                 150                 155                 160

Asp Cys Phe Glu Ala Ala Leu Gly Ser Glu Val Arg Ile Trp Ser Tyr
                165                 170                 175

Thr Gln Pro Arg Tyr Glu Lys Glu Leu Asn His Thr Gln Gln Leu Pro
            180                 185                 190

Ala Leu Pro Trp Leu Asn Val Ser Ala Asp Gly Asp Asn Val His Leu
        195                 200                 205

Val Leu Asn Val Ser Glu Glu Gln His Phe Gly Leu Ser Leu Tyr Trp
    210                 215                 220

Asn Gln Val Gln Gly Pro Pro Lys Pro Arg Trp His Lys Asn Leu Thr
225                 230                 235                 240

Gly Pro Gln Ile Ile Thr Leu Asn His Thr Asp Leu Val Pro Cys Leu
                245                 250                 255

Cys Ile Gln Val Trp Pro Leu Glu Pro Asp Ser Val Arg Thr Asn Ile
            260                 265                 270

Cys Pro Phe Arg Glu Asp Pro Arg Ala His Gln Asn Leu Trp Gln Ala

275                 280                 285

Ala Arg Leu Arg Leu Leu Thr Leu Gln Ser Trp Leu Leu Asp Ala Pro
    290                 295                 300

Cys Ser Leu Pro Ala Glu Ala Ala Leu Cys Trp Arg Ala Pro Gly Gly
305                 310                 315                 320

Asp Pro Cys Gln Pro Leu Val Pro Pro Leu Ser Trp Glu Asn Val Thr
                325                 330                 335

Val Asp Lys Val Leu Glu Phe Pro Leu Leu Lys Gly His Pro Asn Leu
            340                 345                 350

Cys Val Gln Val Asn Ser Ser Glu Lys Leu Gln Leu Gln Glu Cys Leu
        355                 360                 365

Trp Ala Asp Ser Leu Gly Pro Leu Lys Asp Asp Val Leu Leu Leu Glu
    370                 375                 380

Thr Arg Gly Pro Gln Asp Asn Arg Ser Leu Cys Ala Leu Glu Pro Ser
385                 390                 395                 400

Gly Cys Thr Ser Leu Pro Ser Lys Ala Ser Thr Arg Ala Ala Arg Leu
                405                 410                 415

Gly Glu Tyr Leu Leu Gln Asp Leu Gln Ser Gly Gln Cys Leu Gln Leu
            420                 425                 430

Trp Asp Asp Asp Leu Gly Ala Leu Trp Ala Cys Pro Met Asp Lys Tyr
        435                 440                 445

Ile His Lys Arg Trp Ala Leu Val Trp Leu Ala Cys Leu Leu Phe Ala
    450                 455                 460

Ala Ala Leu Ser Leu Ile Leu Leu Leu Lys Lys Asp His Ala Lys Gly
465                 470                 475                 480

Trp Leu Arg Leu Leu Lys Gln Asp Val Arg Ser Gly Gly Glu Trp Glu
                485                 490                 495

Gln Ala Leu Gly Gly Gly Pro Pro Pro Gly Ser Gln Ala Cys Ala Ser
            500                 505                 510

Ser Pro Leu Pro Ser Pro Ser Val Phe Ser Gly Ser Gly Arg Gln Gly
        515                 520                 525

Pro Arg Gly Ser Ala Pro Leu Leu Ser Arg
    530                 535

<210>  13
<211>  2584
<212>  DNA
<213>  Homo sapiens

<220>
<221>  CDS
<222>  (219)..(1022)

<400>  13
aaaacgaaag cactccgtgc tggaagtagg aggagagtca ggactcccag gacagagagt

gcacaaacta cccagcacag ccccctccgc cccctctgga ggctgaagag ggattccagc

ccctgccacc cacagacacg ggctgactgg ggtgtctgcc ccccttgggg gggggcagca

cagggcctca ggcctgggtg ccacctggca cctagaag atg cct gtg ccc tgg ttc
                                         Met Pro Val Pro Trp Phe
                                         1               5

ttg ctg tcc ttg gca ctg ggc cga agc cca gtg gtc ctt tct ctg gag
Leu Leu Ser Leu Ala Leu Gly Arg Ser Pro Val Val Leu Ser Leu Glu
            10                  15                  20

agg ctt gtg ggg cct cag gac gct acc cac tgc tct ccg ggc ctc tcc
Arg Leu Val Gly Pro Gln Asp Ala Thr His Cys Ser Pro Gly Leu Ser
        25                  30                  35

tgc cgc ctc tgg gac agt gac ata ctc tgc ctg cct ggg gac atc gtg
Cys Arg Leu Trp Asp Ser Asp Ile Leu Cys Leu Pro Gly Asp Ile Val
    40                  45                  50

cct gct ccg ggc ccc gtg ctg gcg cct acg cac ctg cag aca gag ctg
Pro Ala Pro Gly Pro Val Leu Ala Pro Thr His Leu Gln Thr Glu Leu
55                  60                  65                  70

gtg ctg agg tgc cag aag gag acc gac tgt gac ctc tgt ctg cgt gtg
Val Leu Arg Cys Gln Lys Glu Thr Asp Cys Asp Leu Cys Leu Arg Val
                75                  80                  85

gct gtc cac ttg gcc gtg cat ggg cac tgg gaa gag cct gaa gat gag
Ala Val His Leu Ala Val His Gly His Trp Glu Glu Pro Glu Asp Glu
            90                  95                  100

gaa aag ttt gga gga gca gct gac tca ggg gtg gag gag cct agg aat
Glu Lys Phe Gly Gly Ala Ala Asp Ser Gly Val Glu Glu Pro Arg Asn
        105                 110                 115

gcc tct ctc cag gcc caa gtc gtg ctc tcc ttc cag gcc tac cct act
Ala Ser Leu Gln Ala Gln Val Val Leu Ser Phe Gln Ala Tyr Pro Thr
    120                 125                 130

gcc cgc tgc gtc ctg ctg gag gtg caa gtg cct gct gcc ctt gtg cag
Ala Arg Cys Val Leu Leu Glu Val Gln Val Pro Ala Ala Leu Val Gln
135                 140                 145                 150

ttt ggt cag tct gtg ggc tct gtg gta tat gac tgc ttc gag gct gcc
Phe Gly Gln Ser Val Gly Ser Val Val Tyr Asp Cys Phe Glu Ala Ala
                155                 160                 165

cta ggg agt gag gta cga atc tgg tcc tat act cag ccc agg tac gag
Leu Gly Ser Glu Val Arg Ile Trp Ser Tyr Thr Gln Pro Arg Tyr Glu
            170                 175                 180

aag gaa ctc aac cac aca cag cag ctg cct gcc ctg ccc tgg ctc aac
Lys Glu Leu Asn His Thr Gln Gln Leu Pro Ala Leu Pro Trp Leu Asn
        185                 190                 195

gtg tca gca gat ggt gac aac gtg cat ctg gtt ctg aat gtc tct gag
Val Ser Ala Asp Gly Asp Asn Val His Leu Val Leu Asn Val Ser Glu
    200                 205                 210

gag cag cac ttc ggc ctc tcc ctg tac tgg aat cag gtc cag ggc ccc
Glu Gln His Phe Gly Leu Ser Leu Tyr Trp Asn Gln Val Gln Gly Pro
215                 220                 225                 230

cca aaa ccc cgg tgg cac aaa aac ctg gtg agg cct ccc cct tcc caa
Pro Lys Pro Arg Trp His Lys Asn Leu Val Arg Pro Pro Pro Ser Gln
                235                 240                 245

gtc cat tcc cac tgt agg ccg atg cct gtg caa agg acg cag tgc cat
Val His Ser His Cys Arg Pro Met Pro Val Gln Arg Thr Gln Cys His
            250                 255                 260

atc aga gag gat cct tga agaggactca ccccaagcaa gggaaattg
Ile Arg Glu Asp Pro

        265

gtgggggaac ttctgccttc ctggtttcct tgactttggc ctcctcctct tcctccttat 1112

cttctccaac ctccttcctt tatttgttcc acagactgga ccgcagatca ttaccttgaa 1172

ccacacagac ctggttccct gcctctgtat tcaggtgtgg cctctggaac ctgactccgt 1232

taggacgaac atctgcccct tcagggagga cccccgcgca caccagaacc tctggcaagc 1292

cgcccgactg cgactgctga ccctgcagag ctggctgctg gacgcaccgt gctcgctgcc 1352

cgcagaagcg gcactgtgct ggcgggctcc gggtggggac ccctgccagc cactggtccc 1412

accgctttcc tgggagaacg tcactgtgga caaggttctc gagttcccat tgctgaaagg 1472

ccaccctaac ctctgtgttc aggtgaacag ctcggagaag ctgcagctgc aggagtgctt 1532

gtgggctgac tccctggggc ctctcaaaga cgatgtgcta ctgttggaga cacgaggccc 1592

ccaggacaac agatccctct gtgccttgga acccagtggc tgtacttcac tacccagcaa 1652

agcctccacg ctatgggacg atgacttggg agcgctatgg gcctgcccca tggacaaata 1712

catccacaag cgctgggccc tcgtgtggct ggcctgccta ctctttgccg ctgcgctttc 1772

cctcatcctc cttctcaaaa aggatcacgc gaaagggtgg ctgaggctct tgaaacagga 1832

cgtccgctcg ggggcggccg ccaggggccg cgcggctctg ctcctctact cagccgatga 1892

ctcgggtttc gagcgcctgg tgggcgccct ggcgtcggcc ctgtgccagc tgccgctgcg 1952

cgtggccgta gacctgtgga gccgtcgtga actgagcgcg caggggcccg tggcttggtt 2012

tcacgcgcag cggcgccaga ccctgcagga gggcggcgtg gtggtcttgc tcttctctcc 2072

cggtgcggtg gcgctgtgca gcgagtggct acaggatggg gtgtccgggc ccggggcgca 2132

cggcccgcac gacgccttcc gcgcctcgct cagctgcgtg ctgcccgact tcttgcaggg 2192

ccgggcgccc ggcagctacg tgggggcctg cttcgacagg ctgctccacc cggacgccgt 2252

acccgccctt ttccgcaccg tgcccgtctt cacactgccc tcccaactgc cagacttcct 2312

gggggccctg cagcagcctc gcgccccgcg ttccgggcgg ctccaagaga gagcggagca 2372

agtgtcccgg gcccttcagc cagccctgga tagctacttc catcccccgg ggactcccgc 2432

gccgggacgc ggggtgggac caggggcggg acctggggcg gggacgggga cttaaataaa 2492

ggcagacgct gtttttctac ccatgtggcc caaaaaaaaa aaaaaaaaaa aaaaaaaaaa 2552

aaaaaaaaaa aaaaaaaaaa aaaaaaaaaa aa 2584

<210> 14
<211> 267
<212> PRT
<213> Homo sapiens

<400> 14

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Gly Leu Ser Cys Arg Leu Trp Asp Ser Asp Ile Leu Cys
        35                  40                  45

Leu Pro Gly Asp Ile Val Pro Ala Pro Gly Pro Val Leu Ala Pro Thr
    50                  55                  60

His Leu Gln Thr Glu Leu Val Leu Arg Cys Gln Lys Glu Thr Asp Cys
65                  70                  75                  80

Asp Leu Cys Leu Arg Val Ala Val His Leu Ala Val His Gly His Trp
                85                  90                  95

Glu Glu Pro Glu Asp Glu Glu Lys Phe Gly Gly Ala Ala Asp Ser Gly
            100                 105                 110

Val Glu Glu Pro Arg Asn Ala Ser Leu Gln Ala Gln Val Val Leu Ser
        115                 120                 125

Phe Gln Ala Tyr Pro Thr Ala Arg Cys Val Leu Leu Glu Val Gln Val
    130                 135                 140

Pro Ala Ala Leu Val Gln Phe Gly Gln Ser Val Gly Ser Val Val Tyr
145                 150                 155                 160

Asp Cys Phe Glu Ala Ala Leu Gly Ser Glu Val Arg Ile Trp Ser Tyr
                165                 170                 175

Thr Gln Pro Arg Tyr Glu Lys Glu Leu Asn His Thr Gln Gln Leu Pro
            180                 185                 190

Ala Leu Pro Trp Leu Asn Val Ser Ala Asp Gly Asp Asn Val His Leu
        195                 200                 205

Val Leu Asn Val Ser Glu Glu Gln His Phe Gly Leu Ser Leu Tyr Trp
    210                 215                 220

Asn Gln Val Gln Gly Pro Pro Lys Pro Arg Trp His Lys Asn Leu Val
225                 230                 235                 240

Arg Pro Pro Pro Ser Gln Val His Ser His Cys Arg Pro Met Pro Val
                245                 250                 255

Gln Arg Thr Gln Cys His Ile Arg Glu Asp Pro
            260                 265

<210>  15
<211>  2427
<212>  DNA
<213>  Homo sapiens

<220>
<221>  CDS
<222>  (219)..(494)

<400>  15
aaaacgaaag cactccgtgc tggaagtagg aggagagtca ggactcccag gacagagagt      60

gcacaaacta cccagcacag ccccctccgc cccctctgga ggctgaagag ggattccagc     120

ccctgccacc cacagacacg ggctgactgg ggtgtctgcc ccccttgggg gggggcagca     180

cagggcctca ggcctgggtg ccacctggca cctagaag atg cct gtg ccc tgg ttc     236
                                          Met Pro Val Pro Trp Phe
                                          1               5

ttg ctg tcc ttg gca ctg ggc cga agc cca gtg gtc ctt tct ctg gag       284
Leu Leu Ser Leu Ala Leu Gly Arg Ser Pro Val Val Leu Ser Leu Glu
            10                  15                  20

agg ctt gtg ggg cct cag gac gct acc cac tgc tct ccg ggc ctc tcc 332
Arg Leu Val Gly Pro Gln Asp Ala Thr His Cys Ser Pro Gly Leu Ser
        25                  30                  35

tgc cgc ctc tgg ggt gcc acc aaa ttc tgg gct tgg aac agc ttc agc 380
Cys Arg Leu Trp Gly Ala Thr Lys Phe Trp Ala Trp Asn Ser Phe Ser
    40                  45                  50

tcc cac ccg ctc ctc cac aca cag aca gtg aca tac tct gcc tgc ctg 428
Ser His Pro Leu Leu His Thr Gln Thr Val Thr Tyr Ser Ala Cys Leu
55                  60                  65                  70

ggg aca tcg tgc ctg ctc cgg gcc ccg tgc tgg cgc cta cgc acc tgc 476
Gly Thr Ser Cys Leu Leu Arg Ala Pro Cys Trp Arg Leu Arg Thr Cys
                75                  80                  85

aga cag agc tgg tgc tga ggtgccagaa ggagaccgac tgtgacctct 524
Arg Gln Ser Trp Cys
            90

gtctgcgtgt ggctgtccac ttggccgtgc atgggcactg ggaagagcct gaagatgagg 584

aaaagtttgg aggagcagct gactcagggg tggaggagcc taggaatgcc tctctccagg 644

cccaagtcgt gctctccttc caggcctacc ctactgcccg ctgcgtcctg ctggaggtgc 704

aagtgcctgc tgcccttgtg cagtttggtc agtctgtggg ctctgtggta tatgactgct 764

tcgaggctgc cctagggagt gaggtacgaa tctggtccta tactcagccc aggtacgaga 824

aggaactcaa ccacacacag cagctgcctg ccctgccctg gctcaacgtg tcagcagatg 884

gtgacaacgt gcatctggtt ctgaatgtct ctgaggagca gcacttcggc ctctccctgt 944

actggaatca ggtccagggc cccccaaaac cccggtggca caaaaacctg gtgaggcctc 1004

ccccttccca agtccattcc cactgtaggc cgatgcctgt gcaaaggacg cagtgccata 1064

tcagagagga tccttgaaga ggactcaccc caagcaaggg aaaattgact ggaccgcaga 1124

tcattacctt gaaccacaca gacctggttc cctgcctctg tattcaggtg tggcctctgg 1184

aacctgactc cgttaggacg aacatctgcc ccttcaggga ggacccccgc gcacaccaga 1244

acctctggca agccgcccga ctgcgactgc tgaccctgca gagctggctg ctggacgcac 1304

cgtgctcgct gcccgcagaa gcggcactgt gctggcgggc tccgggtggg gacccctgcc 1364

agccactggt cccaccgctt tcctgggaga acgtcactgt ggacaaggtt ctcgagttcc 1424

cattgctgaa aggccaccct aacctctgtg ttcaggtgaa cagctcggag aagctgcagc 1484

tgcaggagtg cttgtgggct gctatgggac gatgacttgg gagcgctatg ggcctgcccc 1544

atggacaaat acatccacaa gcgctgggcc ctcgtgtggc tggcctgcct actctttgcc 1604

gctgcgcttt ccctcatcct ccttctcaaa aaggatcacg cgaaagggtg gctgaggctc 1664

ttgaaacagg acgtccgctc gggggcggcc gccaggggcc gcgcggctct gctcctctac 1724

tcagccgatg actcgggttt cgagcgcctg gtgggcgccc tggcgtcggc cctgtgccag 1784

ctgccgctgc gcgtggccgt agacctgtgg agccgtcgtg aactgagcgc gcaggggccc 1844

gtggcttggt ttcacgcgca gcggcgccag accctgcagg agggcggcgt ggtggtcttg 1904

ctcttctctc ccggtgcggt ggcgctgtgc agcgagtggc tacaggatgg ggtgtccggg 1964

cccggggcgc acggcccgca cgacgccttc cgcgcctcgc tcagctgcgt gctgcccgac 2024

ttcttgcagg gccgggcgcc cggcagctac gtgggggcct gcttcgacag gctgctccac 2084

ccggacgccg tacccgccct tttccgcacc gtgcccgtct tcacactgcc ctcccaactg 2144

ccagacttcc tgggggccct gcagcagcct cgcgccccgc gttccgggcg gctccaagag 2204

agagcggagc aagtgtcccg ggcccttcag ccagccctgg atagctactt ccatcccccg 2264

gggactcccg cgccgggacg cggggtggga ccaggggcgg gacctggggc gggggacggg 2324

acttaaataa aggcagacgc tgtttttcta cccatgtggc ccaaaaaaaa aaaaaaaaaa 2384

aaaaaaaaaa aaaaaaaaaa aaaaaaaaaa aaaaaaaaaa aaa 2427

<210> 16
<211> 91
<212> PRT
<213> Homo sapiens

<400> 16

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Gly Leu Ser Cys Arg Leu Trp Gly Ala Thr Lys Phe Trp
        35                  40                  45

Ala Trp Asn Ser Phe Ser Ser His Pro Leu Leu His Thr Gln Thr Val
    50                  55                  60

Thr Tyr Ser Ala Cys Leu Gly Thr Ser Cys Leu Leu Arg Ala Pro Cys
65                  70                  75                  80

Trp Arg Leu Arg Thr Cys Arg Gln Ser Trp Cys
                85                  90

<210> 17
<211> 21
<212> RNA
<213> Artificial

<220>
<223> siRNA IL-17R

<400> 17
gaacaccaau gaacguuugu u 21

<210> 18
<211> 21
<212> RNA
<213> Artificial

<220>
<223> siRNA IL-17R

<400> 18
   caaacguuca uugguguucu u 21

<210> 19
<211> 21
<212> RNA
<213> Artificial

<220>
<223> siRNA IL-17R

<400> 19
gcaccuacgu agucugcuau u 21

<210> 20

<211>  21
<212>  RNA
<213>  Artificial

<220>
<223>  siRNA IL-17R

<400>  20
uagcagacua cguaggugcu u                                                21


<210>  21
<211>  21
<212>  RNA
<213>  Artificial

<220>
<223>  siRNA IL-17R

<400>  21
cagaaccaau uccggacuau u                                                21


<210>  22
<211>  21
<212>  RNA
<213>  Artificial

<220>
<223>  siRNA IL-17R

<400>  22
uaguccggaa uugguucugu u                                                21


<210>  23
<211>  21
<212>  RNA
<213>  Artificial

<220>
<223>  siRNA IL-17R

<400>  23
aauaugaggu gaccguucau u                                                21


<210>  24
<211>  21
<212>  RNA
<213>  Artificial

<220>
<223>  siRNA IL-17R

<400>  24
ugaacgguca ccucauauuu u                                                21


<210>  25
<211>  21
<212>  RNA
<213>  Artificial

<220>
<223>  siRNA IL-17RC

<400>  25
guacgaaucu gguccuauau u                                                21


<210>  26
<211>  21
<212>  RNA
<213>  Artificial

<220>

<223>  siRNA IL-17RC

<400>  26
uauaggacca gauucguacu u                                                21


<210>  27
<211>  21
<212>  RNA
<213>  Artificial

<220>
<223>  siRNA IL-17RC

<400>  27
gaaccugacu ccguuaggau u                                                21


<210>  28
<211>  21
<212>  RNA
<213>  Artificial

<220>
<223>  siRNA IL-17RC

<400>  28
uccuaacgga gucagguucu u                                                21


<210>  29
<211>  21
<212>  RNA
<213>  Artificial

<220>
<223>  siRNA IL-17RC

<400>  29
gcuaugggac gaugacuugu u                                                21


<210>  30
<211>  21
<212>  RNA
<213>  Artificial

<220>
<223>  siRNA IL-17RC

<400>  30
caagucaucg ucccauagcu u                                                21


<210>  31
<211>  21
<212>  RNA
<213>  Artificial

<220>
<223>  siRNA IL-17RC

<400>  31
gaccgcagau cauuaccuuu u                                                21


<210>  32
<211>  21
<212>  RNA
<213>  Artificial

<220>
<223>  siRNA IL-17RC

<400>  32
aagguaauga ucugcggucu u                                                21

<210>  33
<211>  21
<212>  PRT
<213>  Apis mellifera

<400>  33

Met Lys Phe Leu Val Asn Val Ala Leu Val Phe Met Val Val Tyr Ile
1               5                   10                  15

Ser Tyr Ile Tyr Ala
            20

<210>  34
<211>  527
<212>  PRT
<213>  artificial

<220>
<223>  fusion protein: human IL-17R - human IgG1

<400>  34

Leu Arg Leu Leu Asp His Arg Ala Leu Val Cys Ser Gln Pro Gly Leu
1               5                   10                  15

Asn Cys Thr Val Lys Asn Ser Thr Cys Leu Asp Asp Ser Trp Ile His
            20                  25                  30

Pro Arg Asn Leu Thr Pro Ser Ser Pro Lys Asp Leu Gln Ile Gln Leu
        35                  40                  45

His Phe Ala His Thr Gln Gln Gly Asp Leu Phe Pro Val Ala His Ile
    50                  55                  60

Glu Trp Thr Leu Gln Thr Asp Ala Ser Ile Leu Tyr Leu Glu Gly Ala
65                  70                  75                  80

Glu Leu Ser Val Leu Gln Leu Asn Thr Asn Glu Arg Leu Cys Val Arg
                85                  90                  95

Phe Glu Phe Leu Ser Lys Leu Arg His His His Arg Arg Trp Arg Phe
            100                 105                 110

Thr Phe Ser His Phe Val Val Asp Pro Asp Gln Glu Tyr Glu Val Thr
        115                 120                 125

Val His His Leu Pro Lys Pro Ile Pro Asp Gly Asp Pro Asn His Gln
    130                 135                 140

Ser Lys Asn Phe Leu Val Pro Asp Cys Glu His Ala Arg Met Lys Val
145                 150                 155                 160

Thr Thr Pro Cys Met Ser Ser Gly Ser Leu Trp Asp Pro Asn Ile Thr
                165                 170                 175

Val Glu Thr Leu Glu Ala His Gln Leu Arg Val Ser Phe Thr Leu Trp
            180                 185                 190

Asn Glu Ser Thr His Tyr Gln Ile Leu Leu Thr Ser Phe Pro His Met
        195                 200                 205

Glu Asn His Ser Cys Phe Glu His Met His His Ile Pro Ala Pro Arg
    210                 215                 220

Pro Glu Glu Phe His Gln Arg Ser Asn Val Thr Leu Thr Leu Arg Asn
225                 230                 235                 240

Leu Lys Gly Cys Cys Arg His Gln Val Gln Ile Gln Pro Phe Phe Ser
                245                 250                 255

Ser Cys Leu Asn Asp Cys Leu Arg His Ser Ala Thr Val Ser Cys Pro
            260                 265                 270

Glu Met Pro Asp Thr Pro Glu Pro Ile Pro Asp Tyr Met Pro Leu Trp
        275                 280                 285

Gly Ser Gly Ser Gly Ser Gly Glu Pro Lys Ser Cys Asp Lys Thr His
    290                 295                 300

Thr Cys Pro Pro Cys Pro Ala Pro Glu Ala Leu Gly Ala Pro Ser Val
305                 310                 315                 320

Phe Leu Phe Pro Pro Lys Pro Lys Asp Thr Leu Met Ile Ser Arg Thr
                325                 330                 335

Pro Glu Val Thr Cys Val Val Val Asp Val Ser His Glu Asp Pro Glu
            340                 345                 350

Val Lys Phe Asn Trp Tyr Val Asp Gly Val Glu Val His Asn Ala Lys
        355                 360                 365

Thr Lys Pro Arg Glu Glu Gln Tyr Asn Ser Thr Tyr Arg Val Val Ser
    370                 375                 380

Val Leu Thr Val Leu His Gln Asp Trp Leu Asn Gly Lys Glu Tyr Lys
385                 390                 395                 400

Cys Lys Val Ser Asn Lys Ala Leu Pro Ala Pro Ile Glu Lys Thr Ile
                405                 410                 415

Ser Lys Ala Lys Gly Gln Pro Arg Glu Pro Gln Val Tyr Thr Leu Pro
            420                 425                 430

Pro Ser Arg Glu Glu Met Thr Lys Asn Gln Val Ser Leu Thr Cys Leu
        435                 440                 445

Val Lys Gly Phe Tyr Pro Ser Asp Ile Ala Val Glu Trp Glu Ser Asn
    450                 455                 460

Gly Gln Pro Glu Asn Asn Tyr Lys Thr Thr Pro Pro Val Leu Asp Ser
465                 470                 475                 480

Asp Gly Ser Phe Phe Leu Tyr Ser Lys Leu Thr Val Asp Lys Ser Arg
                485                 490                 495

Trp Gln Gln Gly Asn Val Phe Ser Cys Ser Val Met His Glu Ala Leu
            500                 505                 510

His Asn His Tyr Thr Gln Lys Ser Leu Ser Leu Ser Pro Gly Lys
        515                 520                 525

<210>  35
<211>  660
<212>  PRT
<213>  artificial

<220>
<223>  Fusion protein: human IL-17RC - human IgG1

<400>  35

Met Pro Val Pro Trp Phe Leu Leu Ser Leu Ala Leu Gly Arg Ser Pro
1               5                   10                  15

Val Val Leu Ser Leu Glu Arg Leu Val Gly Pro Gln Asp Ala Thr His
            20                  25                  30

Cys Ser Pro Gly Leu Ser Cys Arg Leu Trp Asp Ser Asp Ile Leu Cys
        35                  40                  45

Leu Pro Gly Asp Ile Val Pro Ala Pro Gly Pro Val Leu Ala Pro Thr
    50                  55                  60

His Leu Gln Thr Glu Leu Val Leu Arg Cys Gln Lys Glu Thr Asp Cys
65                  70                  75                  80

Asp Leu Cys Leu Arg Val Ala Val His Leu Ala Val His Gly His Trp
                85                  90                  95

Glu Glu Pro Glu Asp Glu Glu Lys Phe Gly Gly Ala Ala Asp Ser Gly
            100                 105                 110

Val Glu Glu Pro Arg Asn Ala Ser Leu Gln Ala Gln Val Val Leu Ser
        115                 120                 125

Phe Gln Ala Tyr Pro Thr Ala Arg Cys Val Leu Leu Glu Val Gln Val
    130                 135                 140

Pro Ala Ala Leu Val Gln Phe Gly Gln Ser Val Gly Ser Val Val Tyr
145                 150                 155                 160

Asp Cys Phe Glu Ala Ala Leu Gly Ser Glu Val Arg Ile Trp Ser Tyr
                165                 170                 175

Thr Gln Pro Arg Tyr Glu Lys Glu Leu Asn His Thr Gln Gln Leu Pro
            180                 185                 190

Ala Leu Pro Trp Leu Asn Val Ser Ala Asp Gly Asp Asn Val His Leu
        195                 200                 205

Val Leu Asn Val Ser Glu Glu Gln His Phe Gly Leu Ser Leu Tyr Trp
    210                 215                 220

Asn Gln Val Gln Gly Pro Pro Lys Pro Arg Trp His Lys Asn Leu Thr
225                 230                 235                 240

Gly Pro Gln Ile Ile Thr Leu Asn His Thr Asp Leu Val Pro Cys Leu
                245                 250                 255

Cys Ile Gln Val Trp Pro Leu Glu Pro Asp Ser Val Arg Thr Asn Ile
            260                 265                 270

Cys Pro Phe Arg Glu Asp Pro Arg Ala His Gln Asn Leu Trp Gln Ala
        275                 280                 285

Ala Arg Leu Arg Leu Leu Thr Leu Gln Ser Trp Leu Leu Asp Ala Pro
    290                 295                 300

Cys Ser Leu Pro Ala Glu Ala Ala Leu Cys Trp Arg Ala Pro Gly Gly
305                 310                 315                 320

Asp Pro Cys Gln Pro Leu Val Pro Pro Leu Ser Trp Glu Asn Val Thr
                325                 330                 335

Val Asp Lys Val Leu Glu Phe Pro Leu Leu Lys Gly His Pro Asn Leu
            340                 345                 350

Cys Val Gln Val Asn Ser Ser Glu Lys Leu Gln Leu Gln Glu Cys Leu
        355                 360                 365

Trp Ala Asp Ser Leu Gly Pro Leu Lys Asp Asp Val Leu Leu Leu Glu
    370                 375                 380

Thr Arg Gly Pro Gln Asp Asn Arg Ser Leu Cys Ala Leu Glu Pro Ser
385                 390                 395                 400

Gly Cys Thr Ser Leu Pro Ser Lys Ala Ser Thr Arg Ala Ala Arg Leu
                405                 410                 415

Gly Glu Tyr Leu Leu Gln Asp Leu Gln Ser Gly Gln Cys Leu Gln Leu
            420                 425                 430

Trp Asp Asp Asp Leu Gly Ala Leu Trp Ala Cys Pro Met Asp Lys Tyr
        435                 440                 445

Ile His Lys Arg Ala Gly Ser Gly Ser Gly Ser Gly Glu Pro Lys Ser
    450                 455                 460

Cys Asp Lys Thr His Thr Cys Pro Pro Cys Pro Ala Pro Glu Ala Leu
465                 470                 475                 480

Gly Ala Pro Ser Val Phe Leu Phe Pro Pro Lys Pro Lys Asp Thr Leu
                485                 490                 495

Met Ile Ser Arg Thr Pro Glu Val Thr Cys Val Val Val Asp Val Ser
            500                 505                 510

His Glu Asp Pro Glu Val Lys Phe Asn Trp Tyr Val Asp Gly Val Glu
        515                 520                 525

Val His Asn Ala Lys Thr Lys Pro Arg Glu Glu Gln Tyr Asn Ser Thr
    530                 535                 540

Tyr Arg Val Val Ser Val Leu Thr Val Leu His Gln Asp Trp Leu Asn
545                 550                 555                 560

Gly Lys Glu Tyr Lys Cys Lys Val Ser Asn Lys Ala Leu Pro Ala Pro
                565                 570                 575

Ile Glu Lys Thr Ile Ser Lys Ala Lys Gly Gln Pro Arg Glu Pro Gln
            580                 585                 590

Val Tyr Thr Leu Pro Pro Ser Arg Glu Glu Met Thr Lys Asn Gln Val
        595                 600                 605

Ser Leu Thr Cys Leu Val Lys Gly Phe Tyr Pro Ser Asp Ile Ala Val
    610                 615                 620

Glu Trp Glu Ser Asn Gly Gln Pro Glu Asn Asn Tyr Lys Thr Thr Pro
625                 630                 635                 640

Pro Val Leu Asp Ser Asp Gly Ser Phe Phe Leu Tyr Ser Lys Leu Thr
                645                 650                 655

Val Asp Lys Ser
            660

<210>  36
<211>  510
<212>  DNA
<213>  artificial

<220>
<223>  His-tagged human IL-17F

<400>  36
atgaaattct tagtcaacgt tgcccttgtt tttatggtcg tgtacatttc ttacatctat      60

gcgggatctg gtcaccacca tcatcaccac ggtgacgatg acgataagcg gaaaatcccc     120

aaagtaggac atactttttt ccaaaagcct gagagttgcc cgcctgtgcc aggaggtagt     180

atgaagcttg acattggcat catcaatgaa aaccagcgcg tttccatgtc acgtaacatc     240

gagagccgct ccacctcccc ctggaattac actgtcactt gggaccccaa ccggtacccc     300

tcggaagttg tacaggccca gtgtaggaac ttgggctgca tcaatgctca aggaaaggaa     360

gacatctcca tgaattccgt tcccatccag caagagaccc tggtcgtccg gaggaagcac     420

caaggctgct ctgtttcttt ccagttggag aaggtgctgg tgactgttgg ctgcacctgc     480

gtcacccctg tcatccacca tgtgcagtaa                                      510

<210>  37
<211>  5
<212>  PRT
<213>  Homo sapiens

<400>  37

Arg Lys Ile Pro Lys
1               5

<210>  38
<211>  5
<212>  PRT
<213>  homo sapiens

<400>  38

Ile Val Lys Ala Gly
1               5

<210>  39
<211>  507
<212>  DNA
<213>  artificial

<220>
<223>  Flag-tagged human IL-17A

<400>  39
atgaaattct tagtcaacgt tgcccttgtt tttatggtcg tgtacatttc ttacatctat      60

gcgggatctc ctgactataa agacgatgac gataagatag tgaaggcagg aatcacaatc     120

ccacgaaatc caggatgccc aaattctgag gacaagaact tcccccggac tgtgatggtc     180

aacctgaaca tccataaccg gaataccaat accaatccca aaaggtcctc agattactac     240

aaccgatcca cctcaccttg gaatctccac cgcaatgagg accctgagag atatccctct     300

gtgatctggg aggcaaagtg ccgccacttg ggctgcatca acgctgatgg gaacgtggac     360

taccacatga actctgtccc catccagcaa gagatcctgg tcctgcgcag ggagcctcca     420

cactgcccca actccttccg gctggagaag atactggtgt ccgtgggctg cacctgtgtc     480

accccgattg tccaccatgt ggcctaa                                         507


<210>  40
<211>  492
<212>  DNA
<213>  Macaca sp.

<400>  40
atgacagtga agaccctgca tggcccagtc atggtcaagt acttgctgct gttgatattg      60

ggacttgcct ttctgaatga ggtggcagct aggaaaatcc ccaaagtagg acatactttt     120

ttccaaaagc ctgagagttg cccacctgtg ccagaaggta gtatgaagct tgacactggc     180

atcatcaatg aaaaccagcg tgtttccatg tcacgtaaca tcgagagccg ctccacctcc     240

ccctggaatt acactgtcac ttgggacccc aaccggtatc cctcggaagt tgtacaggcc     300

cagtgtaagc acttgggctg catcaatgct caaggaaagg aagacatctc catgaattcc     360

gttcccatcc agcaagagac cctggtcctc cggaggaagc accaaggctg ctctgtttct     420

ttccagttgg agaaggtgct ggtgactgtt ggctgcacct gcgtcacccc cgtcgtccac     480

catgtgcagt aa                                                         492